(19) Europäisches Patentamt
European Patent Office
Office européen des brevets

(11) EP 1 419 150 B1

(12) FASCICULE DE BREVET EUROPEEN

(45) Date de publication et mention de la délivrance du brevet: **27.04.2005 Bulletin 2005/17**

(21) Numéro de dépôt: **02774822.7**

(22) Date de dépôt: **15.07.2002**

(51) Int Cl.[7]: **C07D 401/12**, C07D 401/14, C07D 403/12, A61K 31/496, A61P 13/00

(86) Numéro de dépôt international: **PCT/FR2002/002500**

(87) Numéro de publication internationale: **WO 2003/008407 (30.01.2003 Gazette 2003/05)**

# (54) DERIVES DE 1-PHENYLSULFONYL-1,3-DIHYDRO-2H-INDOL-2-ONE, LEUR PREPARATION ET LEUR APPLICATION EN THERAPEUTIQUE

PHENYLSULFONYL-1,3-DIHYDRO-2H-INDOL-2-ON DERIVATE, DEREN HERSTELLUNG UND DEREN THERAPEUTISCHE VERWENDUNG

PHENYLSULFONYL-1,3-DIHYDRO-2H-INDOLE-2-ONE DERIVATIVES, THEIR PREPARATION AND THEIR THERAPEUTIC USE

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **17.07.2001 FR 0110359**

(43) Date de publication de la demande: **19.05.2004 Bulletin 2004/21**

(73) Titulaire: **Sanofi-Aventis**
**75013 Paris (FR)**

(72) Inventeurs:
- **DI MALTA, Alain**
  **F-34980 Saint-Clément-de-Rivière (FR)**
- **GARCIA, Georges**
  **F-34110 Frontignan (FR)**
- **ROUX, Richard**
  **F-34570 Vailhauques (FR)**
- **SCHOENTJES, Bruno**
  **76230 Bois-Guillame (FR)**
- **SERRADEIL-LE GAL, Claudine**
  **F-31750 Escalquens (FR)**
- **TONNERRE, Bernard**
  **F-34570 Vailhauques (FR)**
- **WAGNON, Jean**
  **F-34070 Montpellier (FR)**

(74) Mandataire: **Varady, Peter et al**
**Sanofi-Aventis**
**174 avenue de France**
**75013 Paris (FR)**

(56) Documents cités:
**WO-A-95/18105** **GB-A- 2 326 639**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 419 150 B1

Printed by Jouve, 75001 PARIS (FR)

## Description

La présente invention a pour objet des dérivés de 1,3-dihydro-2*H*-indol-2-one, leur préparation et leur application en thérapeutique.

Les composés selon la présente invention présentent une affinité pour les récepteurs $V_{1b}$ de l'arginine-vasopressine (AVP) et/ou pour les récepteurs de l'ocytocine (OT) et, de plus, pour certains d'entre eux une affinité pour les récepteurs $V_{1a}$ de l'AVP.

L'AVP est une hormone connue pour son effet antidiurétique et son effet dans la régulation de la pression artérielle. Elle stimule plusieurs types de récepteurs : $V_1$ ($V_{1a}$,$V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardio-vasculaires, hépatiques, pancréatiques, antidiurétiques, agrégants des plaquettes et des effets sur le système nerveux central et périphérique, et sur la sphère utérine.

L'OT est une hormone neurohypophysaire, de structure nonapeptidique cyclique proche de celle de l'AVP. Les récepteurs de l'OT se trouvent essentiellement sur le muscle lisse de l'utérus et sur les cellules myoépithéliales des glandes mammaires. Ainsi, l'OT joue un rôle important dans la parturition puisqu'elle intervient dans la contraction du muscle utérin et dans la lactation. Par ailleurs, les récepteurs de l'OT sont également localisés dans d'autres tissus périphériques et dans le système nerveux central ; l'OT peut donc avoir des effets dans les domaines cardiovasculaire, rénal, endocrinien ou comportemental.

La localisation des différents récepteurs est décrite dans : S. JARD et al., Vasopressin and oxytocin receptors : an overview, in Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ils ont été clonés, en particulier chez le rat et l'homme, et ils régulent la majorité des effets connus de l'AVP : l'agrégation plaquettaire ; les contractions utérines ; la contraction des vaisseaux ; la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire, ...).

Les récepteurs $V_{1b}$ ont été initialement identifiés dans l'adénohypophyse de différentes espèces animales (rat, porc, boeuf, mouton...) y compris chez l'homme (S. JARD et al., Mol. Pharmacol., 1986, 30, 171-177 ; Y. ARSENIJEVIC et al., J. Endocrinol., 1994, 141, 383-391 ; J. SCHWARTZ et al., Endocrinology, 1991, 129 (2), 1107-1109 ; Y. DE KEYSER et al., FEBS Letters, 1994, 356, 215-220) où ils stimulent la libération d'hormone adrénocorticotrophique par l'AVP et potentialisent les effets du CRF sur la libération d'ACTH (G.E. GILLIES et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. DE KEYSER, FEBS Letters, 1994, 356, 215-220 ; T. SUGIMOTO et al., J. Biol. Chem., 1994, 269 (43), 27088-27092 ; M. SAITO et al., Biochem. Biophys. Res. Commun., 1995, 212 (3), 751-757 ; S.J. LOLAIT et al., Neurobiology, 1996, 92, 6783-6787 ; M.A. VENTURA et al., Journal of Molecular endocrinology, 1999, 22, 251-260) et différentes études (hybridation *in situ*, PCR, de l'anglais Polymerase Chain Reaction, ...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régule le pancréas endocrine en stimulant la sécrétion d'insuline et de glucagon (B. LEE et al., Am. J. Physiol. 269 (Endocrinol. Metab. 32) : E1095-E1100, 1995) ou la production de catécholamines dans la medullo-surrénale qui est le siège d'une synthèse locale d'AVP (E. GRAZZINI et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétants de l'AVP et induisant de ce fait une production soutenue de catécholamines à l'origine d'hypertensions résistantes aux antagonistes des récepteurs de l'angiotensine II et aux inhibiteurs de l'enzyme de conversion. Le cortex surrénalien est aussi riche en récepteurs $V_{1a}$ impliqués dans la production de gluco- et minéralo-corticoides (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1b}$ et/ou $V_{1a}$ portés par les cellules de la médulla (G. MAZZOCCHI et al.,

Peptides, 1997, 18 (2), 191-195 ; E. GRAZZINI et al., J. Clin. Endocrinol. Metab., 1999, 84 (6), 2195-2203).

Les récepteurs $V_{1b}$ sont aussi considérés comme un marqueur de tumeurs. Par exemple, les tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroidiens, induisant un syndrome de Cushing dans certains cas (J. BERTHERAT et al., Eur. J. Endocrinol., 1996, 135,173 ; G.A. WITTERT et al., Lancet, 1990, 335, 991-994 ; G. DICKSTEIN et al., J. Clin. Endocrinol. Metab., 1996, 81 (8), 2934-2941) surexpriment les récepteurs $V_{1b}$. Les récepteurs $V_{1a}$ sont, quant à eux, un marqueur plus spécifique des cancers pulmonaires à petites cellules (SCLC) (P.J. WOLL et al., Biochem. Biophys. Res. Commun., 1989, 164 (1), 66-73). Ainsi, les composés selon la présente invention sont des outils de diagnostic évidents et offrent une approche thérapeutique nouvelle dans la prolifération et la détection de ces tumeurs, à un stade même précoce (radiomarquage ; SPECT, de l'anglais Single Photon Emission Computed Tomography ; PET Scan, de l'anglais Positron Emission Tomography Scanner).

La présence abondante du messager des récepteurs $V_{1b}$ au niveau stomacal et intestinal, suggère une implication de l'AVP via ce récepteur sur la libération d'hormones gastrointestinales comme la cholécystokinine, la gastrine ou la sécrétine (T. SUGIMOTO et al., Molecular cloning and functional expression of $V_{1b}$ receptor gene, dans Neurohypophysis : Recent Progress of Vasopressin and Oxytocin Research ; T. SAITO, K. KUROKAWA and S. YOSHIDA ed., Elvesier Science, 1995, 409-413).

Des dérivés de 1,3-dihydro-2*H*-indol-2-one ont été décrits dans certaines demandes de brevet comme des ligands des récepteurs de l'arginine-vasopressine et/ou de l'ocytocine : on peut citer les demandes de brevets WO 93/15051, EP 636608, EP 636609, WO 95/18105, WO 97/15556 et WO 98/25901.

On a maintenant trouvé de nouveaux dérivés de 1,3-dihydro-2*H*-indol-2-one qui présentent une affinité et une sélectivité pour les récepteurs $V_{1b}$ de l'arginine-vasopressine et/ou pour les récepteurs de l'ocytocine et, de plus, pour certains composés une affinité pour les récepteurs $V_{1a}$. Ces dérivés n'ont pas d'affinité pour les récepteurs $V_2$ de l'AVP.

Ainsi, selon un de ses aspects, la présente invention a pour objet des composés de formule :

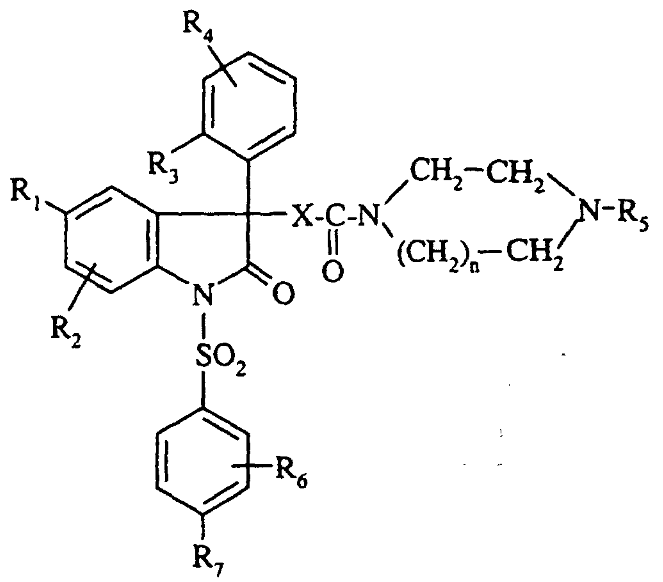

(I)

dans laquelle :

- n est 1 ou 2 ;
- X représente un groupe $-CH_2-$ ; -O- ; -NH- ; $-O-CH_2-$ ; $-NH-CH_2-$ ; $-NH-CH_2-CH_2-$ ;
- $R_1$ représente un atome d'halogène ; un $(C_1-C_4)$alkyle ; un $(C_1-C_4)$alcoxy ;
- $R_2$ représente un atome d'hydrogène ; un atome d'halogène ; un $(C_1-C_4)$alkyle ; un $(C_1-C_4)$alcoxy ; un radical trifluorométhyle ;
- $R_3$ représente un atome d'halogène ; un $(C_1-C_3)$alkyle ; un $(C_1-C_3)$alcoxy ; un radical trifluorométhyle ; un radical trifluorométhoxy ;
- $R_4$ représente un atome d'hydrogène ; un halogène ; un $(C_1-C_3)$alkyle ; un $(C_1-C_3)$alcoxy ;
- $R_5$ représente un radical choisi parmi :

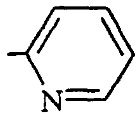 ; 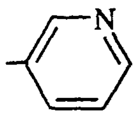 ; 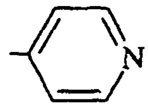 ; 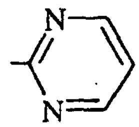 ; 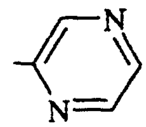 ; 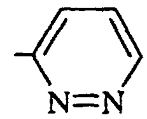 ;

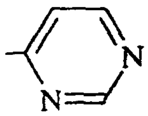 ; 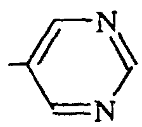 ; 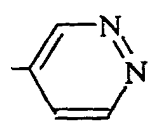 ; 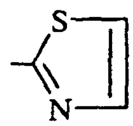 ; 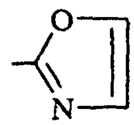 ; 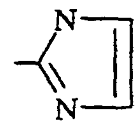

- $R_6$ représente un ($C_1$-$C_4$)alcoxy ;
- $R_7$ représente un ($C_1$-$C_4$)alcoxy ;

ainsi que leurs sels avec des acides minéraux ou organiques, leurs solvats et/ou leurs hydrates.

Les composés de formule (I) peuvent comporter un ou plusieurs atomes de carbone asymétriques. Ils peuvent donc exister sous forme d'énantiomères, ou de diastéréoisoméres. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Les sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Par halogène on entend un atome de chlore, de brome, de fluor ou d'iode.

Par alkyle on entend un radical alkyle linéaire ou ramifié de un à trois atomes de carbone ou de un à quatre atomes de carbone, tel que le radical méthyle, éthyle, propyle, isopropyle, butyle, isobutyle, *sec*-butyle ou *tert*-butyle.

Par alcoxy on entend un radical alcoxy linéaire ou ramifié de un à trois atomes de carbone ou de un à quatre atomes de carbone, tel que le radical méthoxy, éthoxy, propoxy, isopropoxy, butoxy, isobutoxy, *sec*-butoxy ou *tert*-butoxy.

Avantageusement, l'invention concerne des composés de formule (I) dans laquelle $R_5$ représente un radical choisi parmi :

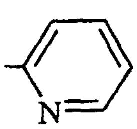 ; 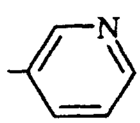 ; 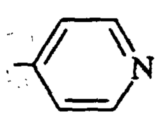 ; 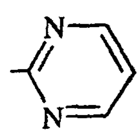 ; 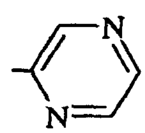 ; 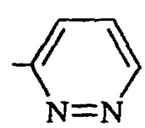 ;

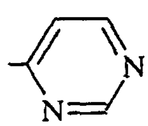 ; 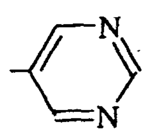 ; 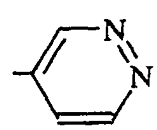

Selon la présente invention, on préfère les composés de formule (I) dans laquelle $R_1$ représente un atome de chlore ou un radical méthyle.

Selon la présente invention, on préfère les composés de formule (I) dans laquelle $R_2$ représente un atome d'hydrogène, un atome de chlore, un radical méthyle, un radical méthoxy ou un radical trifluorométhyle.

Selon la présente invention, on préfère les composés de formule (I) dans laquelle $R_3$ représente un atome de chlore, un atome de fluor, un radical méthoxy, un radical éthoxy, un radical isopropoxy, un radical trifluorométhoxy ou un radical trifluorométhyle.

Selon la présente invention, on préfère les composés de formule (I) dans laquelle $R_4$ représente un atome d'hydrogène ou un radical méthoxy.

Selon la présente invention, on préfère les composés de formule (I) dans laquelle $R_5$ représente un 2-pyridi-

nyle, un 3-pyridinyle, un 4-pyridinyle, un 2-pyrimidinyle, un 2-pyrazinyle, un 3-pyridazinyle, un 1,3-thiazol-2-yle.

Selon la présente invention, on préfère les composés de formule (I) dans laquelle $R_6$ est en position -2- du phényle et représente un radical méthoxy.

Selon la présente invention, on préfère les composés de formule (I) dans laquelle $R_7$ représente un radical méthoxy.

Particulièrement, on préfère les composés de formule (I) dans laquelle :

- n et X sont tels que définis pour un composé de formule (I) ;
- $R_1$ représente un atome de chlore ou un radical méthyle ;
- $R_2$ représente un atome d'hydrogène ou est en position -4- ou -6- de l'indol-2-one et représente un atome de chlore, un radical méthyle, un radical méthoxy ou un radical trifluorométhyle ;
- $R_3$ représente un atome de chlore, un atome de fluor, un radical méthoxy, un radical éthoxy, un radical isopropoxy, un radical trifluorométhyle ou un radical trifluorométhoxy ;
- $R_4$ représente un atome d'hydrogène ou un radical méthoxy ;
- $R_5$ représente un 2-pyridinyle, un 3-pyridinyle, un 4-pyridinyle, un 2-pyrimidinyle, un 2-pyrazinyle, un 3-pyridazinyle, un 1,3-thiazol-2-yle ;
- $R_6$ est en position -2- du phényle et représente un radical méthoxy ;
- $R_7$ représente un radical méthoxy ; ainsi que leurs sels avec des acides minéraux ou organiques, leurs solvats et/ou hydrates.

Plus particulièrement, on préfère les composés de formule (I) dans laquelle :

- n est 1 ou 2 ;
- X représente un groupe $-CH_2-$ ; -O- ; -NH- ;
- $R_1$ représente un atome de chlore ;
- $R_2$ représente un atome d'hydrogène ;
- $R_3$ représente un radical méthoxy, un radical éthoxy, un radical isopropoxy ;
- $R_4$ représente un atome d'hydrogène ;
- $R_5$ représente un radical choisi parmi :

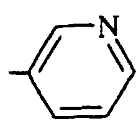 ; 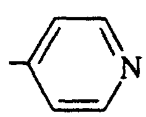 ; 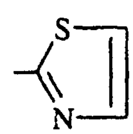

- $R_6$ est en position -2- du phényle et représente un radical méthoxy ;
- $R_7$ représente un radical méthoxy ;

ainsi que leurs sels avec des acides minéraux ou organiques, leurs solvats et/ou hydrates.

Plus particulièrement aussi, on préfère les composés de formule (I) dans laquelle :

- n est 1 ;
- X représente un groupe $-CH_2-$ ; -O- ; -NH- ;
- $R_1$ représente un atome de chlore ou un radical méthyle ;
- $R_2$ représente un atome d'hydrogène ou est en position -4- ou -6- de l'indol-2-one et représente un atome de chlore ; un radical méthyle ; un radical méthoxy ;
- $R_3$ représente un atome de chlore, un atome de fluor ou un radical méthoxy ;
- $R_4$ représente un atome d'hydrogène ;
- $R_5$ représente un radical choisi parmi :

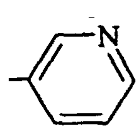 ; 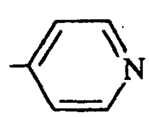

- $R_6$ est en position -2- du phényle et représente un radical méthoxy ;
- $R_7$ représente un radical méthoxy ;

ainsi que leurs sels avec des acides minéraux ou organiques, leurs solvats et/ou hydrates.

Plus particulièrement enfin, on préfère les composés de formule (I) dans laquelle :

- n est 1 ;
- X représente un groupe -O- ; -NH- ; $-NH-CH_2-CH_2-$ ;
- $R_1$ représente un atome de chlore ;
- $R_2$ représente un atome d'hydrogène ;
- $R_3$ représente un atome de chlore ou un radical méthoxy ;
- $R_4$ représente un atome d'hydrogène ;
- $R_5$ représente un radical

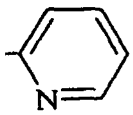 ;

- $R_6$ est en position -2- du phényle et représente un radical méthoxy ;
- $R_7$ représente un radical méthoxy ;

ainsi que leurs sels avec des acides minéraux ou organiques, leurs solvats et/ou hydrates.

Les composés suivants :

- 5-Chloro-3-(2-éthoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one ;
- 5-Chloro-3-(2-isopropoxyphényl)-1-[(2,4-diméthoxyphényl)sulfonyl]-3-[2-oxo-2-[4-(4-pyridinyl)-1-pipérazinyl] éthyl]-1,3-dihydro-2*H*-indol-2-one ;
- 4-(2-Pyridinyl)-1-pipérazinecarboxylate de 5-chloro-1-[(2,4-diméthoxyphényl)sulfonyl]-3-(2-méthoxyphényl)-2-oxo-2,3-dihydro-1*H*-indol-3-yle ;
- 4-(4-Pyridinyl)-1-pipérazinecarboxylate de 5-chloro-1-[(2,4-diméthoxyphényl)sulfonyl]-3-(2-isopropoxyphényl)-2-oxo-2,3-dihydro-1*H*-indol-3-yle ;
- N-[5-Chloro-1-[(2,4-diméthoxyphényl)sulfonyl]-3-(2-méthoxyphényl)-2-oxo-2,3-dihydro-1*H*-indol-3-yl]-4-(4-pyridinyl)homopipérazine-1-carboxamide ;
- N-[5-Chloro-1-[(2,4-diméthoxyphényl)sulfonyl]-3-(2-isopropoxyphényl)-2-oxo-2,3-dihydro-1*H*-indol-3-yl]-4-(3-pyridinyl)pipérazine-1-carboxamide ;
- N-[5-Chloro-1-[(2,4-diméthoxyphényl)sulfonyl]-3-(2-isopropoxyphényl)-2-oxo-2,3-dihydro-1*H*-indol-3-yl]-4-(4-pyridinyl)pipérazine-1-carboxamide ;
- 5-Chloro-1-[(2,4-diméthoxyphényl)sulfonyl]-3-(2-méthoxyphényl)-3-[[3-oxo-3-[4-(2-pyridinyl)-1-pipérazinyl]propyl]amino]-1,3-dihydro-2*H*-indol-2-one ;
- 5,6-Dichloro-1-[(2,4-diméthoxyphényl)sulfonyl]-3-(2-méthoxyphényl)-3-[2-oxo-2-[4-(4-pyridinyl)-1-pipérazinyl] éthyl]-1,3-dihydro-2*H*-indol-2-one ;
- 5-Chloro-1-[(2,4-diméthoxyphényl)sulfonyl]-3-(2-méthoxyphényl)-6-méthyl-3-[2-oxo-2-[4-(4-pyridinyl)-1-pipérazinyl]éthyl]-1,3-dihydro-2*H*-indol-2-one ;
- 4-(4-Pyridinyl)-1-pipérazinecarboxylate de 5-chloro-1-[(2,4-diméthoxyphényl)sulfonyl]-3-(2-méthoxyphényl)-6-méthyl-2-oxo-2,3-dihydro-1*H*-indol-3-yle ;
- 4-(4-Pyridinyl)-1-pipérazinecarboxylate de 5-chloro-1-[(2,4-diméthoxyphényl)sulfonyl]-6-méthoxy-3-(2-méthoxyphényl)-2-oxo-2,3-dihydro-1*H*-indol-3-yle ;
- N-[5-Chloro-3-(2-chlorophényl)-1-[(2,4-diméthoxyphényl)sulfonyl]-2-oxo-2,3-dihydro-1*H*-indol-3-yl]-4-(2-pyridinyl)pipérazine-1-carboxamide ;
- N-[5-Chloro-1-[(2,4-diméthoxyphényl)sulfonyl]-3-(2-méthoxyphényl)-6-méthyl-2-oxo-2,3-dihydro-1*H*-indol-3-yl]-4-(4-pyridinyl)pipérazine-1-carboxamide ;
- N-[6-Chloro-3-(2-chlorophényl)-1-[(2,4-diméthoxyphényl)sulfonyl]-5-méthyl-2-oxo-2,3-dihydro-1*H*-indol-3-yl]-4-(4-pyridinyl)pipérazine-1-carboxamide ;
- 5-Chloro-1-[(2,4-diméthoxyphényl)sulfonyl]-3-(2-fluorophényl)-3-[2-oxo-2-[4-(4-pyridinyl)-1-pipérazinyl]éthyl]-1,3-dihydro-2*H*-indol-2-one ;
- 5,6-Dichloro-1-[(2,4-diméthoxyphényl)sulfonyl]-3-(2-fluorophényl)-3-[2-oxo-2-[4-(4-pyridinyl)-1-pipérazinyl]éthyl]-1,3-dihydro-2*H*-indol-2-one ;

- 5-Chloro-3-(2,3-diméthoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one ;
- 5-Chloro-1-[(2,4-diméthoxyphényl)sulfonyl]-3-(2-éthoxyphényl)-3-[2-oxo-2-[4-(3-pyridinyl)-1-pipérazinyl]éthyl]-1,3-dihydro-2*H*-indol-2-one ;
- 5-Chloro-1-[(2,4-diméthoxyphényl)sulfonyl]-3-(2-isopropoxyphényl)-3-[2-oxo-2-[4-(3-pyridinyl)-1-pipérazinyl]éthyl]-1,3-dihydro-2*H*-indol-2-one ;
- 5-Chloro-1-[(2,4-diméthoxyphényl)sulfonyl]-3-(2-isopropoxyphényl)-3-[2-oxo-2-[4-(4-pyridinyl)-1-homopipérazine]éthyl]-1,3-dihydro-2*H*-indol-2-one ;
- 5-Chloro-1-[(2,4-diméthoxyphényl)sulfonyl]-3-(2-isopropoxyphényl)-3-[2-oxo-2-[4-(1,3-thiazol-2-yl)-1-pipérazinyl]éthyl]-1,3-dihydro-2*H*-indol-2-one ;
- 4-(3-Pyridinyl)-1-pipérazinecarboxylate de 5-chloro-1-[(2,4-diméthoxyphényl)sulfonyl]-3-(2-éthoxyphényl)-2-oxo-2,3-dihydro-1*H*-indol-3-yle ;
- 4-(4-Pyridinyl)-1-pipérazinecarboxylate de 5-chloro-1-[(2,4-diméthoxyphényl)sulfonyl]-3-(2-éthoxyphényl)-2-oxo-2,3-dihydro-1*H*-indol-3-yle ;

sous forme d'isomères optiquement purs ou sous forme de mélange, ainsi que leurs sels avec des acides minéraux ou organiques, leurs solvats et/ou hydrates sont tout particulièrement préférés.

Selon un autre de ses aspects, la présente invention a pour objet un procédé de préparation des composés de formule (I) caractérisé en ce que :
on fait réagir un composé de formule :

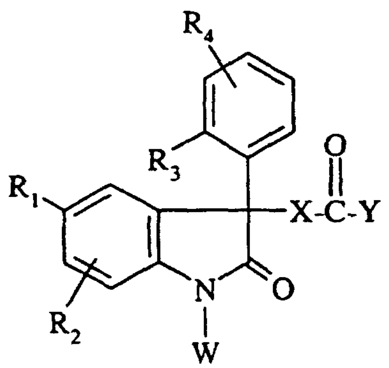

(II)

dans laquelle $R_1$, $R_2$, $R_3$, $R_4$ et X sont tels que définis pour un composé de formule (I), et :

- Y représente un hydroxy ou un atome de chlore lorsque X représente un groupe $-CH_2-$ ; $-OCH_2-$ ; $-NH-CH_2-$ ; $-NH-CH_2-CH_2-$ ;
- ou Y représente un phénoxy lorsque X représente un groupe -O- ; -NH- ;
- W représente un atome d'hydrogène lorsque X représente un groupe $-CH_2-$ ; $-OCH_2-$ ;
- ou W représente un groupe

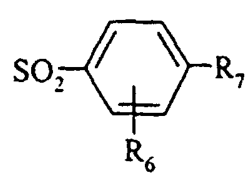

dans lequel $R_6$ et $R_7$ sont tels que définis pour un composé de formule (I) lorsque X représente un groupe -O- ; -NH-; $-NH-CH_2-$ ; $-NH-CH_2-CH_2-$ ;

avec un composé de formule :

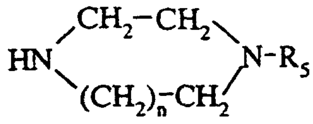 (III)

dans laquelle n et $R_5$ sont tels que définis pour un composé de formule (I) ;

- lorsque W représente un groupe

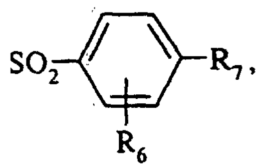

on obtient le composé de formule (I) attendu ;

- ou lorsque W représente un atome d'hydrogène, on fait réagir, en présence d'une base, le composé ainsi obtenu de formule :

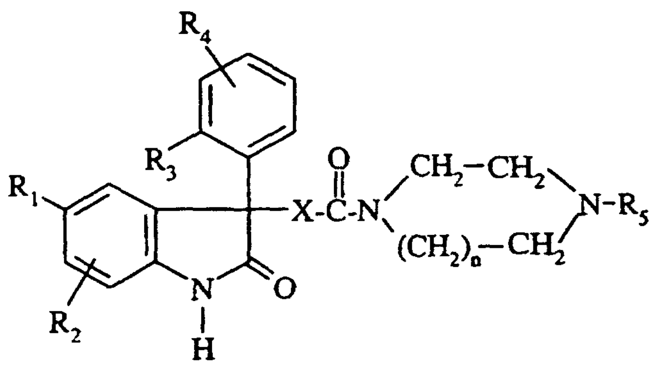 (IV)

avec un halogénure de sulfonyle de formule :

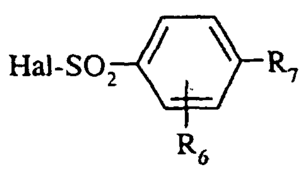 (V)

dans laquelle $R_6$ et $R_7$ sont tels que définis pour un composé de formule (I) et Hal représente un atome d'halogène.

Eventuellement, on transforme le composé de formule (I) en l'un de ses sels avec des acides minéraux ou organiques.

Lorsque Y représente un hydroxy, la réaction du composé de formule (II) avec le composé de formule (III) s'effectue en présence d'un agent de couplage utilisé en chimie peptidique tel que l'hexafluorophosphate de benzotriazol-1-yloxytris(diméthyl amino)phosphonium ou l'hexafluorophosphate de benzotriazol-1-yloxytripyrrolidino phosphonium et en présence d'une base telle que la triéthylamine ou la N,N-diisopropyléthylamine, dans un solvant tel que le dichlorométhane ou le N,N-diméthylformamide, à une température comprise entre 0°C et la température ambiante.

Lorsque Y représente un atome de chlore, la réaction du composé (II) avec le composé (III) s'effectue en l'absence ou en présence d'une base telle que la triéthylamine ou la N,N-diisopropyléthylamine, dans un solvant tel que la dichlorométhane ou le chloroforme et à une température comprise entre - 60°C et la température ambiante. En l'absence de base on utilise un excès de composé (III).

Lorsque Y représente un phénoxy, la réaction du composé (II) avec le composé (III) s'effectue dans un solvant tel que le dichlorométhane , le chloroforme ou le tétrahydrofurane ou un mélange de ces solvants et à une température comprise entre la température ambiante et la température de reflux du solvant.

On obtient ainsi soit directement un composé de formule (I), soit un composé de formule (IV). La réaction du composé (IV) avec l'halogénure de sulfonyle (V) s'effectue en présence d'une base forte comme un hydrure métallique tel que l'hydrure de sodium ou un alcoolate alcalin tel que le *tert*-butylate de potassium, dans un solvant tel que le N, N-diméthylformamide ou le tétrahydrofurane et à une température comprise entre - 70°C et la température ambiante. La réaction s'effectue de préférence en utilisant un composé de formule (V) dans laquelle Hal représente un atome de chlore.

Les composés de formule (I) ainsi obtenus peuvent être ultérieurement séparés du milieu réactionnel et purifiés selon les méthodes classiques, par exemple, par cristallisation ou chromatographie.

Les composés de formule (I) ainsi obtenus sont isolés sous forme de base libre ou de sel, selon les techniques classiques.

Les composés de formule (II) se préparent selon différents modes opératoires.

Les composés de formule (II) dans laquelle -X- représente un groupe $-CH_2-$, Y représente un hydroxy et W représente l'hydrogène se préparent selon le SCHEMA 1 ci-après dans lequel $R_1$, $R_2$, $R_3$ et $R_4$ sont tels que définis pour un composé de formule (I) et Alk représente un $(C_1-C_2)$alkyle.

SCHEMA 1

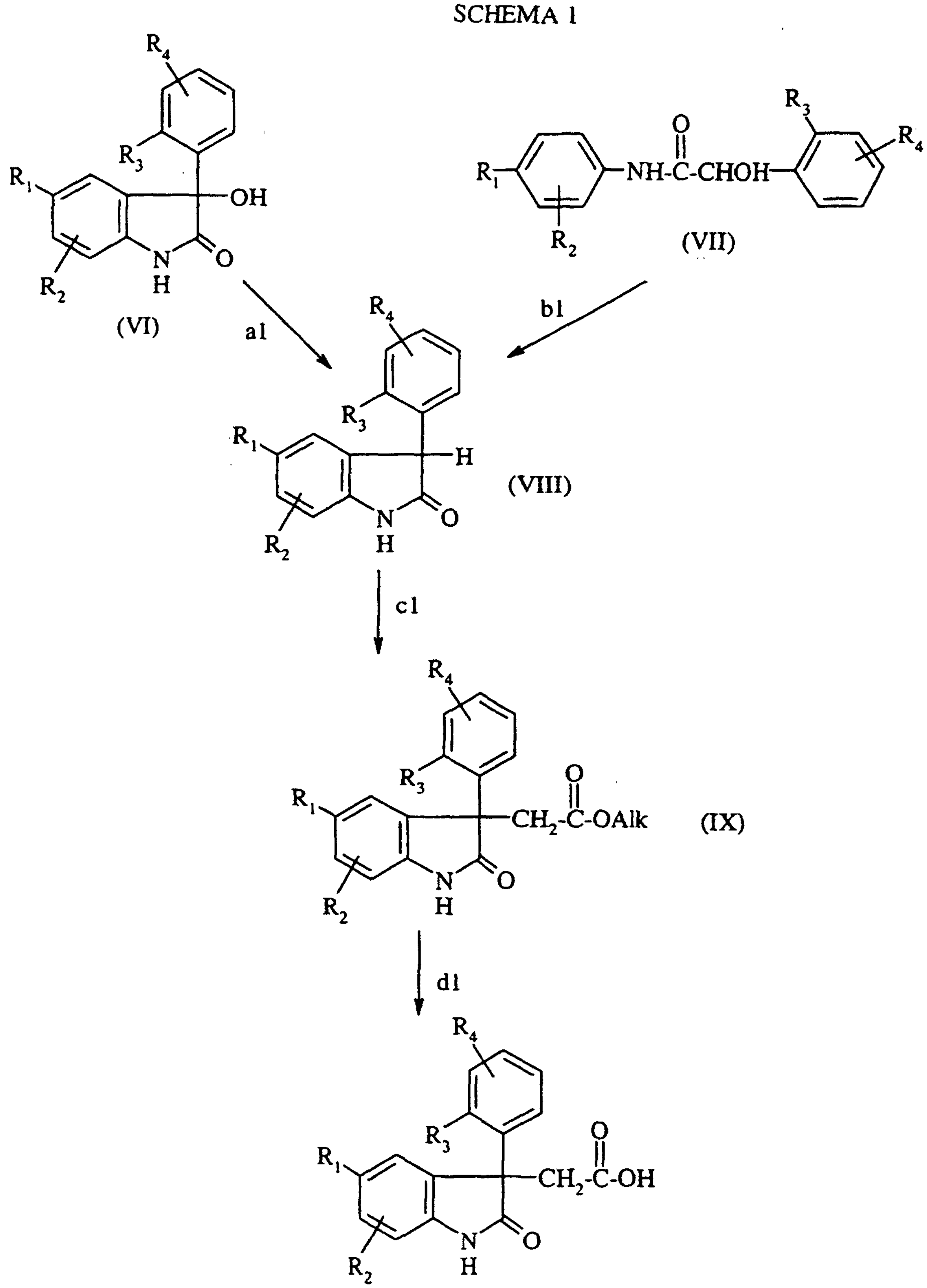


(VI)
(VII)
(VIII)
(IX)

(II) : X = $-CH_2-$ ; Y = -OH ; W = H;

A l'étape a1 du SCHEMA 1, on prépare un composé de formule (VIII) par deshydroxylation d'un composé de formule (VI) correspondant par action du triéthylsilane selon Bioorganic and Medicinal Chemistry Letters, 1997, 7 (10),

1255-1260.

On peut également préparer un composé de formule (VIII) par réaction de cyclisation (étape b1) d'un composé de formule (VII) en milieu acide fort, l'acide sulfurique ou l'acide polyphosphorique par exemple, selon la méthode décrite dans WO 95/18105 ou dans J. Org. Chem., 1968, 33, 1640-1643..

On fait réagir à l'étape c1 le composé de formule (VIII) avec un composé de formule Hal-$CH_2COOAlk$ dans laquelle Hal représente un atome d'halogène de préférence le brome ou le chlore et Alk représente un ($C_1$-$C_2$)alkyle, en présence d'une base tel qu'un carbonate de métal alcalin (le carbonate de potassium par exemple) et d'un iodure de métal alcalin (l'iodure de potassium par exemple) ou en présence d'une base forte tel qu'un hydrure de métal alcalin (l'hydrure de sodium par exemple), ou un alcoolate de métal alcalin (l'éthylate de sodium par exemple) pour obtenir le composé de formule (IX). La réaction s'effectue dans un solvant tel que l'acétone ou le N,N-diméthylformamide et à une température comprise entre 0°C et la température de reflux du solvant.

A l'étape d1 on obtient le composé de formule (II) attendu par hydrolyse en milieu alcalin, du composé de formule (IX) en utilisant un hydroxyde de métal alcalin tel que l'hydroxyde de sodium, l'hydroxyde de potassium ou l'hydroxyde de lithium dans un solvant tel que l'eau, le méthanol, l'éthanol, le tétrahydrofurane, le dioxane ou un mélange de ces solvants, à une température comprise entre 0°C et la température de reflux du solvant.

Les composés de formule (VI) sont connus et se préparent selon des méthodes connues telles que celles décrites dans WO 95/18105.

Par exemple, on prépare un composé de formule (VI) par réaction d'un dérivé de 1*H*-indole-2,3-dione de formule :

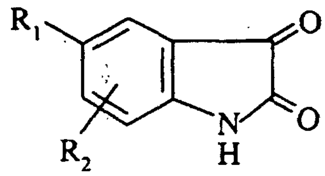

(X)

dans laquelle $R_1$ et $R_2$ sont tels que définis pour un composé de formule (I), avec un dérivé organomagnésien de formule :

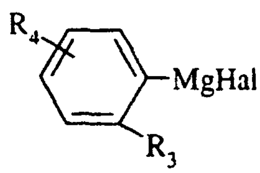

(XI)

dans laquelle $R_3$ et $R_4$ sont tels que définis pour un composé de formule (I) et Hal représente un atome d'halogène, de préférence le brome ou l'iode, dans un solvant tel que le tétrahydrofurane ou l'éther diéthylique et à une température comprise entre 0°C et la température de reflux du solvant.

On peut également préparer un composé de formule (VI) dans laquelle $R_3$ est tel que défini pour un composé de formule (I) et $R_4$, différent de l'hydrogène, est en position -3- ou -6- du phényle, par réaction d'un composé de formule :

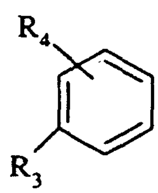

(XXXIV)

dans laquelle $R_3$ est tel que défini pour un composé de formule (I) et $R_4$ est en position -2- ou -5- du phényle, avec un dérivé du lithium tel que le n-butyllithium, puis l'intermédiaire lithié ainsi obtenu est mis en réaction avec un composé de formule (X). La réaction s'effectue dans un solvant tel que l'éther diéthylique, le tétrahydrofurane, l'hexane ou un mélange de ces solvants, à une température comprise entre -70°C et la température ambiante.

Les dérivés de 1*H*-indole-2,3-dione (X) sont commerciaux ou se préparent selon les méthodes décrites dans

Tetrahedron Letters, 1998, 39, 7679-7682 ; Tetrahedron Letters, 1994, 35, 7303-7306 ; J. Org. Chem., 1977, 42 (8), 1344-1348 ; J. Org. Chem., 1952, 17, 149-156 ; J. Am. Chem. Soc., 1946, 68, 2697-2703 ; Organic Synthèses, 1925, V, 71-74 et Advances in Heterocyclic Chemistry, A.R. Katritzky and A.J. Boulton, Academic Press, New-York, 1975, 18, 2-58.

Les dérivés organomagnésiens (XI) sont préparés selon les méthodes classiques bien connues de l'homme de l'art.

De façon particulière, on peut préparer les composés de formule (VI) dans laquelle $R_3$ = $(C_1\text{-}C_2)$alcoxy et $R_4$ = H, ou bien $R_3$ = $R_4$ = $(C_1\text{-}C_2)$alcoxy avec $R_4$ en position -3 ou -6 du phényle, $R_2$ est différent d'un atome d'halogène et $R_1$ est tel que défini pour un composé de formule (I), en suivant le procédé décrit dans le SCHEMA 2.

SCHEMA 2

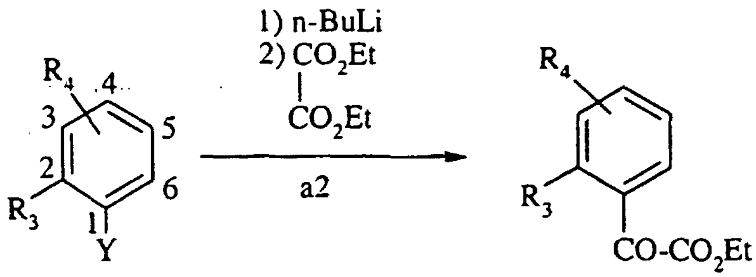


(XIII)

(XII) : $R_3$ = $(C_1\text{-}C_2)$alcoxy, $R_4$ = H ;
$R_3$ = $R_4$ = $(C_1\text{-}C_2)$alcoxy avec
$R_4$ en position -3 ou -6 ;
Y = H ou Br.

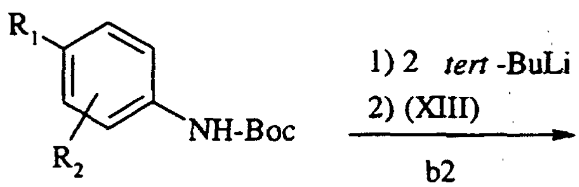


(VI)

(XIV)

A l'étape a2 du SCHEMA 2, on fait d'abord réagir un composé de formule (XII) avec un dérivé du lithium tel que le n-butyllithium, en l'absence ou en présence d'une base telle que la N, N, N', N'-tétraméthyléthylènediamine, puis l'intermédiaire lithié ainsi obtenu est mis en réaction avec l'oxalate de diéthyle pour donner le composé de formule (XIII). La réaction s'effectue dans un solvant tel que l'éther diéthylique, le tétrahydrofurane, l'hexane ou un mélange de ces solvants et à une température comprise entre -70°C et la température ambiante.

A l'étape b2, on fait d'abord réagir un composé de formule (XIV) avec deux équivalents d'un dérivé du lithium tel que le *tert*-butyllithium, puis l'intermédiaire lithié ainsi obtenu est mis en réaction avec le composé de formule (XIII) pour donner le composé de formule (VI) attendu. La réaction s'effectue dans un solvant tel que l'éther diéthylique, le tétrahydrofurane , le pentane ou un mélange de ces solvants et à une température comprise entre -70°C et la température ambiante.

Les composés de formule (XII) sont commerciaux ou synthétisés de manière classique.

Les composés de formule (XIV) sont préparés par réaction des dérivés de l'aniline correspondants avec du di-*tert*-butyldicarbonate selon les méthodes classiques.

Les composés de formule (VII) sont connus et se préparent selon des méthodes connues telles que celles décrites dans WO 95/18105 ou dans J. Org. Chem., 1968, 33, 1640-1643.

Les composés de formule (II) dans laquelle X = $-CH_2-$, Y représente un atome de chlore et W = H se préparent à partir des composés de formule (II) correspondants dans laquelle Y = OH par réaction avec du chlorure de thionyle dans un solvant tel que le toluène et à une température comprise entre 0°C et la température de reflux du solvant.

Les composés de formule (II) dans laquelle -X- représente un groupe -O-, Y représente un phénoxy et W =

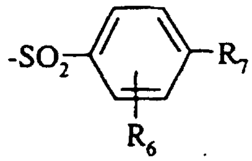

se préparent selon le SCHEMA 3 ci-après dans lequel $R_1$, $R_2$, $R_3$, $R_4$, $R_6$ et $R_7$ sont tels que définis pour un composé de formule (I).

SCHEMA 3

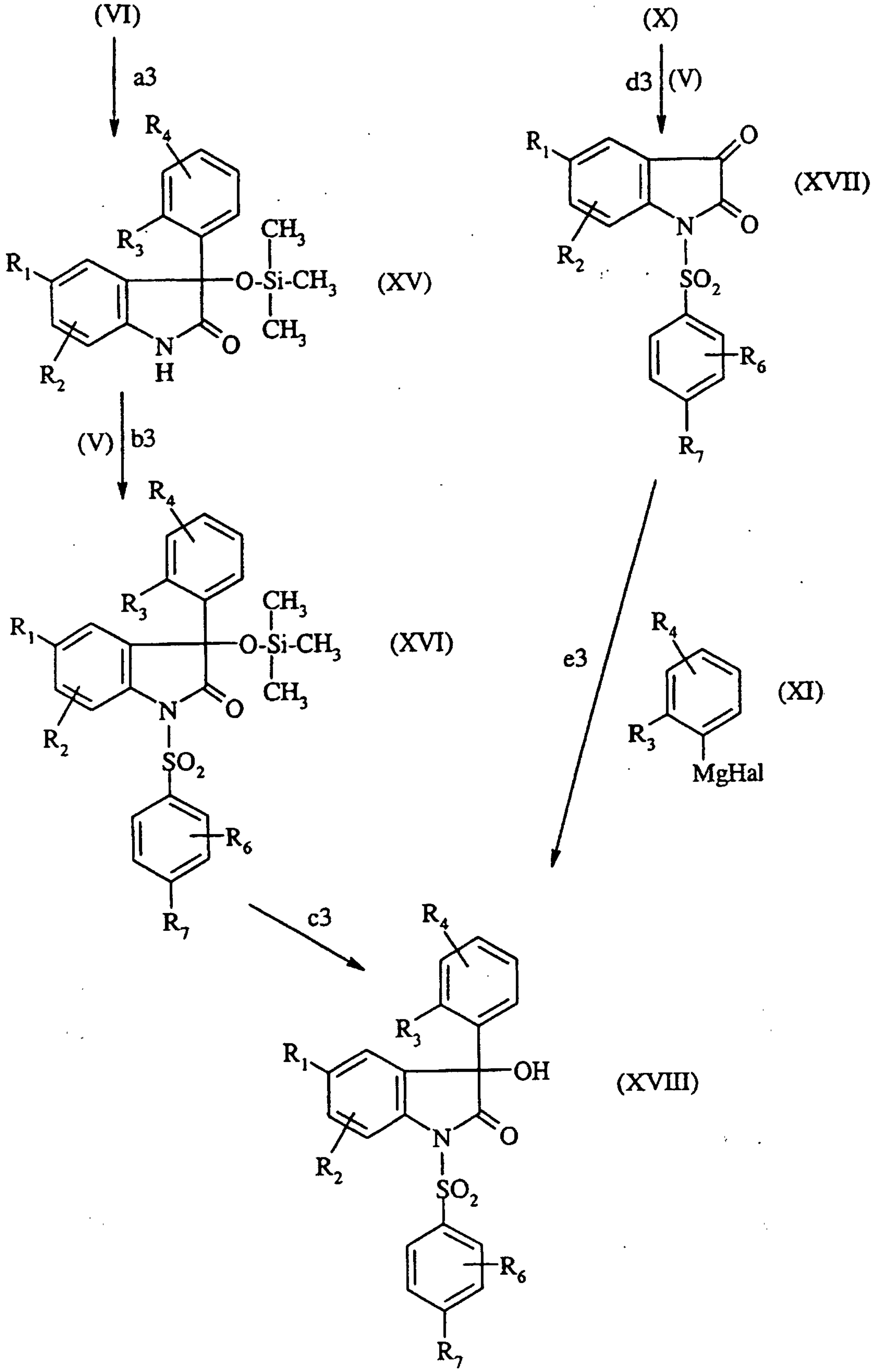


(VI) (X) (XV) (XVII) (XVI) (XI) (XVIII)

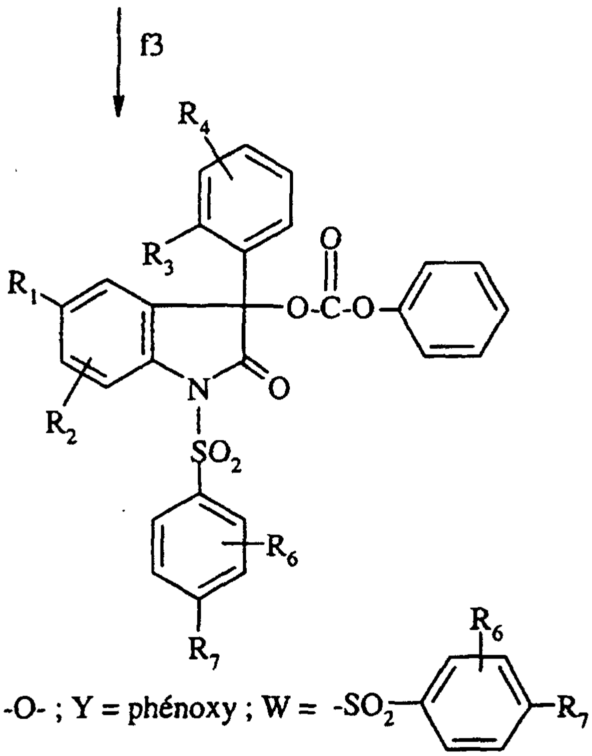


(II) : X = -O- ; Y = phénoxy ; W = $-SO_2-$

A l'étape a3 du SCHEMA 3, on protège sélectivement l'hydroxy d'un composé de formule (VI) en utilisant par exemple l'hexaméthyldisilazane selon la méthode décrite dans Synthetic Communications, 1993, 23 (12), 1633-1641.

Le composé de formule (XV) ainsi obtenu est mis en réaction, à l'étape b3, avec un halogénure de sulfonyle de formule (V), en présence d'une base forte, selon les conditions précédemment décrites.

A l'étape c3, la déprotection du groupe triméthylsilyle du composé de formule (XVI) ainsi obtenu permet d'obtenir le composé de formule (XVIII). La réaction s'effectue par action d'un acide fort tel que l'acide chlorhydrique ou l'acide trifluoroacétique, dans un solvant tel que le dichlorométhane, l'acétone, le tétrahydrofurane, l'eau ou un mélange de ces solvants et à une température comprise entre la température ambiante et la température de reflux du solvant.

On peut également obtenir un composé de formule (XVIII) par réaction à l'étape d3, d'un composé de formule (X) avec un halogénure de sulfonyle de formule (V), selon les conditions précédemment décrites, suivi de la réaction du composé de formule (XVII) ainsi obtenu avec un dérivé organomagnésien de formule (XI) selon les conditions précédemment décrites.

A l'étape f3, on fait réagir le composé de formule (XVIII) ainsi obtenu avec du chloroformiate de phényle, en présence d'une base telle que la pyridine, dans un solvant tel que le dichlorométhane ou sans solvant, à une température comprise entre 0°C et 100°C et obtient le composé de formule (II) attendu.

Le composé de formule (VI) se prépare selon les méthodes précédemment décrites. On peut également préparer un composé de formule (VI) par oxydation par l'air d'un composé de formule (VIII) en présence d'une base telle que l'hydrure de sodium et en présence de diméthyldisulfure.

On peut aussi préparer un composé de formule (VI) par hydrolyse d'un halogénure de formule :

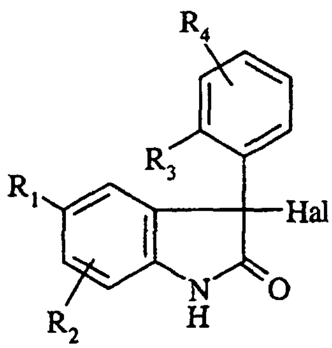

(XIX)

dans laquelle $R_1$, $R_2$, $R_3$ et $R_4$ sont tels que définis pour un composé de formule (I) et Hal représente un atome d'halogène, de préférence le brome ou le chlore. La réaction s'effectue dans un solvant tel que le tétrahydrofurane et à une température comprise entre la température ambiante et la température de reflux du solvant.

Les composés de formule (XIX) sont connus et se préparent selon des méthodes connues telles que celles décrites dans WO 95/18105.

Les composés de formule (II) dans laquelle -X- représente un groupe -NH-, Y représente un phénoxy et W représente un groupe

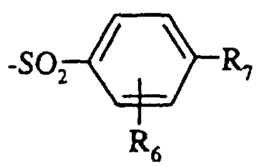

se préparent selon les procédés décrits dans WO 95/18105.

Les composés de formule (II) dans laquelle -X- représente un groupe -O-$CH_2$-, Y représente un hydroxy et W représente l'hydrogène se préparent selon le SCHEMA 4 ci-après dans lequel $R_1$, $R_2$, $R_3$ et $R_4$ sont tels que définis pour une composé de formule (I).

SCHEMA 4

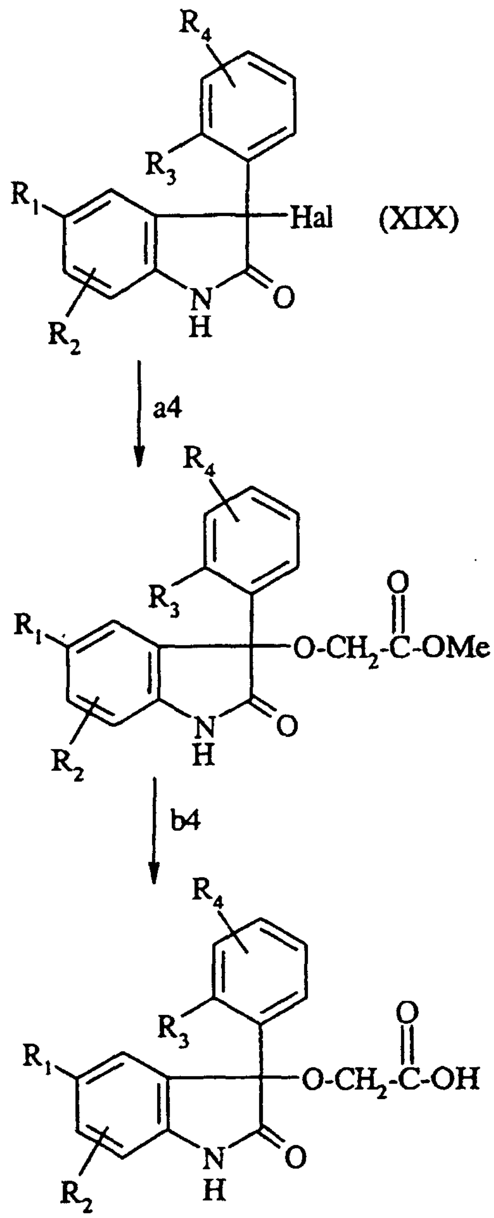


(XIX)

(XX)

(II) : -X- = $-O-CH_2-$ ; Y = OH ; W = H.

A l'étape a4 du SCHEMA 4, on fait réagir un composé de formule (XIX) avec du glycolate de méthyle, en présence d'une base forte telle que l'hydrure de sodium, dans un solvant tel que le tétrahydrofurane ou le dichlorométhane et à une température comprise entre 0°C et la température de reflux du solvant.

Le composé de formule (XX) ainsi obtenu est hydrolysé à l'étape b4 en milieu alcalin selon les méthodes précédemment décrites à l'étape d1 du SCHEMA 1 et on obtient le composé de formule (II) attendu.

Les composés de formule (II) dans laquelle X = $-O-CH_2-$, Y représente un atome de chlore et W = H se préparent à partir des composés de formule (II) correspondants dans laquelle Y = OH par réaction avec du chlorure de thionyle selon la méthode précédemment citée.

Les composés de formule (II) dans laquelle -X- représente un groupe $-NH-CH_2-$,
Y représente un hydroxy et W représente un groupe

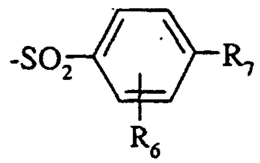

se préparent selon le SCHEMA 5 ci-après dans lequel $R_1$, $R_2$, $R_3$, $R_4$, $R_6$ et $R_7$ sont tels que définis pour un composé de formule (I).

SCHEMA 5

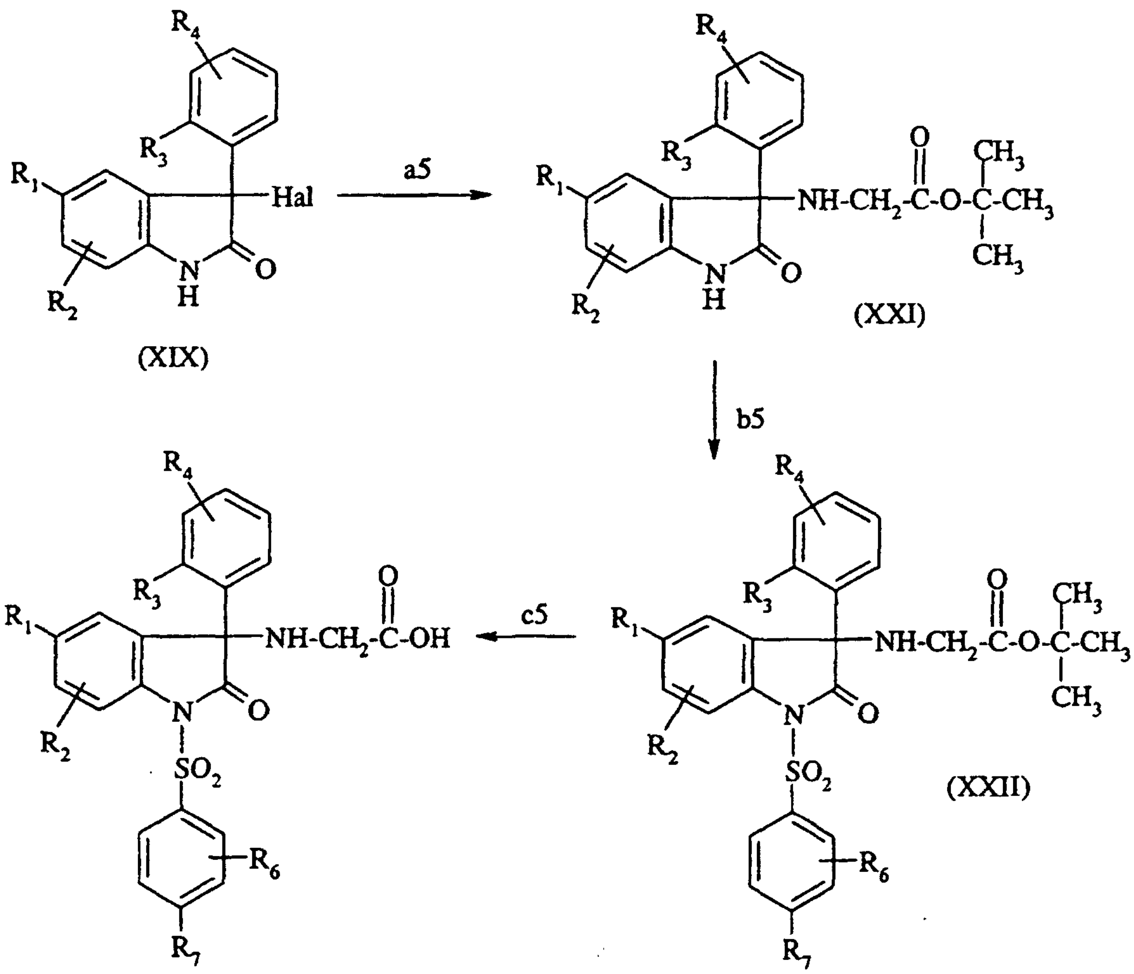


(XIX)

(XXI)

(XXII)

(II) : X = $-NH-CH_2-$ ; Y = OH ; W = 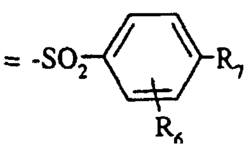

A l'étape a5 du SCHEMA 5, on fait réagir un composé de formule (XIX) avec l'ester *tert*-butylique de la glycine, en présence d'une base telle que la triéthylamine ou la N,N-diisopropyléthylamine, dans un solvant tel que le dichlorométhane, le chloroforme, le tétrahydrofurane ou un mélange de ces solvants et à une température comprise entre 0°C et la température ambiante.

Le composé de formule (XXI) ainsi obtenu est mis en réaction à l'étape b5, avec un halogénure de sulfonyle de formule (V), en présence d'une base forte, selon les conditions précédemment décrites.

A l'étape c5, le composé de formule (XXII) ainsi obtenu est hydrolysé en milieu acide en utilisant un acide fort tel que l'acide chlorhydrique ou l'acide trifluoroacétique, dans un solvant tel que le dichlorométhane, le tétrahydrofurane, l'acétone, l'eau, un mélange de ces solvants ou sans solvant, et à une température comprise entre 0°C et la température ambiante. On obtient ainsi le composé de formule (II) attendu.

Les composés de formule (II) dans laquelle X = $-NH-CH_2-$, Y représente un atome de chlore et

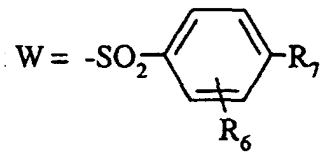

se préparent à partir des composés de formule (II) correspondants dans laquelle Y = OH selon les méthodes précédemment décrites.

Les composés de formule (I) dans laquelle -X- représente un groupe $-NH-CH_2-CH_2-$, Y représente un hydroxy et W représente un groupe

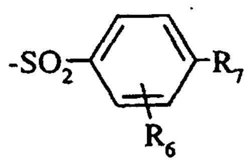

se préparent selon le SCHEMA 6 ci-après dans lequel

$R_1$, $R_2$, $R_3$, $R_4$, $R_6$ et $R_7$ sont tels que définis pour une composé de formule (I).

SCHEMA 6

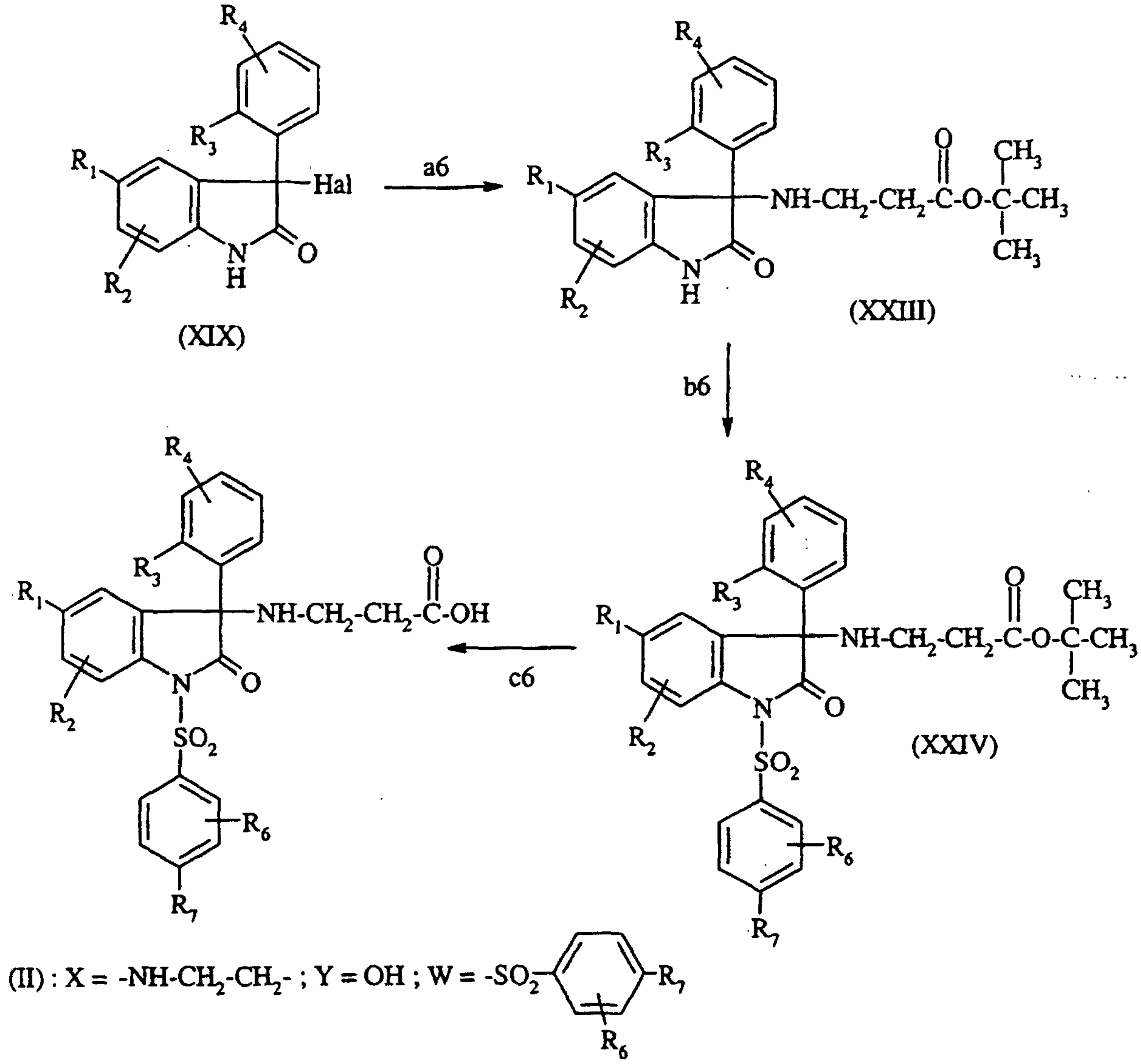


(XIX)

(XXIII)

(XXIV)

(II) : X = $-NH-CH_2-CH_2-$ ; Y = OH ; W = $-SO_2-$

A l'étape a6 du SCHEMA 6, on fait réagir un composé de formule (XIX) avec l'ester *tert*-butylique de la β-alanine, en présence d'une base telle que la triéthylamine ou la N,N-diisopropyléthylamine, dans un solvant tel que le dichlorométhane, le chloroforme, le tétrahydrofurane ou un mélange de ces solvants et à une température comprise entre 0°C et la température ambiante.

Le composé de formule (XXIII) ainsi obtenu est mis en réaction, à l'étape b6, avec un halogénure de sulfonyle de formule (V), en présence d'une base forte, selon les conditions précédemment décrites.

A l'étape c6, le composé de formule (XXIV) ainsi obtenu est hydrolysé en milieu acide selon les conditions précédemment décrites à l'étape c5 du SCHEMA 5. On obtient ainsi le composé de formule (II) attendu.

Les composés de formule (II) dans laquelle -X- = $-NH-CH_2-CH_2-$, Y représente un atome de chlore et

W = $-SO_2-$ 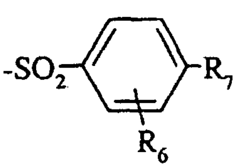

se préparent à partir des composés de formule (II) correspondants dans laquelle Y = -OH selon les méthodes précédemment décrites.

Les composés de formule (III) sont commerciaux ou préparés selon des méthodes connues telles que décrites

dans J. Org. Chem., 1953, 18, 1484-1488, J. Med. Chem., 1978, 21 (6), 536-542, Chem. Pharm. Bull., 1991, 39 (9), 2288-2300, Tetrahedron Letters, 1998, 39, 617-620 ou dans WO 97/28129.

Par exemple, on prépare un composé de formule (III) par réaction d'un composé de formule :

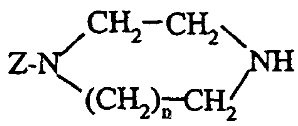

(XXV)

dans laquelle n est tel que défini pour un composé de formule (I) et Z représente l'hydrogène ou un groupe N-protecteur, avec un composé de formule :

$$Hal\text{-}R_5 \quad (XXVI)$$

dans laquelle $R_5$ est tel que défini pour un composé de formule (I) et Hal représente un atome d'halogène, de préférence le chlore, le brome ou l'iode.

La réaction s'effectue en présence ou en l'absence de base, dans un solvant inerte tel que l'éthanol, le propan-2-ol, le n-butanol ou l'acétonitrile et à une température comprise entre 0°C et la température de reflux du solvant. Lorsqu'on utilise une base, celle-ci est choisie parmi les bases organiques telles que la diisopropyléthylamine ou parmi les carbonates de métal alcalin tel que le carbonate de sodium ou de potassium. En l'absence de base, la réaction s'effectue en utilisant un excès du composé de formule (XXV). La réaction peut également s'effectuer sans solvant par chauffage du mélange des composés (XXV) et (XXVI) à des températures de l'ordre de 140°C et 180°C.

Le cas échéant, lorsque Z représente un groupe N-protecteur, on l'élimine selon les méthodes classiques et on obtient les composés de formule (III) attendus.

Les composés de formule (XXV) ou de formule (XXVI) sont connus ou se préparent selon des méthodes connues.

Les composés de formule (V) sont connus ou préparés par des méthodes connues telles que celles décrites dans EP-0 469 984 B et WO 95/18105. Par exemple, les composés de formule (V) peuvent être préparés par halogénation des acides benzènesulfoniques correspondants ou de leurs sels, par exemple de leurs sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant inerte tel qu'un hydrocarbure halogéné ou le N,N-diméthylformamide et à une température comprise entre -10°C et 200°C.

Le chlorure de 2,4-diméthoxybenzènesulfonyle est préparé selon J. Am. Chem. Soc., 1952, 74, 2008. Le chlorure de 3,4-diméthoxybenzènesulfonyle est commercial, ou préparé selon J. Med. Chem., 1977, 20 (10), 1235-1239.

Pour obtenir les composés de formule (I) sous forme d'isomères optiquement purs, on peut utiliser les techniques classiques de séparation : par exemple des recristallisations fractionnées d'un sel formé à partir de la base racémique avec un acide optiquement actif dont le principe est bien connu ou les techniques classiques de chromatographie sur phase chirale.

On peut également préparer les composés de formule (I) optiquement purs à partir d'un composé intermédiaire optiquement pur utile pour la préparation des composés de formule (I).

Ainsi, lorsqu'on veut préparer un composé optiquement pur de formule (I) dans laquelle -X- = $-CH_2-$ on effectue la résolution optique d'un composé de formule (II) dans laquelle -X- = $-CH_2-$, Y = OH et W = H selon le procédé décrit dans J. Am. Chem. Soc., 1989, 111, 7650-7651 en utilisant la (R)-pantolactone comme réactif chiral ou bien en utilisant une amine chirale telle que la (+)-cinchonine.

On peut préparer un composé optiquement pur de formule (I) dans laquelle -X- = -O- selon le procédé décrit dans le SCHEMA 7 ci-après dans lequel $R_1$, $R_2$, $R_3$, $R_4$, n et $R_5$ sont tels que définis pour un composé de formule (I) et R représente un phényle ou un isobutyle.

SCHEMA 7

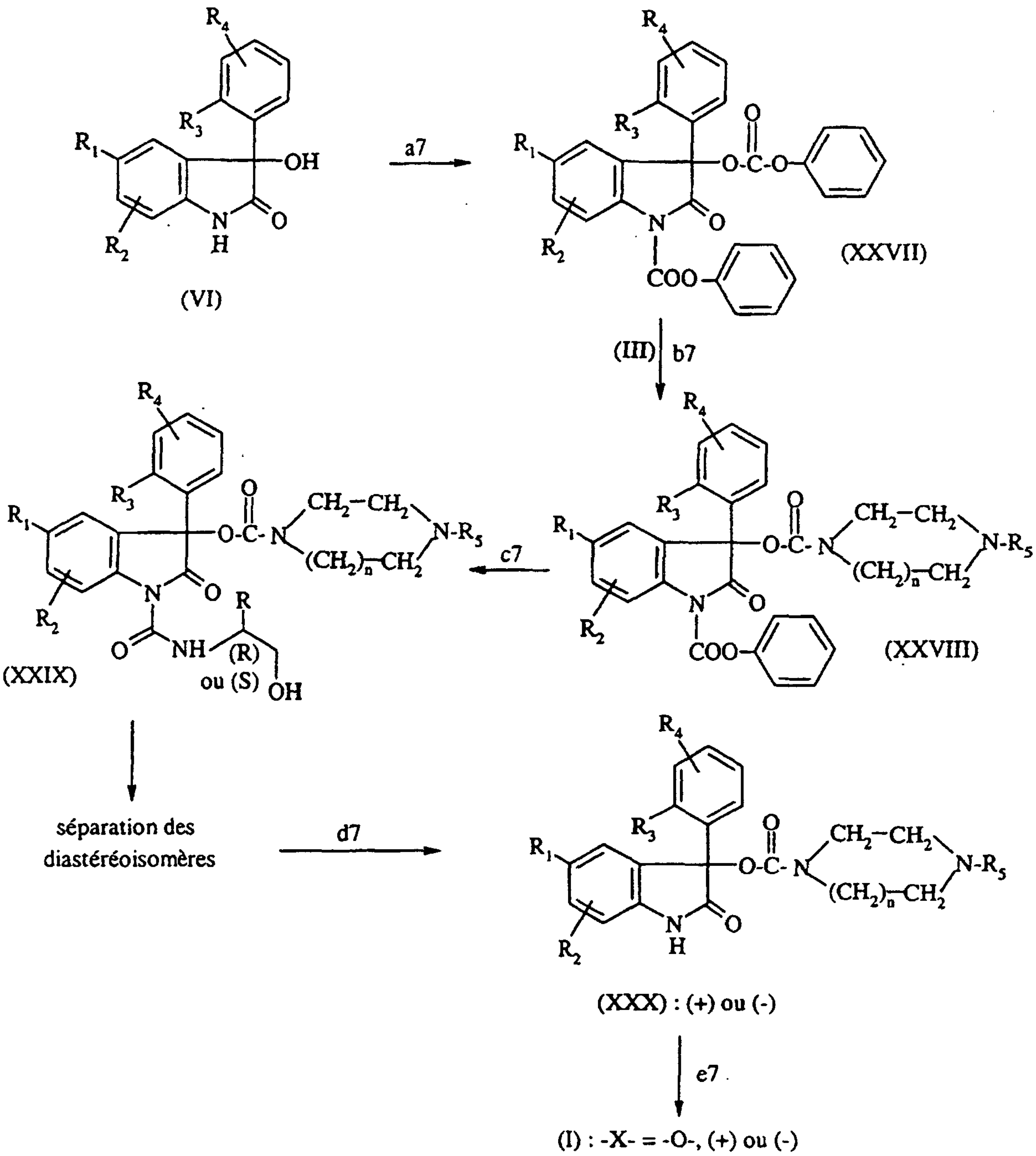


A l'étape a7 du SCHEMA 7, on fait réagir un composé de formule (VI) avec du chloroformiate de phényle, en présence d'une base telle que la pyridine, dans un solvant tel que le dichlorométhane ou sans solvant et à une température comprise entre 0°C et 100°C.

Le composé de formule (XXVII) ainsi obtenu est mis en réaction, à l'étape b7, avec un composé de formule (III), dans un solvant tel que le dichorométhane, le chloroforme ou le tétrahydrofurane ou un mélange de ces solvants, à une température comprise entre la température ambiante et la température de reflux du solvant pour obtenir un composé de formule (XXVIII).

Par réaction du composé (XXVIII) avec le L-leucinol (R = isobutyle) ou le D-Leucinol ou avec le (R)-(-)-α-phénylglycinol (R = phényle) ou le (S)-(+)-α-phénylglycinol, on obtient, à l'étape c7, un mélange de diastéréoisomères d'un composé de formule (XXIX) que l'on peut séparer, par exemple, par chromatographie ou cristallisation.

Par réaction, à l'étape d7, d'un des diastéréoisomères du composé de formule (XXIX) avec une base forte telle que le méthylate de sodium, dans un solvant tel que le méthanol ou le tétrahydrofurane ou un mélange de ces solvants, à une température comprise entre 0°C et la température de reflux du solvant, on obtient un composé de

formule (XXX) optiquement pur.

La réaction du composé (XXX) avec un halogénure de sulfonyle de formule (V) selon les méthodes précédemment décrites permet d'obtenir un composé de formule (I) dans laquelle X = -O-, optiquement pur.

Lorsqu'on veut préparer un composé optiquement pur de formule (I) dans laquelle -X- = -NH-, on prépare un composé de formule (II) dans laquelle -X- = -NH-, Y = phénoxy et

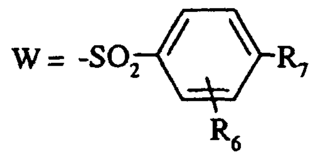

optiquement pur selon le procédé décrit dans le SCHEMA 8 ci-après dans lequel $R_1$, $R_2$, $R_3$, $R_4$, $R_6$ et $R_7$ sont tels que définis pour un composé de formule (I) et Hal représente un atome d'halogène, de préférence le chlore ou le brome.

SCHEMA 8

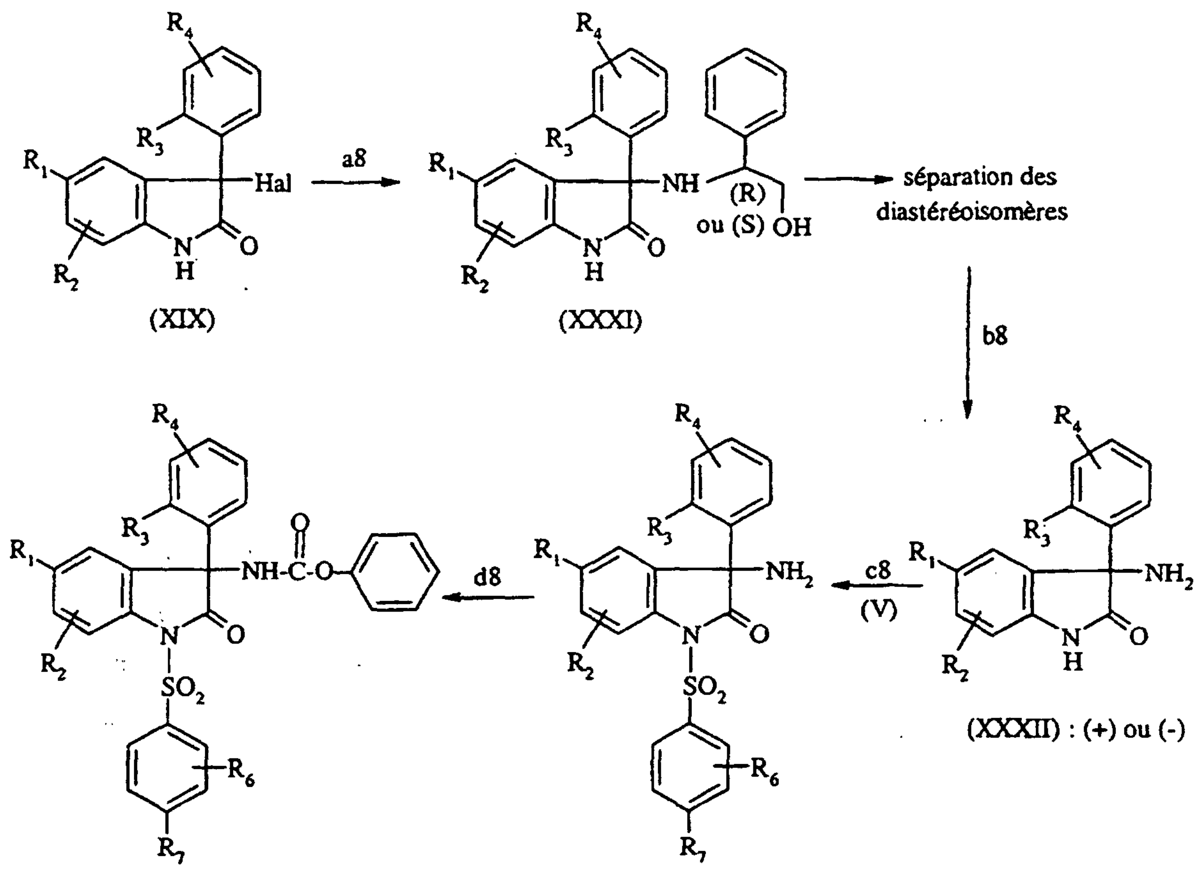


(XIX) (XXXI) (XXXII) : (+) ou (-) (XXXIII) : (+) ou (-) (II) : (+) ou (-)

A l'étape a8 du SCHEMA 8, on fait réagir un composé de formule (XIX) avec le (S)-(+)-α-phénylglycinol ou le (R)-(-)-α-phénylglycinol et obtient un mélange de diastéréoisomères d'un composé de formule (XXXI) que l'on peut séparer par chromatographie ou cristallisation.

A l'étape b8, par oxydation par le tétraacétate de plomb et hydrolyse en milieu acide d'un des diastéréoisomères du composé (XXXI), on obtient un composé de formule (XXXII) optiquement pur.

A l'étape c8, la réaction du composé (XXXII) ainsi obtenu avec un halogénure de sulfonyle de formule (V), suivi de la réaction du composé (XXXIII) ainsi obtenu avec le chloroformiate de phényle (étape d8) selon les méthodes décrites dans WO 95/18105, permettent d'obtenir le composé (II) optiquement pur attendu.

Au cours de l'une quelconque des étapes de préparation des composés de formule (I) ou des composés intermédiaires de formule (II), (III) ou (IV) il peut être nécessaire et/ou souhaitable de protéger les groupes fonctionnels

réactifs ou sensibles, tels que les groupes amine, hydroxyle ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973, dans Protective Groups in Organic Synthesis, T.W. Greene et P.G.M. Wutts, Ed. John Wiley et sons, 1991 ou dans Protecting Groups, Kocienski P.J., 1994, Georg Thieme Verlag. L'élimination des groupes protecteurs peut s'effectuer à une étape ultérieure opportune en utilisant les méthodes connues de l'homme de l'art et qui n'affectent pas le reste de la molécule concernée.

Les groupes N-protecteurs éventuellement utilisés sont les groupes N-protecteurs classiques bien connus de l'homme de l'art tels que par exemple le groupe *tert*-butoxycarbonyle, fluorénylméthoxycarbonyle, benzyle, benzhydrylidène ou benzyloxycarbonyle.

Les composés de formule (IV) sont nouveaux et font partie de l'invention.

Ainsi, selon un autre de ses aspects, l'invention a pour objet des composés de formule :

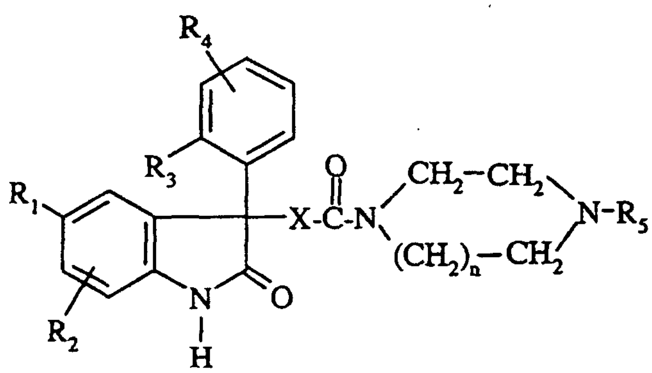 (IV)

dans laquelle :

- n est 1 ou 2 ;
- X représente un groupe $-CH_2-$ ; $-O-CH_2-$ ;
- $R_1$ représente un atome d'halogène ; un $(C_1-C_4)$alkyle ; un $(C_1-C_4)$alcoxy ;
- $R_2$ représente un atome d'hydrogène ; un atome d'halogène ; un $(C_1-C_4)$alkyle ; un $(C_1-C_4)$alcoxy ; un radical trifluorométhyle ;
- $R_3$ représente un atome d'halogène ; un $(C_1-C_3)$alkyle ; un $(C_1-C_3)$alcoxy ; un radical trifluorométhyle ; un radical trifluorométhoxy ;
- $R_4$ représente un atome d'hydrogène ; un atome d'halogène ; un $(C_1-C_3)$alkyle ; un $(C_1-C_3)$alcoxy ;
- $R_5$ représente un radical choisi parmi :

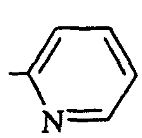 ; 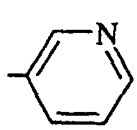 ; 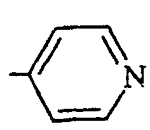 ; 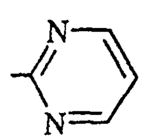 ; 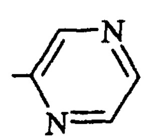 ; 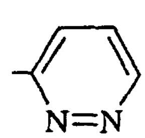 ;

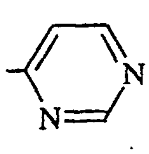 ; 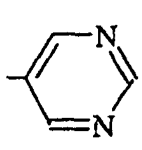 ; 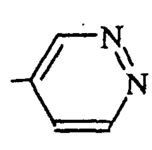 ; 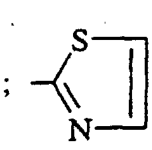 ; 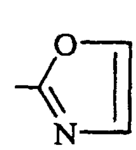 ; 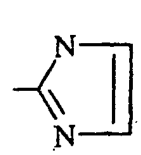

ainsi que leurs sels avec des acides minéraux ou organiques, sous forme d'isomères optiquement purs ou sous forme de mélange.

Les composés de formule (I) ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans

des essais biochimiques en tant que ligand de récepteurs.

Les PREPARATIONS et EXEMPLES suivants illustrent l'invention sans toute fois la limiter.

Dans les Préparations et dans les Exemples on utilise les abréviations suivantes :

éther : éther diéthylique
éther iso : éther diisopropylique
DMF : N,N-diméthylformamide
THF : tétrahydrofurane
DCM : dichlorométhane
AcOEt : acétate d'éthyle
TMEDA : N, N, N', N',- tétraméthylèthylènediamine
DIPEA : diisopropyléthylamine
TFA : acide trifluoroacétique
HMDS : hexaméthyldisilazane
BOP : benzotriazol-1-yloxytris(diméthylamino)phosphonium hexafluorophosphate
PyBOP : benzotriazol-1-yloxytripyrrolidinophosphonium hexafluorophosphate
DCC : 1,3-dicyclohexylcarbodiimide
HOBT : 1-hydroxybenzotriazole hydrate
Ether chlorhydrique : solution saturée d'acide chlorhydrique dans l'éther diéthylique
F : point de fusion
TA : température ambiante
Eb : température d'ébullition
CLHP : chromatographie liquide haute performance.

Les spectres de résonance magnétique du proton (RMN $^1H$) sont enregistrés à 200 MHz dans du DMSO-$d_6$, en utilisant le pic du DMSO-$d_6$ comme référence. Les déplacements chimiques $\delta$ sont exprimés en parties par million (ppm). Les signaux observés sont exprimés ainsi : s: singulet ; se : singulet élargi ; d : doublet ; d.d : doublet dédoublé ; t : triplet ; td : triplet dédoublé ; q : quadruplet ; m : massif ; mt : multiplet.

Les spectres RMN confirment les structures des composés.

PREPARATIONS

Préparations des composés de formule (II).

Préparation 1.1

Acide 2-[5-Chloro-3-(2-méthoxyphényl)-2-oxo-2,3-dihydro-1*H*-indol-3-yl]acétique.

(II) : $R_1$ = Cl ; $R_2$ = H ; $R_3$ = $OCH_3$ ; $R_4$ = H ; X = $-CH_2-$ ; Y = OH ; W = H.

A) 5-Chloro-3-hydroxy-3-(2-méthoxyphényl)-1,3-dihydro-2*H*-indol-2-one.

On prépare ce composé selon le mode opératoire décrit dans WO 95/18105. On prépare une solution de bromure de 2-méthoxyphénylmagnésium à partir de 16 g de magnésium dans 35 ml d'éther et d'une solution de 124 g de 1-bromo-2-méthoxybenzène dans 175 ml d'éther. On ajoute, en goutte à goutte, sous atmosphère d'argon, cette solution à un mélange de 30 g de 5-chloro-1*H*-indole-2,3-dione dans 250 ml de THF, préalablement refroidi au bain de glace, puis laisse sous agitation en laissant remonter la température à TA. Après 1 heure d'agitation à TA, on verse lentement le mélange réactionnel sur une solution saturée de $NH_4Cl$ et évapore le THF sous vide. On essore le précipité formé et le lave à l'éther iso. On obtient 42 g du produit attendu que l'on utilise tel quel à l'étape suivante.

B) 5-Chloro-3-(2-méthoxyphényl)-1,3-dihydro-2*H*-indol-2-one.

On chauffe à reflux pendant 24 heures un mélange de 5,8 g du composé obtenu à l'étape précédente, 25 ml de TFA et 10 ml de triéthylsilane. On concentre sous vide à 100°C le mélange réactionnel, reprend le résidu dans 30 ml d'éther iso et laisse au repos 15 heures à 20°C. On essore le précipité formé et le lave à l'éther iso puis à l'heptane. On obtient 4,3 g du produit attendu.

C) Ester éthylique de l'acide 2-[5-chloro-3-(2-méthoxyphényl)-2-oxo-2,3-dihydro-1*H*-indol-3-yl]acétique.

On chauffe à reflux pendant 20 heures un mélange de 2,85 g du composé obtenu à l'étape précédente, 2,05 g de bromoacétate d'éthyle, 2,05 g de KI et 3,1 g de $K_2CO_3$ dans 15 ml d'acétone. On filtre les sels minéraux et concentre sous vide le filtrat. On extrait le résidu à l'AcOEt, lave la phase organique à l'eau, sèche sur $Na_2SO_4$ et évapore sous vide le solvant. On triture le résidu dans l'éther iso et essore le précipité formé. On chromatographie le précipité sur gel de silice en éluant au DCM puis à l'AcOEt. On obtient 1,55 g du produit attendu après recristallisation dans l'éther iso, F = 184-187°C.

D) Acide 2-[5-Chloro-3-(2-méthoxyphényl)-2-oxo-2,3-dihydro-1*H*-indol-3-yl]acétique.

A un mélange de 1,5 g du composé obtenu à l'étape précédente dans 30 ml de MeOH on ajoute, à TA, une solution de 0,5 g de NaOH en pastilles dans 10 ml d'eau et laisse 20 heures sous agitation à 20°C. On concentre sous vide le mélange réactionnel, reprend le résidu dans 20 ml d'eau, lave la phase aqueuse à l'AcOEt, acidifie la phase aqueuse à pH = 1 par ajout d'HCl concentré et essore le précipité formé. On obtient 1,15 g du produit attendu, F = 135-139°C.

Préparation 1.2

Acide 2-[5-chloro-3-(2-éthoxyphényl)-2-oxo-2,3-dihydro-1*H*-indol-3-yl]acétique.

(II) : $R_1$ = Cl ; $R_2$ = H ; $R_3$ = $OCH_2CH_3$ ; $R_4$ = H ; X = $-CH_2-$ ; Y = OH ; W = H.

A) 1-Bromo-2-éthoxybenzène.

On chauffe 2 heures à reflux un mélange de 17,5 g de 2-bromophénol, 66 ml de sulfate de diéthyle et 170 ml d'une solution de NaOH à 10%. Après refroidissement du mélange réactionnel à TA, on extrait à l'AcOEt, lave la phase organique par une solution de NaOH 2N, sèche sur $Na_2SO_4$ et évapore sous vide le solvant. On obtient 19,6 g du produit attendu.

B) 5-Chloro-3-(2-éthoxyphényl)-3-hydroxy-1,3-dihydro-2*H*-indol-2-one.

On prépare une solution de bromure de 2-éthoxyphénylmagnésium à partir de 2,2 g de magnésium dans 10 ml d'éther et d'une solution de 16,5 g du composé obtenu à l'étape précédente dans 40 ml d'éther. On ajoute, en goutte à goutte et sous atmosphère d'azote, cette solution à un mélange de 5 g de 5-chloro-1*H*-indole-2,3-dione dans 20 ml de THF, en maintenant la température du milieu réactionnel inférieure à 35°C. Après 2 heures d'agitation à TA, on verse le mélange réactionnel sur 200 ml d'HCl 2N, extrait à l'AcOEt, sèche la phase organique sur $Na_2SO_4$ et évapore sous vide les solvants. On reprend le résidu dans l'éther iso à chaud et laisse en cristallisation. On essore le produit cristallisé formé, le lave à l'éther iso et le sèche. On obtient 5,7 g du produit attendu, F = 251°C.

C) 5-Chloro-3-(2-éthoxyphényl)-1,3-dihydro-2*H*-indol-2-one.

On prépare ce composé selon le mode opératoire décrit à l'étape B de la Préparation 1.1 à partir de 4 g du composé obtenu à l'étape précédente, 15 ml de TFA et 5,6 ml de triéthylsilane. On obtient 3,6 g du produit attendu.

D) Ester éthylique de l'acide 2-[5-chloro-3-(2-éthoxyphényl)-2-oxo-2,3-dihydro-1*H*-indol-3-yl]acétique.

On prépare ce composé selon le mode opératoire décrit à l'étape C de la Préparation 1.1 à partir de 3,5 g du composé obtenu à l'étape précédente, 2,2 g de bromoacétate d'éthyle, 2,2 g de KI, 3,5 g de $K_2CO_3$ dans 20 ml d'acétone. On obtient 1,7 g du produit attendu après précipitation dans l'éther iso, F = 181°C.

E) Acide 2-[5-chloro-3-(2-éthoxyphényl)-2-oxo-2,3-dihydro-1*H*-indol-3-yl]acétique.

On laisse 24 heures sous agitation à TA un mélange de 1,65 g du composé obtenu à l'étape précédente et 2 ml d'une solution de NaOH à 30 %, dans 50 ml d'eau et 20 ml de THF. On concentre sous vide, reprend le résidu dans 100 ml d'eau, lave la phase aqueuse à l'AcOEt, acidifie la phase aqueuse à pH = 1 par ajout d'HCl concentré, extrait à l'AcOEt, lave la phase organique à l'eau, sèche sur $Na_2SO_4$ et évapore sous vide le solvant. On obtient 1,2 g du produit attendu après cristallisation dans l'éther iso, F = 212°C.

Préparation 1.3

Acide 2-[5-chloro-3-(2-isopropoxyphényl)-2-oxo-2,3-dihydro-1*H*-indol-3-yl]acétique, isomère dextrogyre.

(II) : $R_1$ = Cl ; $R_2$ = H ; $R_3$ = $OCH(CH_3)_2$ ; $R_4$ = H ; X = $-CH_2-$ ; Y = OH ; W = H.

A) 1-Bromo-2-isopropoxybenzène.

On prépare une solution d'éthylate de sodium à partir de 280 ml d'EtOH et 7,3 g de sodium, ajoute 50 g de 2-bromophénol et laisse 30 minutes sous agitation à TA. On ajoute ensuite 43 g de bromure d'isopropyle et chauffe à reflux pendant 15 heures. On concentre sous vide le mélange réactionnel, reprend le résidu par une solution de NaOH 1N, extrait à l'éther, sèche la phase organique sur $Na_2SO_4$ et évapore sous vide le solvant. On distille sous pression réduite l'huile résultante et obtient 49,3 g du produit attendu sous forme de liquide incolore, Eb = 113-115°C sous 3366 Pa.

B) 5-Chloro-3-hydroxy-3-(2-isopropoxyphényl)-1,3-dihydro-2*H*-indol-2-one.

On prépare une solution de bromure de 2-isopropoxyphénylmagnésium à partir de 6,3 g de magnésium dans 20 ml d'éther et 49,3 g du composé obtenu à l'étape précédente. On ajoute en 3 minutes cette solution à un mélange de 13,3 g de 5-chloro-1*H*-indole-2,3-dione dans 30 ml de THF, puis chauffe à reflux pendant 90 minutes. Après refroidissement à TA, on verse le mélange réactionnel sur un mélange glace/HCl concentré, extrait à l'AcOEt, lave la phase organique par une solution de NaOH 1N, à l'eau, sèche sur $Na_2SO_4$ et évapore sous vide le solvant. On reprend le résidu dans l'éther iso chaud et essore le précipité formé après trituration. On obtient 17,4 g du produit attendu.

C) 5-Chloro-3-(2-isopropoxyphényl)-1,3-dihydro-2*H*-indol-2-one.

On chauffe à reflux pendant 8 heures un mélange de 3,5 g du composé obtenu à l'étape précédente, 15 ml de TFA et 5,5 ml de triéthylsilane. On concentre sous vide, et chromatographie le résidu sur gel de silice en éluant à l'éther iso puis au DCM. On obtient 2,2 g du produit attendu après cristallisation dans l'heptane, F = 154°C.

D) Ester éthylique de l'acide 2-[5-chloro-3-(2-isopropoxyphényl)-2-oxo-2,3-dihydro-1*H*-indol-3-yl]acétique.

On prépare ce composé selon le mode opératoire décrit à l'étape C de la Préparation 1.1 à partir de 2,55 g du composé obtenu à l'étape précédente, 1,67 g de bromoacétate d'éthyle, 1,66 g de KI et 2,52 g de $K_2CO_3$ dans 12 ml d'acétone. On chromatographie le produit obtenu sur alumine en éluant au DCM puis à l'acétone. On obtient 1,6 g du produit attendu après cristallisation dans l'éther iso, F = 193°C.

E) Acide 2-[5-chloro-3-(2-isopropoxyphényl)-2-oxo-2,3-dihydro-1*H*-indol-3-yl]acétique.

A une solution de 1,6 g du composé obtenu à l'étape précédente dans 10 ml d'EtOH, on ajoute, à 40°C, une solution de 1 g de NaOH en pastilles dans 10 ml d'eau et laisse 20 heures sous agitation à 20°C. On concentre sous vide, reprend le résidu dans 30 ml d'eau, chauffe à 40°C et filtre un insoluble. On acidifie le filtrat à pH = 1 par ajout d'HCl concentré, essore le précipité formé et le sèche. On obtient 1,15 g du produit attendu, F = 146-148°C.

F) 2-[5-Chloro-3-(2-isopropoxyphényl)-2-oxo-2,3-dihydro-1*H*-indol-3-yl]acétate de (3R)-4,4-diméthyl-2-oxotétrahydro-3-furanyle, isomère le plus polaire.

On laisse 20 heures sous agitation à 20°C un mélange de 2 g du composé obtenu à l'étape précédente et 10 ml de chlorure de thionyle. On concentre sous vide, reprend le résidu au DCM, concentre sous vide le solvant à TA et obtient 2 g du chlorure d'acide. On dissout le chlorure d'acide dans 20 ml de DCM, ajoute cette solution à un mélange de 0,8 g de (R)-pantolactone et 1 g de N-méthylpyrrolidine dans 10 ml de DCM préalablement refroidi à 10°C et laisse 2 heures sous agitation à TA. On concentre sous vide le mélange réactionnel, reprend le résidu à l'eau, extrait à l'AcOEt, lave la phase organique à l'eau, sèche sur $Na_2SO_4$ et évapore sous vide le solvant. On chromatographie le résidu sur gel de silice H en éluant au DCM. On sépare les diastéréoisomères et recueille le composé le plus polaire. On obtient 0,6 g du produit attendu.

G) Acide 2-[5-chloro-3-(2-isopropoxyphényl)-2-oxo-2,3-dihydro-1*H*-indol-3-yl]acétique, isomère dextrogyre.

A un mélange de 0,8 g du composé obtenu à l'étape précédente dans 30 ml de MeOH, on ajoute une solution de 0,8 g d'hydroxyde de lithium monohydrate dans 10 ml d'eau et laisse 20 heures sous agitation à 20°C. On concentre sous vide le mélange réactionnel, extrait le résidu à l'eau, lave la phase aqueuse à l'éther, acidifie la phase aqueuse à pH = 1 par ajout d'HCl concentré, extrait au DCM, sèche la phase organique sur $Na_2SO_4$ et évapore sous vide le solvant. On obtient 0,5 g du produit attendu.
$\alpha_D^{20}$ = + 84° (c = 0,25 ; chloroforme).

Préparation 1.4

Acide 2-[3-(2-chlorophényl)-5-méthyl-2-oxo-2,3-dihydro-1*H*-indol-3-yl]acétique.

(II) : $R_1 = CH_3$ ; $R_2 = H$ ; $R_3 = Cl$ ; $R_4 = H$ ; X = $-CH_2-$ ; Y = OH ; W = H.

A) 3-(2-Chlorophényl)-5-méthyl-1,3-dihydro-2*H*-indol-2-one.

On prépare ce composé selon le mode opératoire décrit dans WO 95/18105 aux étapes A et B de la Préparation 65.

B) Ester méthylique de l'acide 2-[3-(2-chlorophényl)-5-méthyl-2-oxo-2,3-dihydro-1*H*-indol-3-yl]acétique.

On refroidit à une température inférieure à 10°C un mélange de 5 g du composé obtenu à l'étape précédente dans 10 ml de DMF, ajoute, par petites fractions 0,87 g d'hydrure de sodium à 60 % dans l'huile et laisse 30 minutes sous agitation. On ajoute ensuite 3,15 ml de bromoacétate de méthyle, laisse 4 heures sous agitation à TA, puis rajoute 1 ml de bromoacétate de méthyle et laisse 16 heures sous agitation à TA. On verse le mélange réactionnel dans l'eau, extrait à l'AcOEt, lave la phase organique à l'eau, sèche sur $Na_2SO_4$ et évapore sous vide le solvant. On chromatographie le résidu sur gel de silice en éluant au DCM puis par le mélange DCM/AcOEt (95/5 ; v/v). On obtient 0,57 g du produit attendu.

C) Acide 2-[3-(2-chlorophényl)-5-méthyl-2-oxo-2,3-dihydro-1*H*-indol-3-yl]acétique.

A un mélange de 0,57 g du composé obtenu à l'étape précédente dans 10 ml de dioxane, on ajoute une solution de 0,16 g de NaOH en pastilles dans 40 ml d'eau et laisse 24 heures sous agitation à TA. On ajoute 50 ml d'eau au mélange réactionnel, lave la phase aqueuse à l'AcOEt, acidifie la phase aqueuse à pH = 1 par ajout d'HCl 1N, extrait à l'AcOEt, sèche la phase organique sur $Na_2SO_4$ et évapore sous vide le solvant. On chromatographie le résidu sur gel de silice en éluant par le mélange DCM/AcOEt (95/5 ; v/v). On obtient 0,27 g du produit attendu.

Préparation 1.5

Acide 2-[5,6-dichloro-3-(2-méthoxyphényl)-2-oxo-2,3-dihydro-1*H*-indol-3-yl]acétique.

(II) : $R_1 = Cl$ ; $R_2 = 6\text{-}Cl$ ; $R_3 = OCH_3$ ; $R_4 = H$ ; X = $-CH_2-$ ; Y = OH ; W = H.

A) 5,6-Dichloro-1*H*-indole-2,3-dione.

On prépare ce composé selon le mode opératoire décrit dans J. Am. Chem. Soc., 1946, 68, 2697-2703 ou selon le mode opératoire décrit dans J. Org. Chem., 1952, 17, 149-156.

B) 5,6-Dichloro-3-hydroxy-3-(2-méthoxyphényl)-1,3-dihydro-2*H*-indol-2-one.

A une suspension de 1,1 g de magnésium dans 20 ml d'éther contenant quelques cristaux d'iode, on ajoute, en goutte à goutte, 7,5 g de 1-bromo-2-méthoxybenzène, en maintenant le reflux lorsque celui-ci a démarré. A la fin de l'addition on chauffe 2 heures à reflux. On ajoute ensuite une suspension de 4,3 g de 5,6-dichloro-1*H*-indole-2,3-dione dans 20 ml de THF et chauffe à reflux pendant 30 minutes. Après refroidissement à TA, on verse le mélange réactionnel sur un mélange eau/glace/HCl concentré, extrait à l'AcOEt, sèche la phase organique sur $Na_2SO_4$ et évapore sous vide le solvant. On triture le résidu dans l'éther iso à chaud, essore le précipité formé et le lave à l'éther. On obtient 5,2 g du produit attendu, F = 245-246°C.

C) 5,6-Dichloro-3-(2-méthoxyphényl)-1,3-dihydro-2*H*-indol-2-one.

On chauffe à reflux pendant 15 heures un mélange de 5,1 g du composé obtenu à l'étape précédente, 25 ml de TFA et 10 ml de triéthylsilane. On concentre sous vide, triture le résidu dans l'heptane et essore le précipité formé. On obtient 4,8 g du produit attendu après cristallisation dans l'éther iso, F = 195-197°C.

D) Ester éthylique de l'acide 2-[5,6-dichloro-3-(2-méthoxyphényl)-2-oxo-2,3-dihydro-1*H*-indol-3-yl]acétique.

On chauffe à reflux pendant 16 heures un mélange de 4,8 g du composé obtenu à l'étape précédente, 2,7 g de bromoacétate d'éthyle, 2,7 g de KI et 4,4 g de $K_2CO_3$ dans 25 ml d'acétone. On filtre les sels minéraux et concentre sous vide le filtrat. On chromatographie le résidu sur gel de silice eh éluant au DCM puis à l'AcOEt. On obtient 2,6 g du produit attendu après cristallisation dans l'éther iso, F = 214-219°C.

E) Acide 2-[5,6-dichloro-3-(2-méthoxyphényl)-2-oxo-2,3-dihydro-1*H*-indol-3-yl]acétique.

A un mélange de 2,5 g du composé obtenu à l'étape précédente dans 50 ml de MeOH, on ajoute 3 ml d'une solution de NaOH 2N et laisse 48 heures sous agitation à 20°C. On concentre sous vide, reprend le résidu dans 30 ml d'eau, filtre un insoluble, acidifie le filtrat à pH = 1 par ajout d'HCl concentré, extrait à l'AcOEt, lave la phase organique à l'eau, sèche sur $Na_2SO_4$ et évapore sous vide le solvant. On obtient 2 g du produit attendu après cristallisation dans l'éther iso, F = 222-224°C.

Préparation 1.6

Acide 2-[5-chloro-3-(2-méthoxyphényl)-6-méthyl-2-oxo-2,3-dihydro-1*H*-indol-3-yl]acétique.

(II) : $R_1$ = Cl ; $R_2$ = 6-$CH_3$ ; $R_3$ = $OCH_3$ ; $R_4$ = H ; X = -$CH_2$- ; Y = OH ; W = H.

A) 2-(2-Méthoxyphényl)-2-oxoacétate d'éthyle.

On refroidit à -70°C, sous atmosphère d'argon, une solution de 27 g de 1-bromo-2-méthoxybenzène dans 270 ml d'éther, ajoute, goutte à goutte, 90 ml d'une solution 1,6 M de n-butyllithium dans le pentane, puis laisse 45 minutes sous agitation. On ajoute rapidement 78 ml d'oxalate de diéthyle et laisse sous agitation en laissant remonter la température à TA. Après 1 heure d'agitation à TA, on ajoute au mélange réactionnel une solution saturée de $NH_4Cl$, décante, extrait la phase aqueuse à l'éther, lave les phases organiques jointes à l'eau, par une solution saturée de NaCl, sèche sur $Na_2SO_4$ et évapore les solvants sous vide. On élimine l'oxalate de diéthyle en excès par distillation sous vide (Eb = 87°C sous 2000 Pa). On chromatographie le produit résultant sur gel de silice en éluant par le mélange DCM/hexane (50/50 ; v/v) puis au DCM. Le produit obtenu est purifié par distillation sous vide. On obtient 13 g du produit attendu, Eb = 110°C sous 3 Pa.

B) 5-Chloro-3-hydroxy-3-(2-méthoxyphényl)-6-méthyl-1,3-dihydro-2*H*-indol-2-one.

a) 4-Chloro-3-méthylphénylcarbamate de *tert*-butyle.
On laisse 24 heures sous agitation à TA un mélange de 10 g de 4-chloro-3-méthylaniline et 15,26 g de di-*tert*-butyldicarbonate dans 50 ml de dioxane. On concentre sous vide le mélange réactionnel et chromatographie le résidu sur gel de silice en éluant par le gradient du mélange DCM/hexane de (50/50 ; v/v) à (70/30 ; v/v). On obtient 5,6 g du produit attendu que l'on utilise tel quel.
b) On refroidit à -70°C, sous atmosphère d'argon, une solution de 5 g de 4-chloro-3-méthylphénylcarbamate de *tert*-butyle dans 45 ml d'éther, ajoute, goutte à goutte, 30 ml d'une solution 1,5M de *tert*-butyllithium dans le pentane, laisse 1 heure sous agitation en faisant remonter la température à -10°C et laisse 1 heure 45 minutes sous agitation à -10°C. On refroidit le mélange réactionnel à -70°C, ajoute, goutte à goutte, une solution de 5 g du composé obtenu à l'étape A dans 25 ml de THF, laisse 1 heure sous agitation en laissant remonter la température à -30°C puis une nuit en laissant remonter la température à TA. On ajoute une solution saturée de $NH_4Cl$ au mélange réactionnel, évapore le THF, extrait trois fois la phase aqueuse résultante à l'AcOEt, lave la phase organique à l'eau, par une solution saturée de NaCl, sèche sur $Na_2SO_4$, évapore partiellement le solvant et essore le produit cristallisé. On obtient 2,6 g du produit attendu, F = 254-256°C.

C) 5-Chloro-3-(2-méthoxyphényl)-6-méthyl-1,3-dihydro-2*H*-indol-2-one.

On chauffe à reflux pendant 15 heures un mélange de 3 g du composé obtenu à l'étape précédente, 15 ml de TFA et 6 ml de triéthylsilane. On concentre sous vide, triture le résidu dans le pentane et obtient 2,85 g du produit attendu, F = 193°C.

D) Ester éthylique de l'acide 2-[5-chloro-3-(2-méthoxyphényl)-6-méthyl-2-oxo-2,3-dihydro-1*H*-indol-3-yl]acétique.

On chauffe à reflux pendant 16 heures un mélange de 2,8 g du composé obtenu à l'étape précédente, 1,7 g de bromoacétate d'éthyle, 1,7 g de KI et 2,7 g de $K_2CO_3$ dans 15 ml d'acétone. On filtre les sels minéraux et concentre sous vide le filtrat. On obtient 2,35 g du produit attendu après cristallisation dans l'éther iso, F = 170°C.

E) Acide 2-[5-chloro-3-(2-méthoxyphényl)-6-méthyl-2-oxo-2,3-dihydro-1*H*-indol-3-yl]acétique.

On laisse 20 heures sous agitation à 20°C un mélange de 2,3 g du composé obtenu à l'étape précédente et 3,5 g d'une solution de NaOH 12N dans 100 ml de MeOH et 5 ml d'eau. On concentre sous vide, reprend le résidu dans 30 ml d'eau, acidifie à pH = 1 par ajout d'HCl concentré, extrait à l'AcOEt, lave la phase organique à l'eau, sèche sur $Na_2SO_4$ et évapore sous vide le solvant. On obtient 1,3 g du produit attendu après cristallisation dans l'éther iso, F = 214-216°C.

Préparation 1.7

Acide 2-[5-chloro-3-(2-isopropoxyphényl)-6-méthyl-2-oxo-2,3-dihydro-1*H*-indol-3-yl] acétique.

(II) : $R_1$ = Cl; $R_2$ = 6-$CH_3$ ; $R_3$ = $OCH(CH_3)_2$ ; $R_4$ = H ; X = -$CH_2$- ; Y = OH ; W = H.

A) 2-[2-[(*tert*-butoxycarbonyl)amino]-5-chloro-4-méthylphényl]-2-oxoacétate d'éthyle.

On refroidit à -50°C, sous atmosphère d'azote, une solution de 5,9 g de 4-chloro-3-méthylphénylcarbamate de *tert*-butyle dans 60 ml d'éther, ajoute, goutte à goutte, 42 ml d'une solution de *tert*-butyllithium dans le pentane et laisse 2 heures sous agitation en laissant remonter à -20°C. On refroidit le mélange réactionnel à -70°C, ajoute, goutte à goutte, une solution de 4,5 g d'oxalate de diéthyle dans 30 ml de THF et laisse 1 heure sous agitation en laissant remonter la température à 20°C. On verse le mélange réactionnel sur une solution saturée de $NH_4Cl$, décante, extrait la phase aqueuse à l'AcOEt, lave les phases organiques jointes par HCl 1N, sèche sur $Na_2SO_4$ et évapore sous vide le solvant. On chromatographie le résidu sur gel de silice en éluant par le mélange éther iso/pentane (50/50 ; v/v). On obtient 8 g du produit attendu sous forme d'huile qui cristallise, F = 111°C.

B) 5-Chloro-6-méthyl-1*H*-indole-2,3-dione.

On chauffe à reflux pendant 2 heures un mélange de 8 g du composé obtenu à l'étape précédente et 60 ml d'HCl 3N dans 80 ml de THF. On concentre sous vide le mélange réactionnel, reprend le résidu à l'EtOH et concentre à nouveau sous vide le solvant. On reprend le résidu à l'EtOH, essore le précipité formé et le lave à l'éther iso. On obtient 1,9 g du produit attendu.

C) 5-Chloro-3-hydroxy-3-(2-isopropoxyphényl)-6-méthyl-1,3-dihydro-2*H*-indol-2-one.

A une suspension de 0,36 g de magnésium dans 10 ml d'éther, on ajoute, goutte à goutte, 3,2 g de 1-bromo-2-isopropoxybenzène, en maintenant le reflux lorsque celui-ci a démarré. A la fin de l'addition on chauffe 2 heures à reflux. On ajoute ensuite, en une seule fois, une solution de 1,8 g du composé obtenu à l'étape précédente dans 30 ml de THF et chauffe à reflux pendant 30 minutes. Après refroidissement à TA, on verse le mélange réactionnel sur un mélange glace/HCl concentré, extrait à l'AcOEt, sèche la phase organique sur $Na_2SO_4$ et évapore sous vide le solvant. On triture le résidu dans l'éther iso à chaud, essore le précipité formé et le lave à l'AcOEt chaud. On obtient 1,8 g du produit attendu que l'on utilise tel quel.

D) 5-Chloro-3-(2-isopropoxyphényl)-6-méthyl-1,3-dihydro-2*H*-indol-2-one.

On chauffe 10 heures à reflux un mélange de 2,3 g du composé obtenu à l'étape précédente, 15 ml de TFA et 3,8 ml de triéthylsilane. On concentre sous vide (13 Pa), triture le résidu dans le pentane et essore le précipité formé.

On chromatographie le précipité sur gel de silice en éluant au DCM. On obtient 0,85 g du produit attendu, F = 145-146°C.

E) Ester éthylique de l'acide 2-[5-chloro-3-(2-isopropoxyphényl)-6-méthyl-2-oxo-2,3-dihydro-1*H*-indol-3-yl]acétique.

On chauffe à reflux pendant 15 heures un mélange de 0,8 g du composé obtenu à l'étape précédente, 0,53 g de bromoacétate d'éthyle, 0,53 g de KI et 0,8 g de $K_2CO_3$ dans 6 ml d'acétone. On filtre les sels minéraux, concentre sous vide le filtrat, extrait le résidu au DCM, lave la phase organique à l'eau et filtre un insoluble. On chromatographie le filtrat sur gel de silice en éluant au DCM puis par le mélange DCM/AcOEt (70/30 ; v/v). On obtient 0,55 g du produit attendu, F = 152-154°C.

F) Acide 2-[5-chloro-3-(2-isopropoxyphényl)-6-méthyl-2-oxo-2,3-dihydro-1*H*-indol-3-yl]acétique.

On laisse 20 heures sous agitation à 25°C un mélange de 0,53 g du composé obtenu à l'étape précédente et 0,25 g de NaOH en pastilles dans 20 ml de MeOH et 5 ml d'eau. On concentre sous vide, dissout le résidu dans 20 ml d'eau, acidifie à pH = 1 par ajout d'HCl concentré et essore le précipité formé. On obtient 0,45 g du produit attendu après cristallisation dans le mélange éther iso/pentane (50/50 ; v/v), F = 148-150°C.

Préparation 1.8

5-Chloro-1-[(2,4-diméthoxyphényl)sulfonyl]-3-(2-méthoxyphényl)-2-oxo-2,3-dihydro-1*H*-indol-3-yl phényl carbonate.

(II) : $R_1$ = Cl ; $R_2$ = H ; $R_3$ = $OCH_3$ ; $R_4$ = H ; X = -O- ;

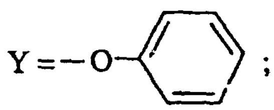

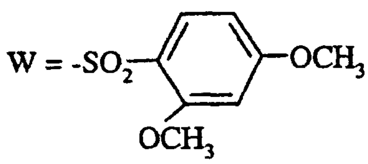

A) 5-Chloro-3-(2-méthoxyphényl)-3-[(triméthylsilyl)oxy]-1,3-dihydro-2*H*-indol-2-one.

On chauffe à reflux un mélange de 2,9 g du composé obtenu à l'étape A de la Préparation 1.1 et 0,16 g de chlorure de zinc anhydre dans 40 ml d'acétonitrile, ajoute 1,7 g d'HMDS et chauffe à reflux pendant 1 heure. On concentre sous vide, extrait le résidu à l'AcOEt, lave la phase organique à l'eau, sèche sur $Na_2SO_4$ et évapore sous vide le solvant. On obtient 2,4 g du produit attendu après cristallisation dans l'éther iso, F = 185-187°C.

B) 5-Chloro-1-[(2,4-diméthoxyphényl)sulfonyl]-3-(2-méthoxyphényl)-3-[(triméthylsilyl)oxy]-1,3-dihydro-2*H*-indol-2-one.

A un mélange de 2,4 g du composé obtenu à l'étape précédente dans 30 ml de THF, on ajoute 0,17 g d'hydrure de sodium à 60 % dans l'huile et laisse 20 minutes sous agitation à TA. On ajoute ensuite 1,7 g de chlorure de 2,4-diméthoxybenzène sulfonyle et laisse 1 heure sous agitation à TA. On concentre sous vide, extrait le résidu à l'AcOEt, lave la phase organique à l'eau, sèche sur $Na_2SO_4$ et évapore sous vide le solvant. On obtient 2,65 g du produit attendu après cristallisation dans l'éther iso, F = 157-158°C.

C) 5-Chloro-1-[(2,4-diméthoxyphényl)sulfonyl]-3-hydroxy-3-(2-méthoxyphényl)-1,3-dihydro-2*H*-indol-2-one.

On laisse 4 heures sous agitation à 20°C un mélange de 2,4 g du composé obtenu à l'étape précédente et 10 ml de TFA dans 20 ml de DCM. On concentre sous vide, extrait le résidu à l'AcOEt, lave la phase organique par une solution à 10 % de $Na_2CO_3$, sèche sur $Na_2SO_4$ et évapore sous vide le solvant. On obtient 1,75 g du produit attendu après cristallisation dans l'éther iso, F = 153-160°C.

D) 5-Chloro-1-[(2,4-diméthoxyphényl)sulfonyl]-3-(2-méthoxyphényl)-2-oxo-2,3-dihydro-1*H*-indol-3-yl phényl carbonate.

On laisse 3 jours sous agitation à 20°C un mélange de 1,6 g du composé obtenu à l'étape précédente et 1 g de chloroformiate de phényle dans 20 ml de pyridine. On concentre sous vide, extrait le résidu à l'AcOEt, lave la phase organique par une solution à 10 % d'AcOH, sèche sur $Na_2SO_4$ et évapore sous vide le solvant. On chromatographie le résidu sur gel de silice en éluant à l'éther iso. On obtient 1,05 g du produit attendu après cristallisation dans l'éther iso, F = 212-213°C.

Préparation 1.9

5-Chloro-1-[(2,4-diméthoxyphényl)sulfonyl]-3-(2-isopropoxyphényl)-2-oxo-2,3-dihydro-1*H*-indol-3-yl phényl carbonate.

(II) : $R_1$ = Cl ; $R_2$ = H ; $R_3$ = $OCH(CH_3)_2$ ; $R_4$ = H ; X = -O- ;

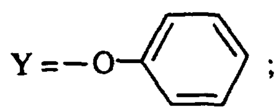

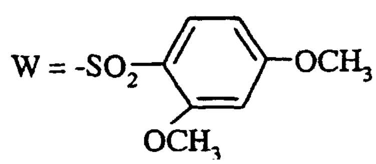

A) 5-Chloro-1-[(2,4-diméthoxyphényl)sulfonyl]-1*H*-indole-2,3-dione.

A un mélange de 3,6 g de 5-chloro-1*H*-indole-2,3-dione dans 20 ml de DMF, on ajoute 0,5 g d'hydrure de sodium à 60 % dans l'huile et laisse 30 minutes sous agitation à 20°C. On ajoute ensuite 4,8 g de chlorure de 2,4-diméthoxybenzènesulfonyle et laisse 1 heure sous agitation à 20°C. On concentre sous vide (1,3 Pa), extrait le résidu à l'AcOEt, lave la phase organique à l'eau, sèche sur $Na_2SO_4$ et évapore sous vide le solvant. On triture le résidu dans l'éther iso et essore le précipité formé. On obtient 2,9 g du produit attendu après cristallisation dans l'AcOEt à chaud, F = 194,5°C.

B) 5-Chloro-1-[(2,4-diméthoxyphényl)sulfonyl]-3-hydroxy-3-(2-isopropoxyphényl)-1,3-dihydro-2*H*-indol-2-one.

On prépare une solution de bromure de 2-isopropoxyphénylmagnésium à partir de 0,5 g de magnésium dans 20 ml de THF et 4 g de 1-bromo-2-isopropoxybenzène. On ajoute en goutte à goutte et à TA, cette solution à un mélange de 4,8 g du composé obtenu à l'étape précédente dans 50 ml de THF puis chauffe à reflux pendant 1 heure. Après refroidissement à TA, on verse le mélange réactionnel sur un mélange glace/HCl 6N, extrait à l'AcOEt, lave la phase organique à l'eau, sèche sur $Na_2SO_4$ et évapore sous vide le solvant. On chromatographie le résidu sur gel de silice en éluant par le mélange DCM/MeOH (99/1 ; v/v). On obtient 3,8 g du produit attendu.

C) 5-Chloro-1-[(2,4-diméthoxyphényl)sulfonyl]-3-(2-isopropoxyphényl)-2-oxo-2,3-dihydro-1*H*-indol-3-yl phényl carbonate.

On laisse 48 heures sous agitation à TA un mélange de 1 g du composé obtenu à l'étape précédente et 1,4 g de chloroformiate de phényle dans 10 ml de pyridine. On concentre sous vide, extrait le résidu à l'AcOEt, lave la phase organique à l'eau, par une solution d'HCl 1N, à l'eau, sèche sur $Na_2SO_4$ et évapore sous vide le solvant. On obtient 0,86 g du produit attendu après cristallisation dans l'éther iso, F = 197°C.

Préparation 1.10

5-Chloro-3-[(2-isopropoxyphényl)-2-oxo-3-[(phénoxycarbonyl)oxy]-1-indolinecarboxylate de phényle.

(XXVII) : $R_1$ = Cl ; $R_2$ = H ; $R_3$ = $OCH(CH_3)_2$ ; $R_4$ = H

On chauffe à 70°C un mélange de 6,34 g du composé obtenu à l'étape B de la Préparation 1.3 dans 60 ml de pyridine, ajoute, en goutte à goutte, 7 g de chloroformate de phényle puis laisse 3 heures sous agitation à 70°C et une nuit à TA. On verse le mélange réactionnel dans l'eau, extrait au DCM, lave la phase organique à l'eau, sèche sur $Na_2SO_4$ et évapore sous vide le solvant. On chromatographie le résidu sur gel de silice en éluant au DCM. On triture le produit obtenu dans l'éther iso et essore le précipité formé. On obtient 8 g du produit attendu.

Préparation 1.11

5-Chloro-3-(2-chlorophényl)-1-[(2,4-diméthoxyphényl)sulfonyl]-2-oxo-2,3-dihydro-1*H*-indol-3-yl phényl carbonate.

(II): $R_1$ = Cl ; $R_2$ = H ; $R_3$ = Cl ; $R_4$ = H ; X = -O- ;

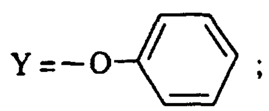

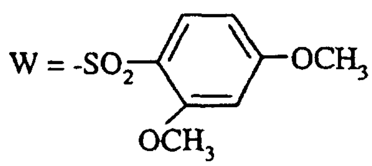

A) 5-Chloro-3-(2-chlorophényl)-1-[(2,4-diméthoxyphényl)sulfonyl]-3-hydroxy-1,3-dihydro-2*H*-indol-2-one.

On prépare une solution de bromure de 2-chlorophénylmagnésium à partir de 0,4 g de magnésium, 0,01 g d'iode, 20 ml d'éther et 2,9 g de 1-bromo-2-chlorobenzène. On ajoute à cette solution au reflux, en 1 minute, un mélange de 2,8 g du composé obtenu à l'étape A de la Préparation 1.9 dans 30 ml de THF et maintient 20 minutes à reflux. Après refroidissement à TA, on verse le mélange réactionnel sur un mélange glace/HCl concentré, extrait à l'AcOEt, lave la phase organique à l'eau, sèche sur $Na_2SO_4$ et évapore sous vide le solvant. On chromatographie le résidu sur gel de silice en éluant à l'éther iso puis au DCM. On obtient 1,55 g du produit attendu sous forme de mousse que l'on utilise telle quelle.

B) 5-Chloro-3-(2-chlorophényl)-1-[(2,4-diméthoxyphényl)sulfonyl]-2-oxo-2,3-dihydro-1*H*-indol-3-yl phényl carbonate.

On laisse 20 heures sous agitation à 20°C un mélange de 0,65 g du composé obtenu à l'étape précédente et 0,3 g de chloroformiate de phényle dans 5 ml de pyridine. On concentre sous vide, extrait le résidu à l'AcOEt, lave la phase organique par une solution d'HCl 1N, par une solution de NaOH 1N, à l'eau, sèche sur $Na_2SO_4$ et évapore sous vide le solvant. On obtient 0,6 g du produit attendu après cristallisation dans l'éther iso, F = 222-223°C.

Préparation 1.12

5-Chloro-1-[(2,4-diméthoxyphényl)sulfonyl]-3-(2-fluorophényl)-2-oxo-2,3-dihydro-1*H*-indol-3-yl phényl carbonate.

(II): $R_1$ = Cl ; $R_2$ = H ; $R_3$ = F ; $R_4$ = H ; X = -O- ;

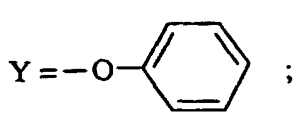

W = 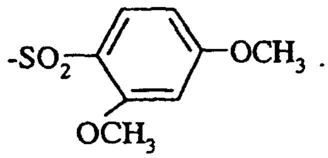

A) Acide DL-2-fluoromandélique.

On prépare ce composé selon le procédé décrit dans J. Org. Chem., 1968,33, 2565-2566. On peut également préparer ce composé en suivant le mode opératoire ci-après. On refroidit à une température inférieure à 10°C, un mélange de 17,4 g de 2-fluorobenzaldéhyde et de 9,6 g de cyanure de potassium dans 30 ml d'éther, ajoute, en 30 minutes, 15 ml d'HCl concentré et laisse 2 heures sous agitation à TA. Après décantation, on sèche la phase organique sur $Na_2SO_4$ et évapore sous vide le solvant. On reprend le produit brut ainsi obtenu dans 20 ml d'HCl concentré et chauffe à reflux pendant 5 heures. Après refroidissement à TA, on extrait le mélange réactionnel à l'éther, lave la phase organique à l'eau, sèche sur $Na_2SO_4$ et évapore sous vide le solvant. On obtient 17,5 g du produit attendu après cristallisation dans l'éther iso.

B) N-p-chlorophényl-DL-2-fluoromandélamide

On chauffe à reflux pendant 3 heures un mélange de 17,5 g du composé obtenu à l'étape précédente et 13 g de p-chloroaniline dans 100 ml de 1,2-dichlorobenzène en éliminant l'eau formée à l'aide d'un appareil de Dean-Stark. Après refroidissement à TA, on laisse en cristallisation. On essore le précipité formé, le dissout dans l'AcOEt, lave deux fois la phase organique par une solution d'HCl 4N, sèche sur $Na_2SO_4$ et évapore sous vide le solvant. On obtient 16,2 g du produit attendu après cristallisation dans l'éther iso.

C) 5-Chloro-3-(2-fluorophényl)-1,3-dihydro-2H-indol-2-one

A un mélange de 64 ml d'$H_2SO_4$ concentré (95%) et 16 ml d'acide sulfurique fumant (oléum à 30%), on ajoute à TA 16,1 g du composé obtenu à l'étape précédente puis laisse 8 heures sous agitation. On verse le mélange réactionnel sur un mélange de glace/eau, extrait à l'AcOEt, lave deux fois la phase organique à l'eau, sèche sur $Na_2SO_4$ et évapore sous vide le solvant. On obtient 12,2 g du produit attendu après cristallisation dans l'éther iso.

D) 3-Bromo-5-chloro-3-(2-fluorophényl)-1,3-dihydro-2*H*-indol-2-one.

A une solution de 4 g du composé obtenu à l'étape précédente dans 100 ml de chloroforme, on ajoute, à TA et lentement, une solution de 0,78 ml de brome dans 20 ml de chloroforme. On concentre sous vide le mélange réactionnel et obtient 4 g du produit attendu après cristallisation dans l'éther iso.

E) 5-Chloro-3-(2-fluorophényl)-5-hydroxy-1,3-dihydro-2*H*-indol-2-one.

On chauffe à reflux pendant 20 heures un mélange de 2 g du composé obtenu à l'étape précédente dans 5 ml d'eau et 20 ml de THF. On concentre sous vide, reprend le résidu par une solution à 10 % de $Na_2CO_3$ et essore le précipité formé. On obtient 1,45 g du produit attendu après cristallisation dans l'éther iso, F = 265°C (déc.).

F) 5-Chloro-3-(2-fluorophényl)-3-[(triméthylsilyl)oxy]-1,3-dihydro-2*H*-indol-2-one.

On chauffe à reflux un mélange de 1,4 g du composé obtenu à l'étape précédente et 0,12 g de chlorure de zinc dans 32 ml d'acétonitrile, ajoute 6 ml d'HMDS et chauffe à reflux pendant 8 heures. On concentre sous vide, extrait le résidu à l'AcOEt, lave la phase organique à l'eau, sèche sur $Na_2SO_4$ et évapore sous vide le solvant. On obtient 1,35 g du produit attendu après cristallisation dans l'heptane, F = 125-127°C.

G) 5-Chloro-1-[(2,4-diméthoxyphényl)sulfonyl]-3-(2-fluorophényl)-3-[(triméthylsilyl)oxy]-1,3-dihydro-2*H*-indol-2-one.

On prépare ce composé selon le mode opératoire décrit à l'étape B de la Préparation 1.8 à partir de 1,3 g du composé obtenu à l'étape précédente, 0,1 g d'hydrure de sodium à 60 % dans l'huile, 15 ml de THF et 1 g de chlorure de 2,4-diméthoxybenzènesulfonyle. On obtient 1,75 g du produit attendu après cristallisation dans l'éther iso, F = 184-186°C.

H) 5-Chloro-1-[(2,4-diméthoxyphényl)sulfonyl]-3-(2-fluorophényl)-3-hydroxy-1,3-dihydro-2*H*-indol-2-one.

On laisse 4 heures sous agitation un mélange de 1,7 g du composé obtenu à l'étape précédente, 2 ml d'HCl 12N et 2 ml d'eau dans 30 ml d'acétone. On concentre sous vide, reprend le résidu au MeOH et concentre à nouveau sous vide. On triture le résidu dans l'éther iso et essore le précipité formé. On obtient 1,3 g du produit attendu, F = 144-146°C.

I) 5-Chloro-1-[(2,4-diméthoxyphényl)sulfonyl]-3-(2-fluorophényl)-2-oxo-2,3-dihydro-1*H*-indol-3-yl phényl carbonate.

A un mélange de 1 g du composé obtenu à l'étape précédente et 0,5 ml de pyridine dans 20 ml de DCM on ajoute, à TA, 0,7 g de chloroformiate de phényle et laisse 48 heures sous agitation. On lave deux fois le mélange réactionnel à l'eau, sèche la phase organique sur $Na_2SO_4$ et évapore sous vide le solvant. On chromatographie le résidu sur gel de silice en éluant par le mélange DCM/MeOH (99,5/0,5 ; v/v). On obtient 0,93 g du produit attendu.

Préparation 1.13

5-Chloro-1-[(2,4-diméthoxyphényl)sulfonyl]-3-(2-trifluorométhylphényl)-2-oxo-2,3-dihydro-1*H*-indol-3-yl phényl carbonate.

(II) : $R_1$ = Cl ; $R_2$ = H ; $R_3$ = $CF_3$ ; $R_4$ = H ; X = -O- ;

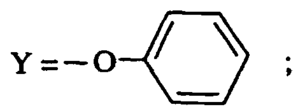

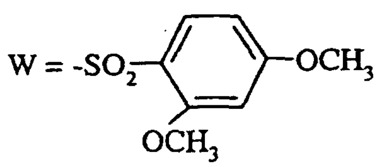

A) 5-Chloro-3-hydroxy-3-(2-trifluorométhylphényl)-1,3-dihydro-2*H*-indol-2-one.

On chauffe à reflux un mélange de 2,3 g de magnésium et 0,01 g d'iode dans 20 ml d'éther, ajoute, en goutte à goutte, une solution de 26 g de 1-bromo-2-trifluorométhyl benzène dans 20 ml d'éther et chauffe à reflux pendant 60 minutes. On ajoute ensuite une solution de 9 g de 5-chloro-1*H*-indole-2,3-dione dans 40 ml de THF et maintient le reflux pendant 30 minutes. Après refroidissement à TA, on verse le mélange réactionnel sur un mélange glace/HCl concentré, extrait à l'éther, lave la phase organique par une solution de NaOH 1N, à l'eau, sèche sur $Na_2SO_4$ et évapore sous vide le solvant. On obtient 5,3 g du produit attendu après cristallisation dans l'heptane, F = 205-208°C.

B) 5-Chloro-3-(2-trifluorométhylphényl)-1,3-dihydro-2*H*-indol-2-one.

On chauffe à reflux un mélange de 3 g du composé obtenu à l'étape précédente et 0,1 g de chlorure de zinc dans 20 ml d'acétonitrile, ajoute 6 ml d'HMDS et chauffe à reflux pendant 1 heure. On concentre sous vide, extrait le résidu à l'éther, lave la phase organique à l'eau, sèche sur $Na_2SO_4$ et évapore sous vide le solvant. On obtient 3,9 g du produit attendu sous forme d'huile qui cristallise, F = 175-176°C.

C) 5-Chloro-1-[(2,4-diméthoxyphényl)sulfonyl]-3-(2-trifluorométhylphényl)-3-[(triméthylsilyl)oxy]-1,3-dihydro-2*H*-indol-2-one.

On prépare ce composé selon le mode opératoire décrit à l'étape B de la Préparation 1.8 à partir de 3,8 g du composé obtenu à l'étape précédente, 0,45 g d'hydrure de sodium à 60 % dans l'huile, 30 ml de THF et 2,5 g de chlorure de 2,4-diméthoxybenzènesulfonyle. On obtient 5 g du produit attendu après cristallisation dans l'heptane, F = 144-145°C.

D) 5-Chloro-1-[(2,4-diméthoxyphényl)sulfonyl]-3-hydroxy-3-(2-trifluorométhyl phényl)-1,3-dihydro-2*H*-indol-2-one.

On laisse 15 heures sous agitation à 20°C un mélange de 4,9 g du composé obtenu à l'étape précédente, 3 ml d'HCl 12N et 5 ml d'eau dans 50 ml de THF. On concentre sous vide à une température inférieure à 40°C, reprend le résidu par 30 ml d'une solution à 10 % de $NaHCO_3$, extrait à l'AcOEt, sèche sur $Na_2SO_4$ et évapore sous vide le solvant. On obtient 3,8 g du produit attendu après cristallisation dans le mélange éther iso/heptane (80/20 ; v/v), F = 143°C.

E) 5-Chloro-1-[(2,4-diméthoxyphényl)sulfonyl]-3-(2-trifluorométhylphényl)-2-oxo-2,3-dihydro-1*H*-indol-3-yl phényl carbonate.

A un mélange de 1,7 g du composé obtenu à l'étape précédente dans 20 ml de pyridine, on ajoute, en goutte à goutte et à 20°C, 2 g de chloroformiate de phényle et laisse 7 heures sous agitation à 20°C. On concentre sous vide, reprend le résidu par 30 ml d'une solution à 10 % d'AcOH, extrait à l'éther, lave la phase organique par une solution à 10 % de $NaHCO_3$, à l'eau, sèche sur $Na_2SO_4$ et évapore sous vide le solvant. On obtient 1,8 g du produit attendu après cristallisation dans l'heptane, F = 191°C.

Préparation 1.14

5-Chloro-1-[(2,4-diméthoxyphényl)sulfonyl]-3-(2-trifluorométhoxyphényl)-2-oxo-2,3-dihydro-1*H*-indol-3-yl phényl carbonate.

(II) : $R_1$ = Cl ; $R_2$= H; $R_3$ =$OCF_3$ ; $R_4$ = H ;X = -O- ;

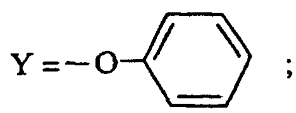

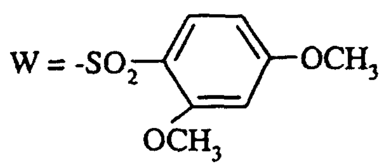

A) 5-Chloro-3-hydroxy-3-(2-trifluorométhoxyphényl)-1,3-dihydro-2*H*-indol-2-one.

A un mélange de 2,8 g de magnésium dans 20 ml d'éther on ajoute, en goutte à goutte, une solution de 25 g de 1-bromo-2-trifluorométhoxybenzène dans 130 ml d'éther, en maintenant le reflux lorsque celui-ci a démarré. A la fin de l'addition on chauffe une heure à reflux. On ajoute ensuite, à une température inférieure à 40°C, un mélange de 7,5 g de 5-chloro-1*H*-indole-2,3-dione dans 100 ml de THF puis chauffe à reflux pendant une heure. Après refroidissement à TA, on verse le mélange réactionnel sur un mélange glace/HCl concentré, extrait à l'AcOEt, lave la phase organique à l'eau par une solution de NaOH 1N, sèche sur $Na_2SO_4$ et évapore sous vide le solvant. On obtient 6,5 g du produit attendu après cristallisation dans le mélange DCM/éther iso (20/80 ; v/v), F = 214°C.

B) 5-Chloro-3-(2-trifluorométhoxyphényl)-3-[(triméthylsilyl)oxy]-1,3-dihydro-2*H*-indol-2-one.

On chauffe 2 heures à reflux un mélange de 2 g du composé obtenu à l'étape précédente, 0,05 g de chlorure de zinc et 1,4 g d'HMDS dans 100 ml d'acétonitrile. On concentre sous vide, extrait le résidu à l'AcOEt, lave la phase organique à l'eau, sèche sur $Na_2SO_4$ et évapore sous vide le solvant. On obtient 2,5 g du produit attendu que l'on utilise tel quel.

C) 5-Chloro-1-[(2,4-diméthoxyphényl)sulfonyl]-3-(2-trifluorométhoxyphényl)-3-[(triméthylsilyl)oxy]-1,3-dihydro-2*H*-indol-2-one.

On prépare ce composé selon le mode opératoire décrit à l'étape B de la Préparation 1.8 à partir de 2,5 g du

composé obtenu à l'étape précédente, 0,3 g d'hydrure de sodium à 60 % dans l'huile, 50 ml de THF et 1,7 g de chlorure de 2,4-diméthoxybenzènesulfonyle. On obtient 2,7 g du produit attendu après cristallisation dans l'éther iso, F = 181°C.

D) 5-Chloro-1-[(2,4-diméthoxyphényl)sulfonyl]-3-hydroxy-3-(2-trifluorométhoxy phényl)-1,3-dihydro-2*H*-indol-2-one.

On laisse 1 heure sous agitation à une température inférieure à 40°C, un mélange de 2,7 g du composé obtenu à l'étape précédente et 1 ml d'HCl concentré dans 50 ml d'acétone. On concentre sous vide, extrait le résidu à l'AcOEt, lave la phase organique à l'eau, sèche sur $Na_2SO_4$ et évapore sous vide le solvant. On obtient 1,66 g du produit attendu après cristallisation dans l'éther iso, F = 81°C.

E) 5-Chloro-1-[(2,4-diméthoxyphényl)sulfonyl]-3-(2-trifluorométhoxyphényl)-2-oxo-2,3-dihydro-1*H*-indol-3-yl phényl carbonate.

A un mélange de 1,6 g du composé obtenu à l'étape précédente et 0,8 ml de pyridine dans 20 ml de DCM, on ajoute, à TA, 1,15 g de chloroformiate de phényle et laisse 1 heure sous agitation à TA. On lave le mélange réactionnel à l'eau, sèche sur $Na_2SO_4$ et évapore sous vide le solvant. On obtient 1,34 g du produit attendu après cristallisation dans l'éther iso, F = 203-204°C.

Préparation 1.15

5-Chloro-1-[(2,4-diméthoxyphényl)sulfonyl]-3-(2-méthoxyphényl)-6-méthyl-2-oxo-2,3-dihydro-1*H*-indol-3-yl phényl carbonate.

(II) : $R_1$ = Cl ; $R_2$ = 6-$CH_3$ ; $R_3$ = $OCH_3$ ; $R_4$ = H ; X = -O- ;

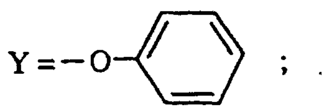

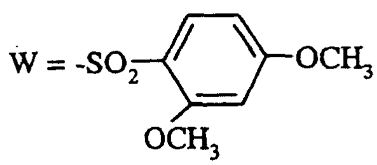

A) 5-Chloro-3-(2-méthoxyphényl)-6-méthyl-3-[(triméthylsilyl)oxy]-1,3-dihydro-2*H*-indol-2-one.

On chauffe à reflux un mélange de 0,65 g du composé obtenu à l'étape B de la Préparation 1.6 et 0,05 g de chlorure de zinc dans 10 ml d'acétonitrile, ajoute 2,1 ml d'HDMS et maintient à reflux pendant 1 heure. On concentre sous vide, extrait le résidu à l'AcOEt, lave la phase organique à l'eau, sèche sur $Na_2SO_4$ et évapore sous vide le solvant. On obtient 0,7 g du produit attendu après cristallisation dans l'heptane, F = 227°C.

B) 5-Chloro-1-[(2,4-diméthoxyphényl)sulfonyl]-3-(2-méthoxyphényl)-6-méthyl-3-[(triméthylsilyl)oxy]-1,3-dihydro-2*H*-indol-2-one.

On prépare ce composé selon le mode opératoire décrit à l'étape B de la Préparation 1.8 à partir de 0,85 g du composé obtenu à l'étape précédente, 0,06 g d'hydrure de sodium à 60 % dans l'huile, 20 ml de THF et 0,6 g de chlorure de 2,4-diméthoxybenzènesulfonyle. On obtient 0,95 g du produit attendu après cristallisation dans l'éther iso, F = 190-191°C.

C) 5-Chloro-1-[(2,4-diméthoxyphényl)sulfonyl]-3-hydroxy-3-(2-méthoxyphényl)-6-méthyl-1,3-dihydro-2*H*-indol-2-one.

A une solution de 0,85 g du composé obtenu à l'étape précédente dans 20 ml d'acétone, on ajoute 0,5 ml d'une solution d'HCl 12 N et laisse 4 heures sous agitation à 20°C. On concentre sous vide, reprend le résidu par 50

ml de DCM et concentre à nouveau sous vide le solvant. On obtient 0,75 g du produit attendu après cristallisation dans l'éther iso, F = 207-208°C.

D) 5-Chloro-1-[(2,4-diméthoxyphényl)sulfonyl]-3-(2-méthoxyphényl)-6-méthyl-2-oxo-2,3-dihydro-1*H*-indol-3-yl phényl carbonate.

A une solution de 0,7 g du composé obtenu à l'étape précédente dans 10 ml de pyridine, on ajoute 1 g de chloroformiate de phényle et laisse 48 heures sous agitation à 20°C. On concentre sous vide, extrait le résidu à l'AcOEt, lave la phase organique par une solution d'HCl 1N, à l'eau, par une solution de NaOH 1N, à l'eau, sèche sur $Na_2SO_4$ et évapore sous vide le solvant. On obtient 0,75 g du produit attendu après cristallisation dans l'éther iso, F = 196°C (déc.).

Préparation 1.16

5-Chloro-3-(2-chlorophényl)-1-[(2,4-diméthoxyphényl)sulfonyl]-6-méthoxy-2-oxo-2,3-dihydro-1*H*-indol-3-yl phényl carbonate.

(II) : $R_1$ = Cl ; $R_2$ = 6-$OCH_3$ ; $R_3$ = Cl ; $R_4$ = H ; X = -O- ;

Y = 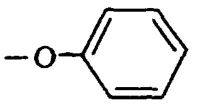 ;

W = 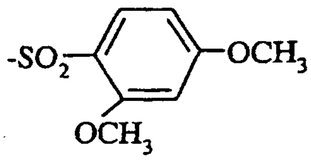

A) 4-Chloro-3-méthoxyaniline.

On hydrogène dans un appareil de Parr pendant 4 heures, à 35°C et sous une pression de 1,3 bars, un mélange de 36 g de 2-chloro-5-nitroanisole et du nickel de Raney® dans 150 ml de MeOH et 200 ml de THF. On filtre le catalyseur sur Célite® et concentre sous vide le filtrat. On obtient 28 g du produit attendu que l'on utilise tel quel.

B) N-(4-chloro-3-méthoxyphényl)-DL-2-chloromandélamide.

On chauffe à 230°C pendant 4 heures un mélange de 28 g du composé obtenu à l'étape précédente et 33,13 g d'acide DL-2-chloromandélique dans 128 ml de 1,2-dichlorobenzène en éliminant l'eau formée à l'aide d'un appareil de Dean-Stark. On concentre sous vide en partie le mélange réactionnel et laisse en cristallisation. On essore le produit cristallisé formé et le lave à l'éther iso. On obtient 40 g du produit attendu.

C) 5-Chloro-3-(2-chlorophényl)-6-méthoxy-1,3-dihydro-2*H*-indol-2-one.

On ajoute rapidement 40 g du composé obtenu à l'étape précédente à 550 g d'acide polyphosphorique puis on chauffe à 60°C pendant 8 heures et laisse une nuit sous agitation en laissant revenir la température à TA. On ajoute de l'eau glacée au mélange réactionnel, essore le précipité formé et le lave à l'eau. On reprend le précipité dans l'AcOEt, essore le produit blanc obtenu après trituration et le lave à l'éther iso. On obtient 17,2 g du produit attendu, F = 243-247°C.

D) 5-Chloro-3-(2-chlorophényl)-3-hydroxy-6-méthoxy-1,3-dihydro-2*H*-indol-2-one,

A une solution de 17,2 g du composé obtenu à l'étape précédente dans 220 ml de THF, on ajoute à TA, sous atmosphère d'argon, 2,56 g d'hydrure de sodium à 60 % dans l'huile. Après cessation du dégagement gazeux, on ajoute 6,85 g de diméthyldisulfure, fait barboter de l'air dans le mélange réactionnel et laisse 72 heures sous agitation à TA. On ajoute de l'eau au mélange réactionnel, évapore sous vide le THF, extrait la phase aqueuse restante à l'AcOEt,

lave la phase organique à l'eau, par une solution saturée de NaCl, sèche sur $Na_2SO_4$ et évapore sous vide le solvant. On dissout le produit obtenu dans du DCM, concentre en partie le solvant, laisse en cristallisation et essore le produit cristallisé formé. On obtient 6 g du produit attendu, F = 237-240°C.

E) 5-Chloro-3-(2-chlorophényl)-6-méthoxy-3-[(triméthylsilyl)oxy]-1,3-dihydro-2*H*-indol-2-one.

On chauffe à reflux un mélange de 1 g du composé obtenu à l'étape précédente et 0,07 g de chlorure de zinc dans 10 ml d'acétonitrile, ajoute 3 ml d'HMDS et maintient le reflux pendant 1 heure. On concentre sous vide, extrait le résidu à l'AcOEt, lave la phase organique à l'eau, sèche sur $Na_2SO_4$ et évapore sous vide le solvant. On obtient 1,25 g du produit attendu après cristallisation dans l'heptane, F = 211-212°C.

F) 5-Chloro-3-(2-chlorophényl)-1-[(2,4-diméthoxyphényl)sulfonyl]-6-méthoxy-3-[(triméthylsilyl)oxy]-1,3-dihydro-2*H*-indol-2-one.

On prépare ce composé selon le mode opératoire décrit à l'étape B de la Préparation 1.8 à partir de 1,2 g du composé obtenu à l'étape précédente, 0,08 g d'hydrure de sodium à 60 % dans l'huile, 15 ml de THF et 0,8 g du chlorure de 2,4-diméthoxybenzènesulfonyle. On obtient 1,6 g du produit attendu après cristallisation dans l'éther iso, F = 217-219°C.

G) 5-Chloro-3-(2-chlorophényl)-1-[(2,4-diméthoxyphényl)sulfonyl]-3-hydroxy-6-méthoxy-1,3-dihydro-2*H*-indol-2-one.

On laisse 4 heures sous agitation à 20°C un mélange de 1,55 g du composé obtenu à l'étape précédente et 2 ml d'HCl 12N dans 30 ml d'acétone. On concentre sous vide, reprend le résidu dans l'acétone et concentre à nouveau sous vide. On reprend le résidu dans du DCM et concentre sous vide. On triture le résidu dans l'éther iso et essore le précipité formé. On obtient 1,3 g du produit attendu, F = 233-235°C.

H) 5-Chloro-3-(2-chlorophényl)-1-[(2,4-diméthoxyphényl)sulfonyl]-6-méthoxy-2-oxo-2,3-dihydro-1*H*-indol—3-yl phényl carbonate.

On refroidit à 10°C un mélange de 1,3 g du composé obtenu à l'étape précédente dans 10 ml de pyridine, ajoute 0,8 g de chloroformiate de phényle et laisse 20 minutes sous agitation à 20°C. On rajoute 0,7 g de chloroformiate de phényle et laisse 20 heures sous agitation à 20°C. On concentre sous vide, extrait le résidu à l'AcOEt, lave la phase organique par une solution d'HCl 1N, sèche sur $Na_2SO_4$ et évapore sous vide le solvant. On chromatographie le résidu sur gel de silice en éluant au DCM. On obtient 0,7 g du produit attendu après cristallisation dans le mélange pentane/éther iso, F = 162-163°C.

Préparation 1.17

5-Chloro-1-[(2,4-diméthoxyphényl)sulfonyl]-3-(2,5-diméthoxyphényl)-2-oxo-2,3-dihydro-1*H*-indol-3-yl phényl carbonate.

(II) : $R_1$ = Cl ; $R_2$ = H ; $R_3$ = $OCH_3$ ; $R_4$ = 5- $OCH_3$ ; X = -O- ;

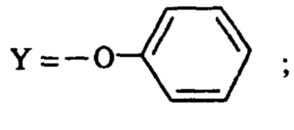

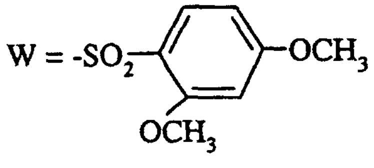

A) 5-Chloro-3-hydroxy-3-(2,5-diméthoxyphényl)-1,3-dihydro-2*H*-indol-2-one.

On prépare une solution de bromure de 2,5-diméthoxyphénylmagnésium à partir de 2,2 g de magnésium, 18 g de 1-bromo-2,5-diméthoxybenzène et 50 ml d'éther. On ajoute, en goutte à goutte, cette solution à un mélange de 5 g de 5-chloro-1*H*-indole-2,3-dione dans 50 ml de THF à une température inférieure à 30°C, puis chauffe 3 heures à reflux. Après refroidissement à TA, on verse le mélange réactionnel sur une solution d'HCl 1N, extrait à l'AcOEt, lave la phase organique à l'eau, sèche sur $Na_2SO_4$ et évapore sous vide le solvant. On obtient 7,1 g du produit attendu après cristallisation dans l'éther iso à chaud.

B) 5-Chloro-3-(2,5-diméthoxyphényl)-3-[(triméthylsilyl)oxy]-1,3-dihydro-2*H*-indol-2-one.

On chauffe à reflux un mélange de 4 g du composé obtenu à l'étape précédente et 0,085 g de chlorure de zinc dans 45 ml d'acétonitrile, ajoute 2,8 ml d'HMDS et maintient à reflux pendant 1 heure. On concentre sous vide, reprend le résidu à l'eau, extrait à l'éther, lave la phase organique à l'eau, sèche sur $Na_2SO_4$ et évapore sous vide le solvant. On obtient 5 g du produit attendu après cristallisation dans l'éther iso.

C) 5-Chloro-3-(2,5-diméthoxyphényl)-1-[(2,4-diméthoxyphényl)sulfonyl]-3-[(triméthylsilyl)oxy]-1,3-dihydro-2*H*-indol-2-one.

On prépare ce composé selon le mode opératoire décrit à l'étape B de la Préparation 1.8 à partir de 2 g du composé obtenu à l'étape précédente, 0,135 g d'hydrure de sodium à 60 % dans l'huile, 40 ml de THF et 1,3 g de chlorure de 2,4-diméthoxybenzènesulfonyle. On obtient 2,2 g du produit attendu après cristallisation dans l'éther iso.

D) 5-Chloro-3-(2,5-diméthoxyphényl)-1-[(2,4-diméthoxyphényl)sulfonyl]-3-hydroxy-1,3-dihydro-2*H*-indol-2-one.

On laisse 4 heures sous agitation à TA un mélange de 1 g du composé obtenu à l'étape précédente et 0,5 ml d'HCl 12N dans 20 ml d'acétone. On concentre sous vide, reprend le résidu au DCM et concentre à nouveau sous vide. On triture le résidu dans l'éther iso et essore le précipité formé. On obtient 0,84 g du produit attendu.

E) 5-Chloro-1-[(2,4-diméthoxyphényl)sulfonyl]-3-(2,5-diméthoxyphényl)-2-oxo-2,3-dihydro-1*H*-indol-3-yl phényl carbonate.

On laisse une nuit sous agitation à TA un mélange de 0,8 g du composé obtenu à l'étape précédente et 0,4 ml de chloroformiate de phényle dans 5 ml de pyridine. On ajoute de l'eau au mélange réactionnel, extrait au DCM, lave la phase organique par une solution d'HCl 2N, sèche sur $Na_2SO_4$ et évapore sous vide le solvant. On obtient 0,84 g du produit attendu après cristallisation dans l'éther iso.

Préparation 1.18

5-Chloro-1-[(2,4-diméthoxyphényl)sulfonyl]-3-(2-méthoxyphényl)-2-oxo-2,3-dihydro-1*H*-indol-3-yl carbamate de phényle.

(II) : $R_1$ = Cl ; $R_2$ = H ; $R_3$ = $OCH_3$ ; $R_4$ = H ; X = -NH- ;

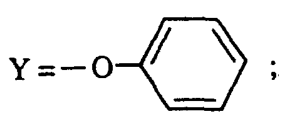

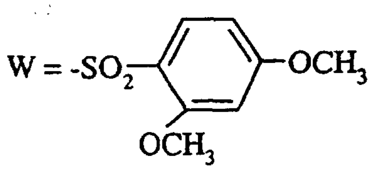

A) 3-Amino-5-chloro-3-(2-méthoxyphényl)-1,3-dihydro-2*H*-indol-2-one.

On prépare ce composé selon le mode opératoire décrit dans WO 95/18105 à la Préparation 7.

B) 3-Amino-5-chloro-1-[(2,4-diméthoxyphényl)sulfonyl]-3-(2-méthoxyphényl)-1,3-dihydro-1*H*-indol-2-one.

On refroidit à 4°C une solution de 1,64 g du composé obtenu à l'étape précédente dans 20 ml de DMF, ajoute 0,25 g d'hydrure de sodium à 60 % dans l'huile et laisse 30 minutes sous agitation à 4°C. On ajoute ensuite 1,34 g de chlorure de 2,4-diméthoxybenzènesulfonyle et laisse 4 heures sous agitation à TA. On ajoute 50 ml d'eau au mélange réactionnel, extrait à l'AcOEt, lave la phase organique par une solution saturée de NaCl, sèche sur $Na_2SO_4$ et évapore sous vide le solvant. On chromatographie le résidu sur gel de silice en éluant par le mélange DCM/AcOEt (97/3 ; v/v). On obtient 2 g du produit attendu après cristallisation dans le mélange DCM/éther iso.

C) 5-Chloro-1-[(2,4-diméthoxyphényl)sulfonyl]-3-(2-méthoxyphényl)-2-oxo-2,3-dihydro-1*H*-indol-3-yl carbamate de phényle.

On refroidit à 4°C une solution de 2 g du composé obtenu à l'étape précédente et 10 ml de pyridine dans 10 ml de DCM, ajoute 0,77 ml de chloroformiate de phényle et laisse 1 heure sous agitation à TA. On concentre sous vide, extrait le résidu à l'AcOEt, lave la phase organique par une solution saturée de NaCl, sèche sur $Na_2SO_4$ et évapore sous vide le solvant. On chromatographie le résidu sur gel de silice en éluant par le mélange DMC/AcOEt (96/4 ; v/v). On obtient 2,6 g du produit attendu après cristallisation dans le mélange DCM/éther iso, F = 191°C.

Préparation 1.19

5-Chloro-1-[(2,4-diméthoxyphényl)sulfonyl]-3-(2-méthoxyphényl)-2-oxo-2,3-dihydro-1*H*-indol-3-yl carbamate de phényle, isomère unique.

(II) : $R_1$ = Cl ; $R_2$ = H ; $R_3$ = $OCH_3$ ; $R_4$ = H ; X=-NH-;

Y = 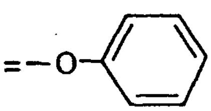 ;

N = 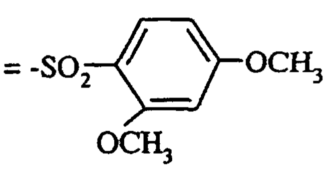

A) 3,5-Dichloro-3-(2-méthoxyphényl)-1,3-dihydro-2*H*-indol-2-one.

On refroidit à 0°C un mélange de 9 g du composé obtenu à l'étape A de la Préparation 1.1 et 3,74 ml de pyridine dans 100 ml de DCM, ajoute, en goutte à goutte et en 3 minutes, une solution de 3,45 ml de chlorure de thionyle dans 3 ml de DCM et laisse 30 minutes sous agitation. On ajoute de l'eau au mélange réactionnel et évapore le DCM sous vide. On essore le précipité formé, le lave quatre fois à l'eau puis à l'éther iso froid et le sèche. On obtient 8,8 g du produit attendu.

B) 5-Chloro-3-[[(1S)-2-hydroxy-1-phényléthyl]amino]-3-(2-méthoxyphényl)-1,3-dihydro-2*H*-indol-2-one, isomère A et B.

On laisse 2 heures sous agitation à TA un mélange de 7 g du composé obtenu à l'étape précédente et 7,65 g de (S)-(+)-α-phénylglycinol dans 100 ml de chloroforme. On ajoute ensuite 2,9 g de DIPEA et laisse 48 heures sous agitation à TA. On essore le précipité formé et recueille l'isomère A. On lave les jus d'essorage par une solution à 5 % de $K_2CO_3$, sèche la phase organique sur $Na_2SO_4$ et évapore sous vide le solvant. On chromatographie le résidu sur gel de silice en éluant par le mélange DCM/AcOEt (70/30 ; v/v). On sépare les deux diastéréoisomères :

- le moins polaire, isomère A : on obtient 4,55 g (précipité et chromatographie) ; $\alpha_D^{20}$ = +193° (c = 0,16 ; chloroforme).
- le plus polaire, isomère B.

C) 3-Amino-5-chloro-3-(2-méthoxyphényl)-1,3-dihydro-2*H*-indol-2-one, isomère dextrogyre.

On laisse 1 heure 30 minutes sous agitation à TA un mélange de 4,55 g du composé obtenu à l'étape précédente (isomère A) et 5,5 g de tétraacétate de plomb dans 75 ml de DCM et 35 ml de MeOH. On concentre sous vide, reprend le résidu par une solution saturée de $NaHCO_3$, extrait à l'AcOEt, lave la phase organique à l'eau, par une solution saturée de NaCl, sèche sur $Na_2SO_4$ et évapore sous vide le solvant. On reprend l'huile obtenue dans 100 ml d'une solution d'HCl 3N, ajoute 10 ml de MeOH et laisse 2 heures sous agitation à TA. On concentre sous vide les solvants organiques, lave deux fois la phase aqueuse à l'éther, alcalinise la phase aqueuse par ajout de KOH en pastilles, extrait à l'AcOEt, lave la phase organique à l'eau, par une solution saturée de NaCl, sèche sur $Na_2SO_4$ et évapore sous vide le solvant. On obtient 1,178 g du produit attendu après cristallisation dans le mélange DCM/éther iso/THF, F = 202°C.
$\alpha_D^{20}$ = + 83,3° (c =0,16 ; chloroforme).

D) 3-Amino-5-chloro-1-[(2,4-diméthoxyphényl)sulfonyl]-3-(2-méthoxyphényl)-1,3-dihydro-2*H*-indol-2-one, isomère dextrogyre

On refroidit à 0°C une solution de 1,178 g du composé obtenu à l'étape précédente dans 10 ml de DMF, ajoute, sous atmosphère d'argon, 0,188 g d'hydrure de sodium à 60 % dans l'huile et laisse sous agitation jusqu'à cessation du dégagement gazeux. On ajoute ensuite 1,02 g de chlorure de 2,4-diméthoxybenzènesulfonyle et laisse 3 heures sous agitation à TA. On verse le mélange réactionnel dans une solution à 5 % de $K_2CO_3$, extrait à l'AcOEt, lave la phase organique à l'eau, par une solution à 5 % de $K_2CO_3$, par une solution saturée de NaCl, sèche sur $Na_2SO_4$ et évapore sous vide le solvant. On chromatographie le résidu sur gel de silice en éluant par le mélange DCM/AcOEt (95/5 ; v/v). On obtient 1,254 g du produit attendu après cristallisation dans le mélange DCM/éther/éther iso, F = 172-173°C.
$\alpha_D^{20}$ = +113° (c = 0,18 ; chloroforme).

E) 5-Chloro-1-[(2,4-diméthoxyphényl)sulfonyl]-3-(2-méthoxyphényl)-2-oxo-2,3-dihydro-1*H*-indol-3-yl carbamate de phényle, isomère unique.

On refroidit au bain de glace une solution de 1,1 g du composé obtenu à l'étape précédente dans 10 ml de DCM, ajoute 1,8 ml de pyridine, puis ajoute, goutte à goutte, 0,37 ml de chloroformiate de phényle et abandonne une nuit à froid. On dilue le mélange réactionnel au DCM, lave trois fois la phase organique à l'eau, sèche sur $Na_2SO_4$ et évapore sous vide le solvant. On chromatographie le résidu sur gel de silice en éluant par le mélange DCM/AcOEt (98/2 ; v/v). On obtient 1,136 g du produit attendu.

Préparation 1.20

5-Chloro-1-[(2,4-diméthoxyphényl)sulfonyl]-3-(2-isopropoxyphényl)-2-oxo-2,3-dihydro-1*H*-indol-3-yl carbamate de phényle, isomère unique.

(II) : $R_1$ = Cl ; $R_2$ = H ; $R_3$ = $OCH(CH_3)_2$ ; $R_4$ = H ; X = -NH- ;

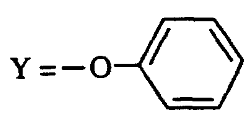 ;

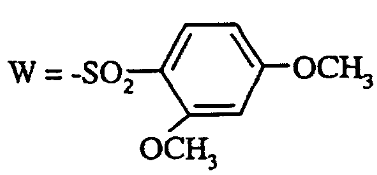

A) 3,5-Dichloro-3-(2-isopropoxyphényl)-1,3-dihydro-2*H*-indol-2-one.

On refroidit à 0°C un mélange de 12 g du composé obtenu à l'étape B de la Préparation 1.3 et 8,35 ml de pyridine dans 165 ml de DCM, ajoute, en goutte à goutte, 7,62 ml de chlorure de thionyle et laisse 15 minutes sous agitation. On concentre sous vide, extrait le résidu à l'AcOEt, lave la phase organique à l'eau, sèche sur $Na_2SO_4$ et évapore sous vide le solvant. On chromatographie le résidu sur gel de silice en éluant par le mélange DCM/AcOEt (90/10 ; v/v). On obtient 15,22 g du produit attendu.

B) 5-Chloro-3-[[(1S)-2-hydroxy-1-phényléthyl]amino]-3-(2-isopropoxyphényl)-1,3-dihydro-2*H*-indol-2-one, isomère A et isomère B.

On laisse 1 heure sous agitation à TA un mélange de 8,17 g du composé obtenu à l'étape précédente et 3,66 g de (S)-(+)-α-phénylglycinol dans 265 ml de chloroforme. On ajoute ensuite 4,66 ml de DIPEA, laisse 3 heures sous agitation à TA puis chauffe à 50°C pendant 18 heures. On concentre sous vide, extrait le résidu à l'AcOEt, lave la phase organique par une solution à 5 % de $K_2CO_3$, à l'eau, sèche sur $Na_2SO_4$ et évapore sous vide le solvant. On chromatographie le résidu sur gel de sillice en éluant par le mélange DCM/AcOEt (90/10 ; v/v) et sépare :

- l'isomère le moins polaire, isomère A : on obtient 4,98 g après cristallisation dans l'éther iso, F = 212,7°C.
  $\alpha_D^{20}$ = + 212,4° (c = 0,2 ; chloroforme).

On poursuit la chromatographie en éluant par le mélange DCM/AcOEt (70/30 ; v/v) et sépare :

- l'isomère le plus polaire, isomère B : on obtient 3,49 g après cristallisation dans l'éther iso, F = 241,3°C.
  $\alpha_D^{20}$ = - 6,6° (c = 0,2 ; chloroforme).

C) 3-Amino-5-chloro-3-(2-isopropoxyphényl)-1,3-dihydro-2*H*-indol-2-one, isomère dextrogyre.

On refroidit à 0°C une solution de 3,9 g du composé obtenu à l'étape précédente (isomère A) dans 75 ml de DCM et 37 ml de MeOH, ajoute 4,35 g de tétraacétate de plomb et laisse 2 heures sous agitation. On ajoute 3 gouttes d'une solution à 5 % de $NaHCO_3$, concentre sous vide, extrait le résidu à l'AcOEt, lave la phase organique à l'eau, par une solution saturée de NaCl, sèche sur $Na_2SO_4$ et évapore sous vide le solvant. On reprend le produit obtenu dans 120 ml d'HCl concentré, lave à l'éther, alcalinise la phase aqueuse par ajout de $K_2CO_3$, extrait à l'AcOEt et laisse en cristallisation. On essore les cristaux formés et les sèche. On concentre sous vide les jus d'essorage, chromatographie le résidu sur gel de silice en éluant par le mélange DCM/AcOEt (65/35 ; v/v) et cristallise le produit obtenu dans l'éther iso. On obtient au total 2,03 g du produit attendu, F = 193°C.
$\alpha_D^{20}$ = + 44° (c = 0,18 ; chloroforme).

D) 3-Amino-5-chloro-1-[(2,4-diméthoxyphényl)sulfonyl]-3-(2-isopropoxyphényl)-1,3-dihydro-2*H*-indol-2-one, isomère dextrogyre.

On prépare ce composé selon le mode opératoire décrit à l'étape D de la Préparation 1.19 à partir de 1,86 g du composé obtenu à l'étape précédente, 0,282 g d'hydrure de sodium à 60 % dans l'huile, 20 ml de DMF et 1,395 g de chlorure de 2,4-diméthoxybenzènesulfonyle. On obtient 2,68 g du produit attendu après cristallisation dans l'hexane.
$\alpha_D^{20}$ = + 79,5° (c = 0,2 ; chloroforme).

E) 5-Chloro-1-[(2,4-diméthoxyphényl)sulfonyl]-3-(2-isopropoxyphényl)-2-oxo-2,3-dihydro-1*H*-indol-3-yl carbamate de phényle, isomère unique.

On refroidit à 0°C un mélange de 2,68 g du composé obtenu à l'étape précédente et 4,5 ml de pyridine dans 25 ml de DCM, ajoute en 30 secondes 1,06 g de chloroformiate de phényle et abandonne à froid pendant 18 heures. On chromatographie directement le mélange réactionnel sur gel de silice préparé dans le mélange DCM/hexane (80/20 ; v/v) et élue par le mélange DCM/AcOEt (95/5 ; v/v). On obtient 2,88 g du produit attendu que l'on utilise tel quel.

Préparation 1.21

5-Chloro-1-[(2,4-diméthoxyphényl)sulfonyl]-3-(2-isopropoxyphényl)-2-oxo-2,3-dihydro-1*H*-indol-3-yl carbamate de phényle, isomère unique.

(II) : $R_1$ = Cl ; $R_2$ = H ; $R_3$ = $OCH(CH_3)_2$ ; $R_4$ = H ; X = -NH- ;

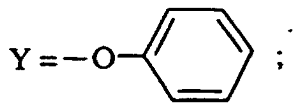

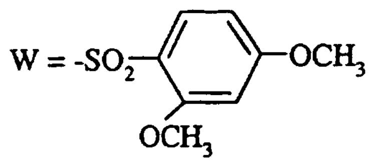

A) 3-Amino-5-chloro-3-(2-isopropoxyphényl)-1,3-dihydro-2*H*-indol-2-one, isomère lévogyre.

On refroidit à 0°C une solution de 3,4 g du composé obtenu à l'étape B de la Préparation 1.20 (isomère B) dans 65 ml de DCM et 32,4 ml de MeOH, ajoute 3,8 g de tétraacétate de plomb et laisse 40 minutes sous agitation. On ajoute 3 gouttes d'une solution à 5 % de $NaHCO_3$, concentre sous vide, extrait le résidu à l'AcOEt, lave la phase organique à l'eau, par une solution saturée de NaCl, sèche sur $Na_2SO_4$ et évapore sous vide le solvant. On reprend le produit obtenu dans 120 ml d'HCl 0,5N, lave à l'éther, alcalinise la phase aqueuse par ajout de $K_2CO_3$ et essore le précipité formé. On obtient 1,9 g du produit attendu après cristallisation dans l'éther iso, F = 192°C.
$\alpha_D^{20}$ = - 42° (c = 0,2 ; chloroforme).

B) 3-Amino-5-chloro-1-[(2,4-diméthoxyphényl)sulfonyl]-3-(2-isopropoxyphényl)-1,3-dihydro-2*H*-indol-2-one, isomère lévogyre.

On prépare ce composé selon le mode opératoire décrit à l'étape D de la Préparation 1.19 à partir de 1,67 g du composé obtenu à l'étape précédente, 0,253 g d'hydrure de sodium à 60 % dans l'huile, 20 ml de DMF et 1,249 g de chlorure de 2,4-diméthoxybenzènesulfonyle. On obtient 2,453 g du produit attendu après cristallisation dans l'hexane.
$\alpha_D^{20}$ = - 79,8° (c = 0,2 ; chloroforme).

C) 5-Chloro-1-[(2,4-diméthoxyphényl)sulfonyl]-3-(2-isopropoxyphényl)-2-oxo-2,3-dihydro-1*H*-indol-3-yl carbamate de phényle, isomère unique.

On refroidit à 0°C un mélange de 2,103 g du composé obtenu à l'étape précédente et 3,5 ml de pyridine dans 20 ml de DCM, ajoute 0,665 ml de chloroformiate de phényle et abandonne à froid pendant 36 heures. On concentre sous vide, reprend le résidu par une solution à 5 % de $K_2CO_3$, extrait à l'AcOEt, lave la phase organique à l'eau, par une solution saturée de NaCl, sèche sur $Na_2SO_4$ et évapore sous vide le solvant. On chromatographie le résidu sur gel de silice en éluant par le mélange DCM/hexane (80/20 ; v/v). On obtient 1,88 g du produit attendu que l'on utilise tel quel.

Préparation 1.22

5-Chloro-1-[(2,4-diméthoxyphényl)sulfonyl]-3-(2,5-diméthoxyphényl)-2-oxo-2,3-dihydro-1*H*-indol-3-yl carbamate de phényle.

(II) : $R_1$ = Cl ; $R_2$ = H ; $R_3$ = $OCH_3$ ; $R_4$ = 5- $OCH_3$ ; X = -NH- ;

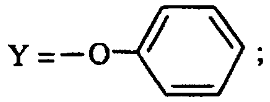

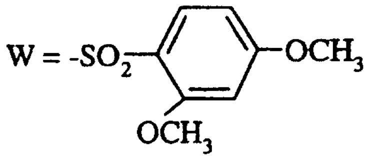

A) 3,5-Dichloro-3-(2,5-diméthoxyphényl)-1,3-dihydro-2*H*-indol-2-one.

On refroidit à une température inférieure à 20°C un mélange de 3 g du composé obtenu à l'étape A de la Préparation 1.17 et 1,2 ml de pyridine dans 50 ml de DCM, ajoute 0,8 ml de chlorure de thionyle et laisse 1 heure sous agitation. On lave le mélange réactionnel à l'eau, sèche la phase organique sur $Na_2SO_4$ et évapore sous vide le solvant. On chromatographie le résidu sur gel de silice en éluant au DCM. On obtient 1,9 g du produit attendu que l'on utilise tel quel.

B) 3-Amino-5-chloro-3-(2,5-diméthoxyphényl)-1,3-dihydro-2*H*-indol-2-one.

On refroidit à 0°C un mélange de 1,25 g du composé obtenu à l'étape précédente dans 7 ml de THF, fait barboter de l'ammoniac gaz 4 fois 10 minutes en 6 heures et laisse 24 heures sous agitation à TA. On concentre sous vide, reprend le résidu au DCM et évapore sous vide le solvant. On chromatographie le résidu sur gel de silice en éluant au DCM puis par le mélange DCM/AcOEt (60/40 ; v/v). On obtient 0,808 g du produit attendu que l'on utilise tel quel.

C) 3-Amino-5-chloro-1-[(2,4-diméthoxyphényl)sulfonyl]-3-(2,5-diméthoxyphényl)-1,3-dihydro-2*H*-indol-2-one.

On prépare ce composé selon le mode opératoire décrit à l'étape D de la Préparation 1.19 à partir de 0,749 g du composé obtenu à l'étape précédente, 0,113 g d'hydrure de sodium à 60 % dans l'huile, 7 ml de DMF et 0,612 g de chlorure de 2,4-diméthoxybenzènesulfonyle. On obtient 0,9 g du produit attendu après cristallisation dans le mélange DCM/éther iso, F = 191°C.

D) 5-Chloro-1-[(2,4-diméthoxyphényl)sulfonyl]-3-(2,5-diméthoxyphényl)-2-oxo-2,3-dihydro-1*H*-indol-3-yl carbamate de phényle.

On refroidit à 0°C un mélange de 0,52 g du composé obtenu à l'étape précédente et 1 ml de pyridine dans 5 ml de DCM, ajoute 0,16 ml de chloroformiate de phényle et laisse 16 heures sous agitation. On ajoute de l'eau au mélange réactionnel, évapore sous vide le DCM, extrait à l'AcOEt, lave la phase organique par une solution à 5 % de $K_2CO_3$, à l'eau, par une solution saturée de NaCl, sèche sur $Na_2SO_4$ et évapore sous vide le solvant. On chromatographie le résidu sur gel de silice en éluant au DCM. On obtient 0,46 g du produit attendu que l'on utilise tel quel.

Préparation 1.23

5-Chloro-3-(2-chlorophényl)-1-[(2,4-diméthoxyphényl)sulfonyl]-2-oxo-2,3-dihydro-1*H*-indol-3-yl carbamate de phényle.

(II) : $R_1$ = Cl ; $R_2$ = H ; $R_3$ = Cl ; $R_4$ = H ; X = -NH- ;

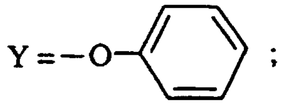

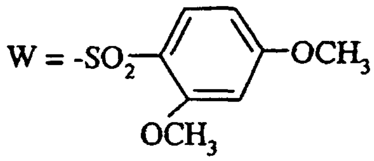

A) 3-Amino-5-chloro-3-(2-chlorophényl)-1-[(2,4-diméthoxyphényl)sulfonyl]-1,3-dihydro-2*H*-indol-2-one.

On prépare ce composé selon le mode opératoire décrit dans WO 95/18105 à l'Exemple 3.

B) 5-Chloro-3-(2-chlorophényl)-1-[(2,4-diméthoxyphényl)sulfonyl]-2-oxo-2,3-dihydro-1*H*-indol-3-yl carbamate de phényle.

On refroidit à 0°C une solution de 3 g du composé obtenu à l'étape précédente dans 25 ml de pyridine , ajoute, goutte à goutte, une solution de 1,25 g de chloroformiate de phényle dans 2 ml de DCM et laisse 3 heures sous agitation à 0°C puis une nuit à TA. On refroidit à nouveau à 0°C, ajoute 0,96 g de chloroformiate de phényle et laisse 18 heures au repos à froid. On concentre sous vide, reprend le résidu par une solution à 5 % de $K_2CO_3$, extrait à l'AcOEt, sèche la phase organique sur $Na_2SO_4$ et évapore sous vide le solvant. On chromatographie le résidu sur gel de silice en éluant par le mélange DCM/AcOEt (95/5 ; v/v). On obtient 2,88 g du produit attendu.

Préparation 1.24

5-Chloro-1-[(2,4-diméthoxyphényl)sulfonyl]-3-(2-méthoxyphényl)-6-méthyl-2-oxo-2,3-dihydro-1*H*-indol-3-yl carbamate de phényle.

(II) : $R_1$ = Cl ; $R_2$ = 6-$CH_3$ ; $R_3$ = $OCH_3$ ; $R_4$ = H ; X = -NH- ;

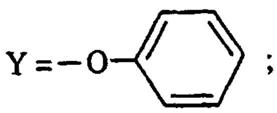

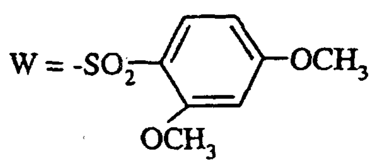

A) 3,5-Dichloro-3-(2-méthoxyphényl)-6-méthyl-1,3-dihydro-2*H*-indol-2-one.

On refroidit à 0°C un mélange de 2,0 g du composé obtenu à l'étape B de la Préparation 1.6 dans 45 ml de DCM, ajoute 0,77 ml de pyridine puis 1,17 g de chlorure de thionyle et laisse 2 heures sous agitation après avoir laissé la température remonter à TA. On ajoute de l'eau et du DCM au mélange réactionnel, après décantation lave quatre fois la phase organique à l'eau, sèche sur $Na_2SO_4$ et évapore sous vide le solvant. On obtient le produit attendu que l'on utilise tel quel.

B) 3-Amino-5-chloro-3-(2-méthoxyphényl)-6-méthyl-1,3-dihydro-2*H*-indol-2-one.

On fait barboter à TA pendant 30 minutes de l'ammoniac gaz dans un mélange de 3,4 g du composé obtenu à l'étape précédente dans 25 ml de DCM et laisse 18 heures sous agitation. On concentre sous vide, extrait le résidu à l'AcOEt, lave la phase organique par une solution saturée de NaCl, sèche sur $Na_2SO_4$ et évapore sous vide le solvant. On

chromatographie le résidu sur gel de silice en éluant par le mélange DCM/MeOH (95/5 ; v/v). On obtient 2 g du produit attendu.

C) 3-Amino-5-chloro-1-[(2,4-diméthoxyphényl)sulfonyl]-3-(2-méthoxyphényl)-6-méthyl-1,3-dihydro-2*H*-indol-2-one.

On prépare ce composé selon le mode opératoire décrit à l'étape B de la Préparation 1.18 à partir de 2 g du composé obtenu à l'étape précédente, 0,29 g d'hydrure de sodium à 60 % dans l'huile, 15 ml de DMF et 1,54 g de chlorure de 2,4-diméthoxybenzènesulfonyle. On obtient 3,2 g du produit attendu.

D) 5-Chloro-1-[(2,4-diméthoxyphényl)sulfonyl]-3-(2-méthoxyphényl)-6-méthyl-2-oxo-2,3-dihydro-1*H*-indol-3-yl carbamate de phényle.

On refroidit à 4°C une solution de 3,2 g du composé obtenu à l'étape précédente et 10 ml de pyridine dans 30 ml de DCM, ajoute, goutte à goutte, 1,2 ml de chloroformiate de phényle et laisse sous agitation 2 heures à TA. On concentre sous vide, extrait le résidu à l'AcOEt, lave la phase organique par une solution à 5 % de $KHSO_4$, par une solution à 5 % de $Na_2CO_3$, à l'eau, par une solution saturée de NaCl, sèche sur $Na_2SO_4$ et évapore sous vide le solvant. On obtient 2,3 g du produit attendu après cristallisation dans le mélange DCM/éther iso.

Préparation 1.25

5-Chloro-1-[(2,4-diméthoxyphényl)sulfonyl]-3-(2-méthoxyphényl)-6-trifluoro méthyl-2-oxo-2,3-dihydro-1*H*-indol-3-yl carbamate de phényle, isomère unique.

(II) : $R_1$ = Cl ; $R_2$ = 6-$CF_3$ ; $R_3$ = $OCH_3$ ; $R_4$ = H ; X = -NH- ;

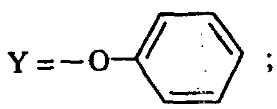

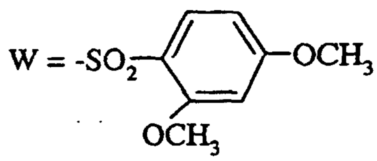

A) 5-Chloro-3-hydroxy-3-(2-méthoxyphényl)-6-trifluorométhyl-1,3-dihydro-2*H*-indol-2-one.

a) 4-Chloro-3-trifluorométhylphénylcarbamate de *tert*-butyle.

On prépare ce composé selon le mode opératoire décrit à l'étape B a) de la Préparation 1.6 à partir de 4-chloro-3-trifluorométhylaniline et de di-*tert*-butyldicarbonate dans le dioxane. On obtient le produit attendu sous forme d'huile qui se solidifie, F = 90°C.

b) On refroidit à -70°C, sous atmosphère d'argon, une solution de 4 g de 4-chloro-3-trifluorométhylphénylcarbamate de *tert*-butyle dans 30 ml d'éther, ajoute, goutte à goutte, 22 ml d'une solution 1,5M de *tert*-butyllithium dans le pentane, laisse 1 heure sous agitation en faisant remonter la température à -10°C et laisse 2 heures 30 minutes sous agitation à -10°C. On refroidit le mélange réactionnel à -70°C, ajoute, goutte à goutte, une solution de 3,05 g du composé obtenu à l'étape A de la Préparation 1.6 dans 15 ml de THF, laisse 1 heure sous agitation en laissant remonter la température à -30°C puis 16 heures en laissant remonter la température à TA. On ajoute une solution saturée de $NH_4Cl$ au mélange réactionnel, évapore l'éther et le THF, extrait la phase aqueuse résultante à l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le mélange DCM/AcOEt (90/10 ; v/v). On obtient 1,48 g du produit attendu après cristallisation dans le mélange éther iso/hexane, F = 230-231°C.

B) 3,5-Dichloro-3-(2-méthoxyphényl)-6-trifluorométhyl-1,3-dihydro-2*H*-indol-2-one.

On refroidit à 0°C une suspension de 1,3 g du composé obtenu à l'étape A dans 8 ml de DCM, ajoute 0,43 ml de pyridine puis 0,4 ml de chlorure de thionyle et laisse 15 minutes sous agitation. On lave trois fois le mélange réactionnel à l'eau, sèche la phase organique sur $Na_2SO_4$ et évapore sous vide le solvant. On obtient 1,2 g du produit attendu que l'on utilise tel quel.

C) 5-Chloro-3-[[(1S)-2-hydroxy-1-phényléthyl]amino]-3-(2-méthoxyphényl)-6-trifluorométhyl-1,3-dihydro-2*H*-indol-2-one, isomére A et isomère B.

On laisse 4 heures sous agitation à TA un mélange de 1,29 g du composé obtenu à l'étape précédente et 0,47 g de (S)-(+)-$\alpha$-phénylglycinol dans 35 ml de chloroforme. On ajoute ensuite 0,6 ml de DIPEA, concentre de moitié le solvant et laisse 22 heures sous agitation à TA. On concentre sous vide le mélange réactionnel et chromatographie le résidu sur gel de silice en éluant par le mélange DCM/AcOEt (85/15 ; v/v). On sépare un diastéréoisomère :

- le moins polaire, isomère A : on obtient 0,712 g après cristallisation dans le mélange DCM/éther iso, F = 205°C. $\alpha_D^{20}$ = + 164° (c = 0,16 ; chloroforme).

On poursuit la chromatographie en éluant par le mélange DCM/AcOEt (70/30 ; v/v) et sépare l'autre diastéréoisomère :

- le plus polaire, isomère B : on obtient 0,45 g, F = 242°C. $\alpha_D^{20}$ = - 55° (c = 0,15 ; chloroforme).

D) 3-Amino-5-chloro-3-(2-méthoxyphényl)-6-trifluorométhyl-1,3-dihydro-2*H*-indol-2-one, isomère unique.

On refroidit à 0°C une solution de 0,7 g du composé obtenu à l'étape précédente (isomère A) dans 15 ml de DCM et 7 ml de MeOH, ajoute 0,907 g de tétraacétate de plomb et laisse 40 minutes sous agitation. On ajoute quelques gouttes d'une solution à 5 % de $NaHCO_3$ et concentre sous vide. On reprend le résidu dans l'eau, extrait 3 fois à l'AcOEt, lave la phase organique à l'eau, par une solution saturée de NaCl, sèche sur $Na_2SO_4$ et évapore sous vide le solvant. On reprend le produit obtenu dans 30 ml d'eau, ajoute, en goutte à goutte, 30 ml d'une solution d'HCl 5N, puis du THF jusqu'à solubilisation et laisse 1 heure sous agitation. On dilue le mélange réactionnel par 50 ml d'eau, lave la phase aqueuse par 150 ml d'éther, alcalinise la phase aqueuse à pH = 12 par ajout de $K_2CO_3$ puis d'une solution de NaOH concentrée, extrait à l'AcOEt, sèche la phase organique sur $Na_2SO_4$ et évapore sous vide le solvant. On chromatographie le résidu sur gel de silice en éluant par le mélange DCM/MeOH (92/8 ; v/v). On obtient 0,19 g du produit attendu.

E) 3-Amino-5-chloro-1-[(2,4-diméthoxyphényl)sulfonyl]-3-(2-méthoxyphényl)-6-trifluorométhyl-1,3-dihydro-2*H*-indol-2-one, isomère unique.

On prépare ce composé selon le mode opératoire décrit à l'étape B de la Préparation 1.18 à partir de 0,19 g du composé obtenu à l'étape précédente, 0,023 g d'hydrure de sodium à 60 % dans l'huile, 2 ml de DMF et 0,138 g de chlorure de 2,4-diméthoxybenzènesulfonyle. On obtient 0,22 g du produit attendu.

F) 5-Chloro-1-[(2,4-diméthoxyphényl)sulfonyl]-3-(2-méthoxyphényl)-6-trifluoro méthyl-2-oxo-2,3-dihydro-1*H*-indol-3-yl carbamate de phényle, isomère unique.

On prépare ce composé selon le mode opératoire décrit à l'étape C de la Préparation 1.18 à partir de 0,215 g du composé obtenu à l'étape précédente, 0,31 ml de pyridine, 5 ml de DCM et 0,079 g de chloroformiate de phényle. On obtient 0,208 g du produit attendu.

Préparation 1.26

6-Chloro-3-(2-chlorophényl)-1-[(2,4-diméthoxyphényl)sulfonyl]-5-méthyl-2-oxo-2,3-dihydro-1*H*-indol-3-yl carbamate de phényle.

(II) ; $R_1 = CH_3$ ; $R_2$ = 6-Cl ; $R_3$ = Cl ; $R_4$ = H ; X = -NH- ;

Y = 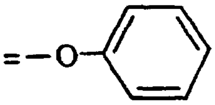 ;

W = 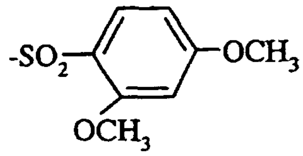

A) N-(3-chloro-4-méthylphényl)-DL-2-chloromandélamide.

On chauffe à reflux pendant 6 heures un mélange de 52,72 g d'acide DL-2-chloromandélique et 40 g de 3-chloro-4-méthylaniline dans 400 ml de 1,2-dichlorobenzène en éliminant l'eau formée à l'aide d'un appareil de Dean-Stark. Après refroidissement à TA, on laisse en cristallisation et essore le précipité formé. On obtient le produit attendu après recristallisation dans le mélange DCM/éther iso, F = 164°C.

B) 6-Chloro-3-(2-chlorophényl)-5-méthyl-1,3-dihydroindol-2-one.

On refroidit à 5°C 102 ml d'$H_2SO_4$ concentré (95 %) et ajoute en goutte à goutte 23 ml d'acide sulfurique fumant (oleum à 30 %). On ajoute ensuite, par petites fractions et à une température inférieure à 5°C, 25 g du composé obtenu à l'étape précédente et laisse 48 heures sous agitation à TA. On verse le mélange réactionnel sur un mélange glace/eau, essore le précipité formé et le lave à l'eau jusqu'à pH = 7. On dissout le précipité dans l'AcOEt, lave la phase organique par une solution saturée de NaCl, sèche sur $Na_2SO_4$, évapore partiellement sous vide le solvant et essore le précipité formé. On obtient le produit attendu, F = 186°C.

C) 3-Bromo-6-chloro-3-(2-chlorophényl)-5-méthyl-1,3-dihydro-2*H*-indol-2-one.

A une suspension de 16,36 g du composé obtenu à l'étape précédente dans 200 ml de DCM on ajoute, à TA et lentement, une solution de 2,6 ml de brome dans 10 ml de DCM. On rajoute une solution de 0,26 ml de brome dans 5 ml de DCM puis concentre sous vide le mélange réactionnel. On reprend deux fois le résidu au DCM et évapore chaque fois sous vide le solvant. On dissout le résidu dans l'AcOEt, lave la phase organique par une solution saturée de NaCl, sèche sur $MgSO_4$ et évapore sous vide le solvant. On obtient le produit attendu.

D) 3-Amino-6-chloro-3-(2-chlorophényl)-5-méthyl-1,3-dihydro-2*H*-indol-2-one.

A une solution de 4,25 g du composé obtenu à l'étape précédente dans 30 ml de THF on ajoute 30 ml d'une solution concentrée d'ammoniaque et laisse 24 heures sous agitation à TA. On rajoute 10 ml d'une solution concentrée d'ammoniaque et laisse une nuit sous agitation à TA. On concentre sous vide, extrait le résidu au DCM, lave la phase organique à l'eau, sèche sur $Na_2SO_4$ et évapore sous vide le solvant. On obtient 3,02 g du produit attendu après cristallisation dans le DCM, F = 233°C.

E) 3-Amino-6-chloro-3-(2-chlorophényl)-1-[(2,4-diméthoxyphényl)sulfonyl]-5-méthyl-1,3-dihydro-2*H*-indol-2-one.

On prépare ce composé selon le mode opératoire décrit à l'étape B de la Préparation 1.18 à partir de 0,54 g du composé obtenu à l'étape précédente, 0,077 g d'hydrure de sodium à 60 % dans l'huile, 5 ml de DMF et 0,435 g de chlorure de 2,4-diméthoxybenzènesulfonyle. On obtient 0,592 g du produit attendu après cristallisation dans le mélange DCM/éther iso.

F) 6-Chloro-3-(2-chlorophényl)-1-[(2,4-diméthoxyphényl)sulfonyl]-5-méthyl-2-oxo-2,3-dihydro-1*H*-indol-3-yl carbamate de phényle.

On prépare ce composé selon le mode opératoire décrit à l'étape C de la Préparation 1.18 à partir de 0,592 g du composé obtenu à l'étape précédente, 1 ml de pyridine, 6 ml de DCM et 0,19 ml de chloroformiate de phényle. On obtient 0,690 g du produit attendu que l'on utilise tel quel.

Préparation 1.27

Acide 2-[[5-chloro-3-(2-isopropoxyphényl)-2-oxo-2,3-dihydro-1*H*-indol-3-yl]oxy]acétique.

(II) : $R_1$ = Cl ; $R_2$ = H ; $R_3$ = $-OCH(CH_3)_2$ ; $R_4$ = H ; X = $-O-CH_2-$ ; Y = OH ; W = H.

A) Ester méthylique de l'acide 2-[[5-chloro-3-(2-isopropoxyphényl)-2-oxo-2,3-dihydro-1*H*-indol-3-yl]oxy]acétique.

A une solution de 1,05 g de glycolate de méthyle dans 20 ml de THF, on ajoute 0,29 g d'hydrure de sodium à 60 % dans l'huile et laisse 10 minutes sous agitation à TA. On ajoute ensuite, en goutte à goutte, une solution de 1,88 g du composé obtenu à l'étape A de la Préparation 1.20 et laisse 10 minutes sous agitation à TA. On concentre sous vide, extrait le résidu à l'AcOEt, lave la phase organique à l'eau, par une solution d'HCl 1N, sèche sur $Na_2SO_4$ et évapore sous vide le solvant. On chromatographie le résidu sur gel de silice en éluant au DCM puis à l'AcOEt. On obtient 0,8 g du produit attendu après cristallisation dans l'éther iso, F = 210-213°C.

B) Acide 2-[[5-chloro-3-(2-isopropoxyphényl)-2-oxo-2,3-dihydro-1*H*-indol-3-yl]oxy]acétique.

A une solution de 0,8 g du composé obtenu à l'étape précédente dans 40 ml de MeOH, on ajoute, à 20°C, une solution de 0,24 g de NaOH en pastilles dans 5 ml d'eau et laisse 15 heures sous agitation à TA. On concentre sous vide, dissout le résidu dans 25 ml d'eau, acidifie par ajout, goutte à goutte, de 1 ml d'HCl 12 N et essore le précipité formé. On obtient 0,8 g du produit attendu, F = 225-228°C.

Préparation 1.28

Acide 2-[5-chloro-1-[(2,4-diméthoxyphényl)sulfonyl]-3-(2-méthoxyphényl)-2-oxo-2,3-dihydro-1*H*-indol-3-yl]amino] acétique.

(II) : $R_1$ = Cl ; $R_2$ = H ; $R_3$ = $-OCH_3$ ; $R_4$ = H ; X = $-NH-CH_2-$ ; Y = OH ;

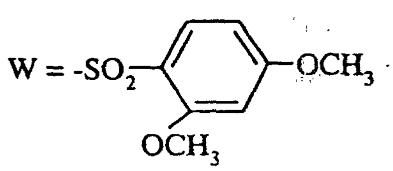

A) Ester *tert*-butylique de l'acide 2-[[5-chloro-3-(2-méthoxyphényl)-2-oxo-2,3-dihydro-1*H*-indol-3-yl]amino]acétique.

On refroidit au bain de glace une solution de 2,4 g de chlorhydrate d'ester *tert*-butylique de la glycine dans 50 ml de chloroforme et 50 ml de THF, ajoute 2,3 g de triéthylamine puis 3,5 g du composé obtenu à l'étape A de la Préparation 1.19 et laisse une nuit sous agitation à TA. On concentre sous vide, reprend le résidu à l'eau, extrait au DCM, sèche la phase organique sur $Na_2SO_4$ et évapore sous vide le solvant. On triture le résidu dans de l'éther iso et essore le précipité formé. On obtient 2,2 g du produit attendu.

B) Ester *tert*-butylique de l'acide 2-[[5-chloro-1-[(2,4-diméthoxyphényl)sulfonyl]-3-(2-méthoxyphényl)-2-oxo-2,3-dihydro-1*H*-indol-3-yl]amino]acétique.

A un mélange de 2,1 g du composé obtenu à l'étape précédente dans 10 ml de DMF, on ajoute 0,225 g d'hydrure de sodium à 60 % dans l'huile et laisse 30 minutes sous agitation à TA. On ajoute ensuite 1,2 g de chlorure de 2,4-diméthoxybenzène sulfonyle et laisse 18 heures sous agitation à TA. On verse le mélange réactionnel dans l'eau, extrait à l'AcOEt, sèche la phase organique sur $Na_2SO_4$ et évapore sous vide le solvant. On chromatographie le résidu sur gel de silice en éluant au DCM. On obtient 2,5 g du produit attendu après cristallisation dans l'éther iso.

C) Acide 2-[[5-chloro-1-[(2,4-diméthoxyphényl)sulfonyl]-3-(2-méthoxyphényl)-2-oxo-2,3-dihydro-1*H*-indol-3-yl]amino] acétique.

On laisse 3 heures sous agitation un mélange de 2,5 g du composé obtenu à l'étape précédente et 10 ml de TFA. On concentre sous vide, reprend le résidu dans l'hexane et essore le précipité formé. On obtient 2 g du produit

attendu.

Préparation 1.29

Acide 3-[[5-chloro-1-[(2,4-diméthoxyphényl)sulfonyl]-3-(2-méthoxyphényl)-2-oxo-2,3-dihydro-1*H*-indol-3-yl]amino] propionique.

(II) : $R_1$ = Cl ; $R_2$ = H ; $R_3$ = $-OCH_3$ ; $R_4$ = H ; X = $-NH-CH_2-CH_2-$ ; Y = OH ;

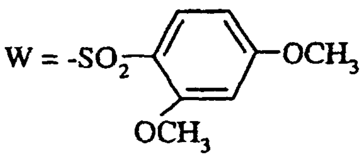

A) Ester *tert*-butylique de l'acide 3-[[5-chloro-3-(2-méthoxyphényl)-2-oxo-2,3-dihydro-1*H*-indol-3-yl]amino]propionique.

A un mélange de 3 g du composé obtenu à l'étape A de la Préparation 1.19 et 2,1 g de chlorhydrate de l'ester *tert*-butylique de la β-alanine dans 50 ml de DCM et 50 ml de THF, on ajoute 2,15 g de triéthylamine et laisse 18 heures sous agitation à TA. On concentre sous vide, extrait le résidu à l'AcOEt, lave la phase organique à l'eau, sèche sur $Na_2SO_4$ et évapore sous vide le solvant. On chromatographie le résidu sur gel de silice en éluant par le mélange DCM/AcOEt (80/20 ; v/v). On obtient 3,2 g du produit attendu après cristallisation dans le mélange DCM/éther iso, F = 170°C.

B) Ester *tert*-butylique de l'acide 3-[[5-chloro-1-[(2,4-diméthoxyphényl)sulfonyl]-3-(2-méthoxyphényl)-2-oxo-2,3-dihydro-1*H*-indol-3-yl]amino]propionique.

On prépare ce composé selon le mode opératoire décrit à l'étape B de la Préparation 1.28 à partir de 2,16 g du composé obtenu à l'étape précédente, 0,225 g d'hydrure de sodium à 60 % dans l'huile, 15 ml de DMF et 1,21 g de chlorure de 2,4-diméthoxybenzènesulfonyle. On obtient 2,4 g du produit attendu après cristallisation dans le mélange heptane/AcOEt, F = 175°C.

C) Acide 3-[[5-chloro-1-[(2,4-diméthoxyphényl)sulfonyl]-3-(2-méthoxyphényl)-2-oxo-2,3-dihydro-1*H*-indol-3-yl]amino] propionique.

On laisse 18 heures sous agitation un mélange de 2,4 g du composé obtenu à l'étape précédente et 15 ml de TFA dans 5 ml de DCM. On concentre sous vide, reprend le résidu à l'éther et essore le précipité formé. On obtient 2,2 g du produit attendu, F > 250°C.

Préparation 1.30

Acide 2-[5-chloro-3-(2-fluorophényl)-2-oxo-2,3-dihydro-1*H*-indol-3-yl]acétique, isomére dextrogyre.

(II) : $R_1$ = Cl ; $R_2$ = H ; $R_3$ = -F; $R_4$ = H ; X = $-CH_2-$ ; Y = OH ; W = H.

A) Ester éthylique de l'acide 2-[5-chloro-3-(2-fluorophényl)-2-oxo-2,3-dihydro-1*H*-indol-3-yl]acétique.

On chauffe à reflux pendant 20 heures un mélange de 2,6 g du composé obtenu à l'étape C de la Préparation 1.12, 1,84 g de bromoacétate d'éthyle, 1,66 g de KI et 2 g de $K_2CO_3$ dans 10 ml d'acétone. On filtre les sels minéraux et concentre sous vide le filtrat. On chromatographie le résidu sur gel de silice en éluant au DCM puis à l'AcOEt. On obtient 2,1 g du produit attendu après précipitation dans l'éther iso.

B) Acide 2-[5-chloro-3-(2-fluorophényl)-2-oxo-2,3-dihydro-1*H*-indol-3-yl]acétique.

On laisse 20 heures sous agitation à TA un mélange de 2 g du composé obtenu à l'étape précédente et 2 ml d'une solution de NaOH à 30 %, dans 1 ml d'eau et 50 ml d'EtOH. On concentre sous vide, reprend le résidu dans 40 ml d'eau, acidifie à pH = 1 par ajout d'HCl concentré, essore le précipité formé et le lave à l'eau. On obtient 1,9 g du

produit attendu.

C) Acide 2-[5-chloro-3-(2-fluorophényl)-2-oxo-2,3-dihydro-1*H*-indol-3-yl]acétique, isomére dextrogyre.

On chauffe à 40°C un mélange de 0,97 g du composé obtenu à l'étape précédente et 0,89 g de (+)-cinchonine ($\alpha_D^{20}$ = + 225° ; c = 0,5 ; EtOH) dans 31,5 ml de MeOH, puis chauffe à reflux. On essore à chaud le précipité formé, le lave au MeOH chaud puis à l'éther et obtient 0,485 g du sel avec la (+)-cinchonine. On reprend 0,485 g du sel ainsi obtenu dans un mélange eau/AcOEt, acidifie à pH = 0 par ajout d'HCl 3N, laisse sous agitation, extrait à l'AcOEt, lave la phase organique à l'eau, sèche sur $Na_2SO_4$ et évapore sous vide le solvant. On obtient 0,22 g du produit attendu sous forme d'isomère dextrogyre.
$\alpha_D^{20}$ = +61,3° (c = 0,15 ; chloroforme).

Préparation 1.31

Acide 2-[5,6-dichloro-3-(2-fluorophényl)-2-oxo-2,3-dihydro-1*H*-indol-3-yl acétique.

(II) : $R_1$ = Cl ; $R_2$ = 6-Cl ; $R_3$ = -F; $R_4$ = H ; X = $-CH_2-$ ; Y = OH ; W = H.

A) N-(3,4-dichlorophényl)-DL-2-fluoromandélamide.

On chauffe à reflux pendant 3 heures un mélange de 7,5 g du composé obtenu à l'étape A de la Préparation 1.12 et 7,5 g de 3,4-dichloroaniline dans 40 ml de 1,2-dichlorobenzène en éliminant l'eau formée à l'aide d'un appareil de Dean-Stark. On concentre sous vide, reprend le résidu dans l'éther iso et essore le précipité formé. On obtient 9 g du produit attendu.

B) 5,6-Dichloro-3-(2-fluorophényl)-1,3-dihydro-2*H*-indol-2-one.

A un mélange de 36 ml d'$H_2SO_4$ concentré (95 %) et 9 ml d'acide sulfurique fumant (oléum à 30 %), on ajoute, à TA, 8,9 g du composé obtenu à l'étape précédente, puis laisse 8 heures sous agitation. On verse le mélange réactionnel sur un mélange glace/eau, essore le précipité formé et le lave à l'eau. On chromatographie le précipité sur gel de silice en éluant au DCM puis à l'éther iso. On sépare les deux isomères :

- le moins polaire, composé de l'étape B et obtient 3,7 g, F = 204-205°C ;
- le plus polaire, le 4,5-dichloro-3-(2-fluorophényl)-1,3-dihydro-2H-indol-2-one et obtient 1,5 g, F = 244-247°C.

B) Ester éthylique de l'acide 2-[5,6-dichloro-3-(2-fluorophényl)-2-oxo-2,3-dihydro-1*H*-indol-3-yl]acétique.

On chauffe à reflux pendant 10 heures un mélange de 3 g du composé obtenu à l'étape précédente, 1,7 g de bromoacétate d'éthyle, 1,8 g et 1,4 g de KI de $K_2CO_3$ dans 20 ml d'acétone. On concentre sous vide, reprend le résidu à l'eau, extrait à l'AcOEt, sèche la phase organique sur $Na_2SO_4$ et évapore le solvant sous vide. On chromatographie le résidu sur gel de silice en éluant à l'éther iso. On obtient 2,1 g du produit attendu après trituration dans le pentane puis dans l'éther iso, F = 152-158°C.

C) Acide 2-[5,6-dichloro-3-(2-fluorophényl)-2-oxo-2,3-dihydro-1*H*-indol-3-yl]acétique.

On laisse 20 heures sous agitation à TA un mélange de 2 g du composé obtenu à l'étape précédente et 3 ml d'une solution de NaOH à 30 % dans 5 ml d'eau et 40 ml d'EtOH. On concentre sous vide, reprend le résidu à l'eau, lave la phase aqueuse à l'éther, acidifie la phase aqueuse à pH = 1 par ajout d'HCl concentré, extrait à l'AcOEt, sèche la phase organique sur $Na_2SO_4$ et évapore le solvant sous vide. On obtient 1,5 g du produit attendu après trituration dans l'éther iso, F = 245°C.

Préparation 1.32

Acide 2-[5-chloro-3-(2,3-diméthoxyphényl)-2-oxo-2,3-dihydro-1*H*-indol-3-yl]acétique.

(II) : $R_1$ = Cl ; $R_2$ = H ; $R_3$ = $OCH_3$ ; $R_4$ = 3-$OCH_3$ ; X = $-CH_2-$ ; Y = OH ; W = H.

A) 2-(2,3-Diméthoxyphényl)-2-oxoacétate d'éthyle.

On refroidit à -40°C un mélange de 27,6 g de 1,2-diméthoxybenzène dans 160 ml d'éther, ajoute, goutte à goutte, 250 ml d'une solution 1,6 M de n-butyllithium dans l'hexane, puis laisse 24 heures sous agitation en laissant remonter la température à TA. On refroidit le mélange réactionnel à -20°C, ajoute rapidement 136 ml d'oxalate de diéthyle et laisse sous agitation en laissant remonter la température à TA. Après 30 minutes d'agitation à TA, on verse le mélange réactionnel sur une solution saturée de $NH_4Cl$, décante, extrait la phase aqueuse à l'éther, lave les phases organiques jointes deux fois à l'eau, sèche sur $Na_2SO_4$ et évapore sous vide les solvants. On élimine l'oxalate de diéthyle en excès par distillation sous vide (Eb=90°C sous 2400Pa). On chromatographie le produit brut résultant sur gel de silice en éluant par le mélange heptane/éther iso (90/10 ; v/v). On obtient 25 g du produit attendu que l'on utilise tel quel à l'étape suivante.

B) 5-Chloro-3-hydroxy-3-(2,3-diméthoxyphényl)-1,3-dihydro-2*H*-indol-2-one.

a) 4-Chlorophénylcarbamate de *tert*-butyle.
On laisse 24 heures sous agitation à TA un mélange de 12,7 g de 4-chloroaniline et 22 g de di-*tert*-butyldicarbonate dans 60 ml de dioxane. On concentre sous vide le mélange réactionnel, reprend le résidu au pentane, essore le précipité formé et le sèche. On obtient 22,5 g du produit attendu.

b) On refroidit à -40°C, sous atmosphère d'azote sec, un mélange de 11,4 g de 4-chlorophénylcarbamate de *tert*-butyle dans 100ml d'éther, ajoute, goutte à goutte, 80 ml d'une solution 1,5 M de *tert*-butyllithium dans le pentane et laisse 3 heures sous agitation à -20°C. On refroidit le mélange réactionnel à -40°C, ajoute, en une heure, une solution de 14 g du composé obtenu à l'étape A dans 50 ml de THF et laisse 4 jours sous agitation à TA. On verse le mélange réactionnel sur une solution saturée de $NH_4Cl$, essore le précipité formé et le sèche. On obtient 10,2 g du produit attendu que l'on utilise tel quel à l'étape suivante.

C) 5-Chloro-3-(2,3-diméthoxyphényl)-1,3-dihydro-2*H*-indol-2-one.

On chauffe à reflux pendant 5 heures un mélange de 8,5 g du composé obtenu à l'étape précédente, 40 ml de TFA et 11,5 ml de triéthylsilane. On concentre sous vide, reprend le résidu à l'eau, extrait à l'AcOEt, essore le précipité formé, le lave à l'AcOEt puis à l'éther iso et obtient 5,1 g du produit attendu. On décante les jus d'essorage, sèche sur la phase organique sur $Na_2SO_4$ et évapore le solvant sous vide. On obtient de plus 1,4 g du produit attendu après cristallisation dans l'AcOEt, F = 193°C.

D) Ester éthylique de l'acide 2-[5-chloro-3-(2,3-diméthoxyphényl)-2-oxo-2,3-dihydro-1*H*-indol-3-yl]acétique.

On chauffe à 60°c pendant 8 heures un mélange de 6,2 g du composé obtenu à l'étape précédente, 3,6 g de bromoacétate d'éthyle, 3,5 g de KI et 6,2 g de $K_2CO_3$ dans 20 ml de DMF. On concentre sous vide, reprend le résidu à l'eau, extrait à l'AcOEt, lave la phase organique à l'eau, sèche sur $Na_2SO_4$ et évapore sous vide. On chromatographie le résidu sur gel de silice en éluant par le mélange DCM/MeOH (99/1 ; v/v). On obtient 3 g du produit attendu, F = 155°C.

E) Acide 2-[5-chloro-3-(2,3-diméthoxyphényl)-2-oxo-2,3-dihydro-1*H*-indol-3-yl]acétique.

On laisse 24 heures sous sous agitation à TA un mélange de 3 g du composé obtenu à l'étape précédente et 0,5 g de NaOH en pastilles dans 40 ml d'eau et 10 ml de dioxane. On ajoute 150 ml d'eau, lave la phase aqueuse à l'AcOEt, acidifie la phase aqueuse à pH = 1 par ajout d'HCl concentré, essore le précipité formé, le lave à l'eau et le sèche. On obtient 2,5 g du produit attendu, F = 235°C.

Préparation 1.33

Acide 2-[5-chloro-3-(2-éthoxyphényl)-6-méthyl-2-oxo-2,3-dihydro-1*H*-indol-3-yl]acétique.

(II) : $R_1$ = Cl ; $R_2$ = 6-$CH_3$ ; $R_3$ = $OCH_2CH_3$ ; $R_4$ = H ; X = -$CH_2$- ; Y = OH ; W = H.

A) 5-Chloro-3-(2-éthoxyphényl)-3-hydroxy-6-méthyl-1,3-dihydro-2*H*-indol-2-one.

On prépare ce composé selon le mode opératoire décrit à l'étape C de la Préparation 1.7 à partir du composé

obtenu à l'étape A de la Préparation 1.2 et du composé obtenu à l'étape B de la Préparation 1.7. On obtient 1,05 g du produit attendu, F = 253°C.

B) 5-Chloro-3-(2-éthoxyphényl)-6-méthyl-1,3-dihydro-2*H*-indol-2-one.

On chauffe à reflux pendant 5 heures un mélange de 1,05 g du composé obtenu à l'étape précédente, 5,5 ml de TFA et 2,2 ml de triéthylsilane. On concentre sous vide et chromatographie le résidu sur gel de silice en éluant au DCM puis par le mélange DCM/MeOH (99,5/0,5 ; v/v). On obtient 0,5 g du produit attendu, F = 228°C.

C) Ester éthylique de l'acide 2-[5-chloro-3-(2-éthoxyphényl)-6-méthyl-2-oxo-2,3-dihydro-1*H*-indol-3-yl]acétique.

On prépare ce composé selon le mode opératoire décrit à l'étape C de la Préparation 1.1 à partir de 0,5 g du composé obtenu à l'étape précédente, 0,29 g de bromoacétate d'éthyle, 0,29 g de KI et 0,46 g de $K_2CO_3$ dans 5 ml d'acétone. On obtient 0,25 g du produit attendu.

D) Acide 2-[5-chloro-3-(2-éthoxyphényl)-6-méthyl-2-oxo-2,3-dihydro-1*H*-indol-3-yl]acétique.

On prépare ce composé selon le mode opératoire décrit à l'étape E de la Préparation 1.2 à partir de 0,25 g du composé obtenu à l'étape précédente, 0,3 ml d'une solution de NaOH à 30 % dans 2,8 ml d'eau et 7 ml de THF. On obtient 0,2 g du produit attendu.

Préparation 1.34

5-Chloro-1-[(2,4-diméthoxyphényl)sulfonyl]-3-(2-éthoxyphényl)-2-oxo-2,3-dihydro-1*H*-indol-3-yl phénylcarbonate.

(II) : $R_1$ = Cl ; $R_2$ = H ; $R_3$=$OCH_2CH_3$ ; $R_4$ = H ; X = -O- ;

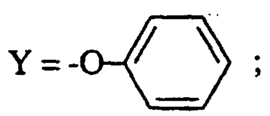

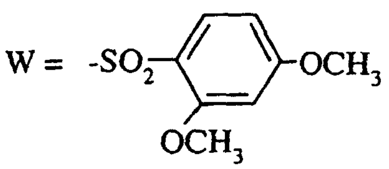

A) 5-Chloro-3-(2-éthoxyphényl)-3-[(triméthylsilyl)oxy]-1,3-dihydro-2*H*-indol-2-one.

On prépare ce composé selon le mode opératoire décrit à l'étape A) de la Préparation 1.8 à partir de 2 g du composé de l'étape B de la Préparation 1.2, 0,1 g de chlorure de zinc, 6,6 ml d'HMDS et 30 ml d'acétonitrile. On obtient 1,5 g du produit attendu après trituration dans l'heptane.

B) 5-Chloro-1-[(2,4-diméthoxyphényl)sulfonyl]-3-(2-éthoxyphényl)-3-[(triméthylsilyl)oxy]-1,3-dihydro-2*H*-indol-2-one.

On prépare ce composé selon le mode opératoire décrit à l'étape B de la Préparation 1.8 à partir de 1,45 g du composé obtenu à l'étape précédente, 20 ml de THF, 0,05 g d'hydrure de sodium à 60 % dans l'huile et 1 g de chlorure de 2,4-diméthoxybenzènesulfonyle. On obtient 1,6 g du produit attendu après trituration dans l'éther iso.

C) 5-Chloro-1-[(2,4-diméthoxyphényl)sulfonyl]-3-(2-éthoxyphényl)-3-hydroxy-1,3-dihydro-2*H*-indol-2-one.

On laisse 3 heures sous agitation à TA un mélange de 1,55 g du composé obtenu à l'étape précédente et 3 ml d'HCl 12N dans 100 ml d'acétone. On concentre sous vide et chromatographie le résidu sur gel de silice en éluant au DCM. On obtient 1,4 g du produit attendu après trituration dans l'éther iso.

D) 5-Chloro-1-[(2,4-diméthoxyphényl)sulfonyl]-3-(2-éthoxyphényl)-2-oxo-2,3-dihydro-1*H*-indol-3-yl phénylcarbonate.

On refroidit à 10°C un mélange de 1,35 g du composé obtenu à l'étape précédente dans 20 ml de pyridine, ajoute 2 g de chloroformiate de phényle et laisse 20 heures sous agitation en laissant remonter la température à TA. On concentre sous vide, reprend le résidu par une solution d'HCl 1N, extrait à l'éther iso, essore le précipité formé et le lave à l'éther iso. On obtient 1,8 g du produit attendu, F = 210-211°C (déc).

Préparations des composés de formule (III).

Préparation 2.1

1-(2-Pyrazinyl)pipérazine.

(III) : n = 1 ;

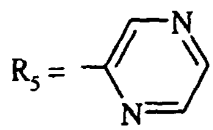

On chauffe 48 heures à reflux un mélange de 3 g de pipérazine, 1,04 ml de 2-chloropyrazine et 1,85 g de $K_2CO_3$ dans 100 ml d'EtOH. On concentre le mélange réactionnel sous vide, reprend le résidu à l'eau, alcalinise à pH = 10 par ajout de NaOH à 10 %, extrait au chloroforme, lave la phase organique à l'eau, sèche sur $Na_2SO_4$ et évapore sous vide le solvant. On obtient 1,8 g du produit attendu après cristallisation dans l'hexane.

Préparation 2.2

1-(3-pyridinyl)pinérazine

(III) : n = 1 ;

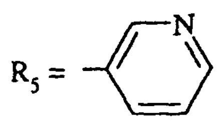

On prépare ce composé selon le procédé décrit dans Tetrahedron Letters, 1998, 39, 617-620.

Préparation 2.3

1-(2-pyridinyl)homopipérazine.

(III) : n = 2 ;

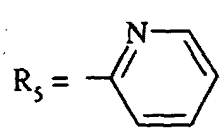

On chauffe à 100°C pendant 6 heures un mélange de 2-bromopyridine et 12 g d'homopipérazine. On ajoute 50 ml d'eau au mélange réactionnel, extrait à l'AcOEt, lave la phase organique par une solution saturée de NaCl, sèche sur $Na_2SO_4$ et évapore sous vide le solvant. On obtient 1,28 g du produit attendu.

Préparation 2.4

1-(4-pyridinyl)homopipérazine.

(III) : n = 2 ;

$R_5$ = 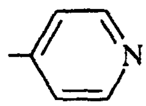

On chauffe à 100°C pendant 4 heures un mélange de 2 g de 4-bromopyridine et 10 g d'homopipérazine. On ajoute 100 ml d'eau au mélange réactionnel, alcalinise à pH = 10 par ajout d'une solution à 10 % de NaOH, extrait trois fois par 100 ml de chloroforme, sèche la phase organique sur $Na_2SO_4$ et évapore sous vide le solvant. On obtient 0,9 g du produit attendu.

Préparation 2.5

Trichlorhydrate de 1-(3-pyridazinyl)pipérazine.

(III), 3HCl : n = 1

$R_5$ = 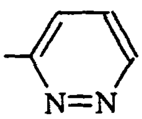

A) 4-(6-Chloro-3-pyridazinyl)-1-pipérazinecarboxylate de *tert*-butyle.

On chauffe pendant 5 heures à reflux un mélange de 13,52 g de 1-pipérazine carboxylate de *tert*-butyle, 10,81 g de 3,6-dichloropyridazine et 20 ml de triéthylamine dans 100 ml de n-butanol. On concentre sous vide et chromatographie le résidu sur gel de silice en éluant par le mélange DCM/AcOEt (90/10 ; v/v). On obtient 14 g du produit attendu que l'on utilise tel quel.

B) 4-(3-Pyridazinyl)-1-pipérazinecarboxylate de *tert*-butyle.

On hydrogène pendant une nuit, à TA et pression atmosphérique, un mélange de 10,5 g du composé obtenu à l'étape précédente et 2,5 g de palladium sur charbon à 10 % dans 30 ml de DMF et 250 ml d'EtOH. On filtre le catalyseur et concentre sous vide le filtrat. On chromatographie le résidu sur gel de silice en éluant par le mélange DCM/MeOH de (97/3 ; v/v) à (90/10 ; v/v). On obtient 9,1 g du produit attendu que l'on utilise tel quel.

C) Trichlorhydrate de 1-(3-pyridazinyl)pipérazine

On laisse une nuit sous agitation à TA un mélange de 3,8 g du composé obtenu à l'étape précédente, 50 ml d'une solution 2N d'HCl dans l'éther et 20 ml de MeOH. On concentre sous vide, reprend le résidu dans l'éther et essore le précipité formé. On obtient 3 g du produit attendu.

Préparation 2.6

Dichlorhydrate de 1-(1,3-thiazol-2-yl)pipérazine.

(III), 2HCl : n = 1 ;

$R_5 =$ 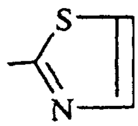

A) 4-(1,3-Thiazol-2-yl)-1-pipérazinecarboxylate de *tert*-butyle.

On chauffe pendant 4 jours à reflux un mélange de 5 g de 1-pipérizinecarboxylate de *tert*-butyle, 4,4 g de 2-bromo-1,3-thiazole et 7,4 g de $K_2CO_3$ dans 50 ml d'EtOH. On ajoute de l'eau au mélange réactionnel, évapore sous vide l'EtOH, extrait la phase aqueuse résultante à l'AcOEt, lave la phase organique par une solution saturée de $K_2CO_3$, par une solution saturée de NaCl, sèche sur $Na_2SO_4$ et évapore le solvant sous vide. On chromatographie le résidu sur gel de silice en éluant par le mélange DCM/MeOH (98/2 ; v/v). On obtient 5 g du produit attendu après précipitation à froid dans le mélange DCM/hexane et essorage, F = 114-116°C.

B) Dichlorhydrate de 1-(1,3-thiazol-2-yl)pipérazine.

On laisse 7 heures sous agitation à TA un mélange de 2,8 g du composé obtenu à l'étape précédente et 50 ml d'une solution d'HCl 2N dans l'éther en ayant ajouté au préalable un minimum de DCM puis de MeOH jusqu'à dissolution du mélange réactionnel. On concentre sous vide et obtient 2,35 g du produit attendu.

EXEMPLE 1

5-Chloro-1-[(2,4-diméthoxyphényl)sulfonyl]-3-(2-méthoxyphényl)-3-[2-oxo-2-[4-(4-pyridinyl)-1-pipérazinyl]éthyl]-1,3-dihydro-2*H*-indol-2-one.

(I) : $R_1$ = Cl; $R_2$ = H ; $R_3$ = $OCH_3$ ; $R_4$ = H ; X = $-CH_2-$ ; n = 1 ;

$R_5 =$ 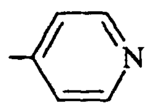 ;

$R_6$ = 2- $OCH_3$ ; $R_7$ = $OCH_3$

A) 5-Chloro-3-(2-méthoxyphényl)-3-[2-oxo-2-[4-(4-pyridinyl)-1-pipérazinyl]éthyl]-1,3-dihydro-2*H*-indol-2-one.

A un mélange de 1,1 g du composé obtenu à la Préparation 1.1 dans 20 ml de DCM, on ajoute, à 20°C, 1,7 g de BOP, 2,5 ml de DIPEA puis 0,6 g de 1-(4-pyridinyl) pipérazine et laisse 2 heures sous agitation à 20°C. On ajoute ensuite 30 ml de NaOH 2N et laisse 15 minutes sous agitation. On extrait le mélange réactionnel à l'AcOEt, lave la phase organique à l'eau, sèche sur $Na_2SO_4$ et évapore sous vide les solvants. On triture le résidu dans l'éther iso et essore le précipité formé. On chromatographie le précipité sur gel de silice en éluant au DCM puis à l'acétone. On obtient 1,4 g du produit attendu après cristallisation dans l'éther iso, F = 111°C.

B) 5-Chloro-1-[(2,4-diméthoxyphényl)sulfonyl]-3-(2-méthoxyphényl)-3-[2-oxo-2-[4-(4-pyridinyl)-1-pipérazinyl]éthyl]-1,3-dihydro-2*H*-indol-2-one.

A un mélange de 0,7 g du composé obtenu à l'étape précédente dans 15 ml de THF, on ajoute, à 20°C, 0,08 g d'hydrure de sodium à 60 % dans l'huile et laisse 20 minutes sous agitation. On ajoute ensuite 0,44 g de chlorure de 2,4-diméthoxybenzène sulfonyle et laisse 1 heure 30 minutes sous agitation. On concentre sous vide le mélange réactionnel, extrait le résidu à l'AcOEt, lave la phase organique à l'eau, sèche sur $Na_2SO_4$ et évapore sous vide le solvant. On chromatographie le résidu sur gel de silice en éluant au DCM, puis à l'AcOEt et à l'acétone. On obtient 0,7 g du produit attendu après cristallisation dans l'éther iso, F = 136-141°C (déc).

EXEMPLE 2

5-Chloro-3-(2-éthoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one.

(I) : $R_1$ = Cl ; $R_2$ = H ; $R_3$ = $OCH_2CH_3$ ; $R_4$ = H ; X = $-CH_2-$ ; n = 1 ;

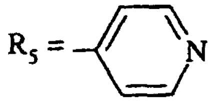 ;

$R_6$ = 2- $OCH_3$ ; $R_7$ = $OCH_3$

A) 5-Chloro-3-(2-éthoxyphényl)-3-[2-oxo-2-[4-(4-pyridinyl)-1-pipérazinyl]éthyl]-1,3-dihydro-2*H*-indol-2-one.

On laisse 24 heures sous agitation à TA un mélange de 1,1 g du composé obtenu à la Préparation 1.2, 1,52 g de BOP, 2 ml de DIPEA et 0,6 g de 1-(4-pyridinyl)pipérazine dans 20 ml de DCM. On essore le produit cristallisé formé, le sèche et obtient 0,81 g du produit attendu. On extrait les jus d'essorage par une solution d'HCl concentré, décante, ajoute de la glace à la phase acide, alcalinise la phase acide par ajout de NaOH 10N, extrait à l'AcOEt, lave la phase organique à l'eau, sèche sur $Na_2SO_4$ et évapore sous vide le solvant. On obtient 0,09 g du produit attendu supplémentaire, F = 185°C.

B) 5-Chloro-3-(2-éthoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one.

A un mélange de 0,9 g du composé obtenu à l'étape précédente dans 20 ml du mélange THF/DMF (90/10 ; v/v) on ajoute 0,085 g d'hydrure de sodium à 60 % dans l'huile et laisse 15 minutes sous agitation à TA. On ajoute ensuite 0,45 g de chlorure de 2,4-diméthoxybenzènesulfonyle et laisse 30 minutes sous agitation à TA. On verse le mélange réactionnel dans l'eau, extrait à l'AcOEt, lave la phase organique à l'eau, sèche sur $Na_2SO_4$ et évapore sous vide le solvant. On chromatographie le résidu sur gel de silice en éluant par le mélange DCM/MeOH (96/4 ; v/v). On obtient 0,42 g du produit attendu après cristallisation dans l'éther iso, F = 225°C.

EXEMPLE 3

5-Chloro-3-(2-isopropoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one, isomère lévogyre.

(I) : $R_1$ = Cl; $R_2$ = H ; $R_3$ = $OCH(CH_3)_2$ ; $R_4$ = H ; X = $-CH_2-$ ; n = 1 ;

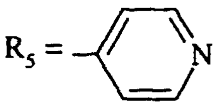 ;

$R_6$ = 2- $OCH_3$ ; $R_7$ = $OCH_3$

A) 5-Chloro-3-(2-isopropoxyphényl)-3-[2-oxo-2-[4-(4-pyridinyl)-1-pipérazinyl]éthyl]-1,3-dihydro-2*H*-indol-2-one, isomère dextrogyre.

On laisse 2 heures sous agitation à 20°C un mélange de 0,43 g du composé obtenu à la Préparation 1.3, 0,6 g de BOP, 0,75 g de DIPEA et 0,2 g de 1-(4-pyridinyl) pipérazine dans 15 ml de DCM. On ajoute ensuite 25 ml de NaOH 2N et laisse 20 minutes sous agitation à 20°C. On extrait le mélange réactionel à l'AcOEt, lave la phase organique à l'eau, sèche sur $Na_2SO_4$ et évapore sous vide le solvant. On chromatographie le résidu sur gel de silice en éluant au DCM puis à l'acétone. On obtient 0,33 g du produit attendu.
$\alpha_D^{20}$ = + 37° (c = 0,25 ; chloroforme).

B) 5-Chloro-3-(2-isopropoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one, isomère lévogyre.

A un mélange de 0,3 g du composé obtenu à l'étape précédente dans 10 ml de THF, on a joute 0,025 g d'hydrure de sodium à 60 % dans l'huile et laisse 15 minutes sous agitation à 20°C. On ajoute ensuite 0,19 g de chlorure de 2,4-diméthoxybenzène sulfonyle et laisse 2 heures sous agitation à 20°C. On concentre sous vide le mélange réactionnel, reprend le résidu à l'eau, extrait à l'AcOEt, sèche la phase organique sur $Na_2SO_4$ et évapore sous vide le solvant. On chromatographie le résidu sur gel de silice en éluant au DCM puis à l'acétone. On obtient 0,1 g du produit attendu.

$\alpha_D^{20}$ = - 21,3° (c = 0,1 ; DCM).

RMN$^1$H : DMSO-$d_6$ : δ (ppm) : 0,6 : d : 3H ; 1,2 : d : 3H ; 3,0 à 4,0 : m+2s : 16H ; 4,6 : mt : 1H ; 6,4 à 7,2 : mt : 9H ; 7,4 : de : 1H ; 7,7 : dd : 2H ; 8,1 : d : 2H.

EXEMPLE 4

3-(2-Chlorophényl)-1-[(2,4-diméthoxyphényl)sulfonyl]-5-méthyl-3-[2-oxo-2-[4-(2-pyridinyl)-1-pipérazinyl]éthyl]-1,3-dihydro-2*H*-indol-2-one.

(I) : $R_1 = CH_3$ ; $R_2 = H$ ; $R_3 = Cl$ ; $R_4 = H$ ; X = $-CH_2-$ ; n = 1 ;

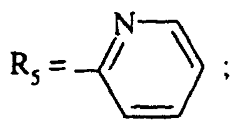

$R_6$ = 2- $OCH_3$ ; $R_7 = OCH_3$

A) 3-(2-Chlorophényl)-5-méthyl-3-[2-oxo-2-[4-(2-pyridinyl)-1-pipérazinyl]éthyl]-1,3-dihydro-2*H*-indol-2-one.

On chauffe à reflux pendant 2 heures un mélange de 0,26 g du composé obtenu à la Préparation 1.4 et 0,2 ml de chlorure de thionyle dans 10 ml de toluène puis concentre sous vide le mélange réactionnel. On dissout le chlorure d'acide ainsi obtenu dans 10 ml de DCM, ajoute cette solution à un mélange de 0,3 g de 1-(2-pyridinyl) pipérazine dans 20 ml de DCM et laisse 2 heures sous agitation à TA. On concentre sous vide le mélange réactionnel, extrait le résidu à l'AcOEt, lave la phase organique à l'eau, sèche sur $Na_2SO_4$ et évapore sous vide le solvant. On chromatographie le résidu sur gel de silice en éluant par le mélange DCM/MeOH (98/2 ; v/v). On obtient 0,32 g du produit attendu.

B) 3-(2-Chlorophényl)-1-[(2,4-diméthoxyphényl)sulfonyl]-5-méthyl-3-[2-oxo-2-[4-(2-pyridinyl)-1-pipérazinyl]éthyl]-1,3-dihydro-2*H*-indol-2-one.

A un mélange de 0,3 g du composé obtenu à l'étape précédente dans 10 ml de THF, on ajoute, à TA, 0,0335 g d'hydrure de sodium à 60 % dans l'huile et laisse 30 minutes sous agitation. On ajoute ensuite 0,2 g de chlorure de 2,4-diméthoxybenzène sulfonyle et laisse 1 heure sous agitation à TA. On ajoute 50 ml d'eau au mélange réactionnel, extrait à l'AcOEt, sèche la phase organique sur $Na_2SO_4$ et évapore sous vide le solvant. On chromatographie le résidu sur gel de silice en éluant par le mélange DCM/MeOH (98/2 ; v/v). On obtient 0,32 g du produit attendu après cristallisation dans l'éther iso, F = 239°C.

EXEMPLE 5

4-(2-Pyridinyl)-1-pipérazinecarboxylate de 5-chloro-1-[(2,4-diméthoxyphényl) sulfonyl]-3-(2-méthoxyphényl)-2-oxo-2,3-dihydro-1*H*-indol-3-yle.

(I) : $R_1 = Cl$ ; $R_2 = H$ ; $R_3 = OCH_3$ ; $R_4 = H$ ; X = -O- ; n = 1 ;

$R_5$ = 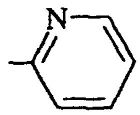 ;

$R_6$ = 2- $OCH_3$ ; $R_7$ = $OCH_3$

On laisse 20 heures sous agitation à 20°C un mélange de 0,5 g du composé obtenu à la Préparation 1.8 et 0,3 g de 1-(2-pyridinyl)pipérazine dans 10 ml de DCM. On chromatographie le mélange réactionnel sur gel de silice en éluant au DCM puis à l'AcOEt. On obtient 0,2 g du produit attendu après cristallisation dans l'éther iso, F = 210-215°C.

EXEMPLE 6

4-(4-Pyridinyl)-1-pipérazinecarboxylate de 5-chloro-1-[(2,4-diméthoxyphényl) sulfonyl]-3-(2-méthoxyphényl)-2-oxo-2,3-dihydro-1*H*-indol-3-yle.

(I) : $R_1$ = Cl; $R_2$ = H ; $R_3$ = $OCH_3$ ; $R_4$ = H ; X = -O- ; n= 1 ;

$R_5$ = 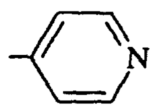

$R_6$ = 2-$OCH_3$ ; $R_7$ = $OCH_3$.

On laisse 20 heures sous agitation à 20°C un mélange de 0,32 g du composé obtenu à la Préparation 1.8 et 0,32 g de 1-(4-pyridinyl)pipérazine dans 15 ml de DCM. On concentre sous vide, extrait le résidu à l'AcOEt, lave la phase organique par une solution de NaOH 2N, à l'eau, sèche sur $Na_2SO_4$ et évapore sous vide le solvant. On chromatographie le résidu sur gel de silice en éluant au DCM, puis à l'AcOEt et enfin à l'acétone. On obtient 0,25 g du produit attendu après cristallisation dans l'éther iso, F = 194-198°C.

EXEMPLE 7

4-(4-Pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle.

(I) : $R_1$ = Cl ; $R_2$ = H ; $R_3$ = $OCH(CH_3)_2$ ; $R_4$ = H ; X = -O- ; n = 1 ;

$R_5$ = 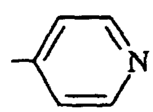 ;

$R_6$ = 2- $OCH_3$ ; $R_7$ = $OCH_3$

On laisse 24 heures sous agitation à TA un mélange de 0,66 g du composé obtenu à la Préparation 1.9 et 0,45 g de 1-(4-pyridinyl)pipérazine dans 20 ml de DCM. On lave le mélange réactionnel à l'eau, sèche la phase organique sur $Na_2SO_4$ et évapore sous vide le solvant. On chromatographie le résidu sur gel de silice en éluant par le mélange DCM/MeOH (99/1 ; v/v). On obtient 0,41 g du produit attendu après cristallisation dans l'AcOEt, F = 253°C.

EXEMPLE 8

1,5-Fumarate de 4-(4-pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle.

(I) : 1,5 $C_2H_2O_4$ : $R_1$ = Cl; $R_2$ = H ; $R_3$ = $OCH(CH_3)_2$ ; $R_4$ = H ; X = -O= ; n = 1 ;

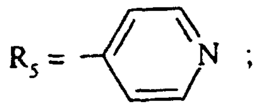

$R_6$ = 2- $OCH_3$ ; $R_7$ = $OCH_3$

On chauffe à reflux pendant 3 heures un mélange de 0,3 g du composé obtenu à l'Exemple 7 et 0,056 g d'acide fumarique dans 15 ml d'acétonitrile. On essore à chaud le précipité formé, le lave à l'éther et le sèche. On obtient 0,24 g du produit attendu, F = 235°C.

EXEMPLE 9

4-(4-Pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle, isomère lévogyre.

(I) : $R_1$ = Cl ; $R_2$ = H ; $R_3$ = $OCH(CH_3)_2$ ; $R_4$ = H ; X = -O- ; n = 1 ;

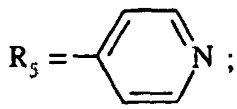

$R_6$ = 2-$OCH_3$ ; $R_7$ = $OCH_3$.

A) 5-Chloro-3-(2-isopropoxyphényl)-2-oxo-3-[[[4-(4-pyridinyl)-1-pipérazinyl] carbonyl]oxy]-1-indolinecarboxylate de phényle .

On laisse 24 heures sous agitation à TA un mélange de 6 g du composé obtenu à la Préparation 1.10 et 1,8 g de 1-(4-pyridinyl)pipérazine dans 60 ml de DCM. On concentre partiellement sous vide le solvant et chromatographie directement la solution résultante sur gel de silice en éluant par le mélange AcOEt/MeOH (95/5 ; v/v). On obtient 4,0 g du produit attendu.

B) 4-(4-Pyridinyl)-1-pipérazinecarboxylate de 5-chloro-1-[[[(1S)-1-(hydroxyméthyl)-3-méthylbutyl]amino]carbonyl]-3-(2-isopropoxyphényl)-2-oxo-2,3-dihydro-1*H*-indol-3-yle, isomère le moins polaire et isomère le plus polaire.

On laisse 48 heures sous agitation un mélange de 3,8 g du composé obtenu à l'étape précédente et 2,16 g de L-Leucinol dans 50 ml de chloroforme. On concentre sous vide, reprend le résidu au DCM et chromatographie la suspension ainsi obtenu sur alumine en éluant par le mélange DCM/MeOH (99/1 ; v/v). On rechromatographie sur gel de silice en éluant par le mélange DCM/MeOH (98,5/1,5 ; v/v). On sépare les diastéréoisomères :

- isomère le moins polaire : on obtient 0,53 g.
  $\alpha_D^{25}$ = - 19,3° (c = 0,34 ; chloroforme).

- isomère le plus polaire que l'on cristallise dans le mélange DCM/éther iso et obtient 0,548 g, F = 199-202°C.
  $\alpha_D^{25}$ = - 8,8° (c = 0,11 ; chloroforme).

C) 4-(4-Pyridinyl)-1-pipérazinecarboxylate de 5-chloro-3-(2-isopropoxyphényl)-2-oxo-2,3-dihydro-1*H*-indol-3-yle, isomère lévogyre.

On laisse 18 heures sous agitation à TA un mélange de 0,5 g du composé obtenu à l'étape précédente (isomère le plus polaire) et 0,042 g de méthylate de sodium dans 5 ml de MeOH et 5 ml de THF. On ajoute de l'eau au mélange réactionnel, concentre sous vide les solvants, extrait 4 fois la phase aqueuse résultante au DCM, sèche sur $Na_2SO_4$ et évapore sous vide le solvant. On chromatographie le résidu sur gel de silice en éluant par le mélange DCM/MeOH (92/8 ; v/v). On obtient 0,225 g du produit attendu après cristallisation dans le mélange DCM/éther iso, F = 195-245°C.
$\alpha_D^{25}$ = - 18,8° (c = 0,266 ; chloroforme).

D) 4-(4-Pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle, isomère lévogyre.

A un mélange de 0,213 g du composé obtenu à l'étape précédente dans 3 ml de DMF on ajoute, sous atmosphère d'argon, 0,02 g d'hydrure de sodium à 60 % dans l'huile, puis, après cessation du dégagement gazeux, on ajoute 0,119 g de chlorure de 2,4-diméthoxybenzènesulfonyle et laisse 3 heures sous agitation à TA. On verse le mélange réactionnel dans une solution à 5 % de $K_2CO_3$, extrait à l'AcOEt, sèche la phase organique sur $Na_2SO_4$ et évapore sous vide le solvant. On chromatographie le résidu sur gel de silice en éluant par le mélange DCM/MeOH de (95/5 ; v/v) à (93/7 ; v/v). On obtient 0,161 g du produit attendu après cristallisation dans le mélange DCM/hexane/éther iso, F = 160-164°C.
$\alpha_D^{20}$ = - 71,8° (c = 0,18 ; chloroforme).

EXEMPLE 10

4-(4-Pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle, isomère dextrogyre.

(I) : $R_1$ = Cl ; $R_2$ = H ; $R_3$ = $OCH(CH_3)_2$ ; $R_4$ = H ; X = -O- ; n = 1 ;

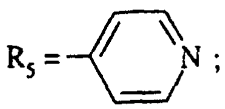

$R_6$ = 2-$OCH_3$ ; $R_7$ = $OCH_3$.

A) 4-(4-Pyridinyl)-1-pipérazinecarboxylate de 5-chloro-3-(2-isopropoxyphényl)-2-oxo-2,3-dihydro-1*H*-indol-3-yle, isomère dextrogyre.

On prépare ce composé selon le mode opératoire décrit à l'étape C de l'Exemple 9 à partir de 0,529 g du composé obtenu à l'étape B de l'Exemple 9 (isomère le moins polaire) et de 0,043 g de méthylate de sodium dans 5 ml de MeOH et 5 ml de THF. On obtient 0,198 g du produit attendu après cristallisation dans le mélange DCM/éther iso, F = 196-198°C.
$\alpha_D^{25}$ = + 20,7° (c = 0,32 ; chloroforme).

B) 4-(4-Pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle, isomère dextrogyre.

On prépare ce composé selon le mode opératoire décrit à l'étape D de l'Exemple 9 à partir de 0,328 g du composé obtenu à l'étape précédente, 0,03 g d'hydrure de sodium à 60 % dans l'huile, 4 ml de DMF et 0,18 g du chlorure de 2,4-diinéthoxybenzènesulfonyle, F = 161-167°C.
$\alpha_D^{20}$ = + 72,5° (c = 0,14 ; chloroforme).

EXEMPLE 11

4-(2-Pyridinyl)-1-pipérazinecarboxylate de 5-chloro-1-[(2,4-diméthoxyphényl) sulfonyl]-3-(2,5-diméthoxyphényl)-2-oxo-2,3-dihydro-1*H*-indol-3-yle.

(I) : $R_1$ = Cl ; $R_2$ = H ; $R_3$ = $OCH_3$ ; $R_4$ = 5-$OCH_3$ ; X = -O- ; n = 1 ;

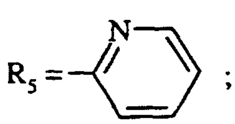

$R_6$ = 2-$OCH_3$ ; $R_7$ = $OCH_3$.

On laisse 72 heures sous agitation à 20°C un mélange de 0,4 g du composé obtenu à la Préparation 1.17 et 0,4 g de 1-(2-pyridinyl)pipérazine dans 5 ml de DCM. On concentre sous vide, reprend le résidu dans 30 ml d'eau et essore le précipité formé. On obtient 0,4 g du produit attendu après cristallisation dans le MeOH, F = 254°C.

EXEMPLE 12

4-(4-Pyridinyl)-1-pipérazinecarboxylate de 5-chloro-1-[(2,4-diméthoxyphényl) sulfonyl]-3-(2,5 diméthoxyphényl)-2-oxo-2,3-dihydro-1*H*-indol-3-yle.

(I) : $R_1$ = Cl ; $R_2$ = H ; $R_3$ = $OCH_3$ ; $R_4$ = 5-$OCH_3$ ; X = -O- ; n = 1 ;

$R_5$ = 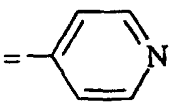 ;

$R_6$ = 2-$OCH_3$ ; $R_7$ = $OCH_3$.

On laisse 72 heures sous agitation à 20°C un mélange de 0,4 g du composé obtenu à la Préparation 1.17 et 0,4 g de 1-(4-pyridinyl)pipérazine dans 5 ml de DCM. On concentre sous vide, reprend le résidu dans 20 ml d'eau et essore le précipité formé. On reprend le précipité dans l'acétone et concentre sous vide le solvant. On chromatographie le résidu sur gel de silice en éluant au DCM, puis à l'AcOEt et enfin à l'acétone. On obtient 0,4 g du produit attendu après cristallisation dans l'éther iso, F = 247-249°C.

EXEMPLE 13

N-[5-Chloro-1-[(2,4-diméthoxyphényl)sulfonyl]-3-(2-méthoxyphényl)-2-oxo-2,3-dihydro-1*H*-indol-3-yl]-4-(4-pyridinyl) pipérazine-1-carboxamide, 0,25 $H_2O$.

(I) : $R_1$ = Cl ; $R_2$ = H ; $R_3$ = $OCH_3$ ; $R_4$ = H ; X = -NH- ; n = 1 ;

$R_5$ = 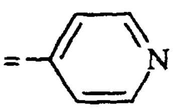 ;

$R_6$ = 2-$OCH_3$ ; $R_7$ = $OCH_3$.

On chauffe à reflux pendant 4 heures un mélange de 0,5 g du composé obtenu à la Préparation 1.18 et 0,268 g de 1-(4-pyridinyl)pipérazine dans 5 ml de chloroforme. On concentre sous vide et chromatographie le résidu sur gel de silice en éluant au DCM puis par le gradient du mélange DCM/AcOEt jusqu'à (85/15 ; v/v). On obtient 0,281 g du produit attendu après cristallisation dans le mélange DCM/éther iso/hexane.

EXEMPLE 14

N-[5-Chloro-1-[(2,4-diméthoxyphényl)sulfonyl]-3-(2-méthoxyphényl)-2-oxo-2,3-dihydro-1*H*-indol-3-yl]-4-(4-pyridinyl) pipérazine-1-carboxamide, 0,75 $H_2O$, isomère lévogyre.

(I) : $R_1$ = Cl ; $R_2$ = H ; $R_3$ = $OCH_3$ ; $R_4$ = H ; X = -NH- ; n = 1 ;

$R_5$ = 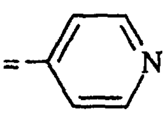 ;

$R_6$ = 2-$OCH_3$ ; $R_7$ = $OCH_3$.

On laisse 48 heures sous agitation à TA un mélange de 1,136 g du composé obtenu à la Préparation 1.19 et 0,608 g de 1-(4-pyridinyl)pipérazine dans 10 ml de chloroforme puis chauffe 1 heure à reflux. Après refroidissement à TA, on chromatographie directement le mélange réactionnel sur gel de silice en éluant par le mélange DCM/MeOH

de (95/5 ; v/v) à (92/8 ; v/v). On cristallise le produit obtenu dans le mélange DCM/hexane/éther iso puis, dissout le produit obtenu dans le mininum de MeOH et précipite par ajout d'éther iso. On obtient 0,65 g du produit attendu.
$\alpha_D^{20}$ = - 43° (c = 0,16 ; chloroforme).

EXEMPLE 15

N-[5-Chloro-1-[(2,4-diméthoxyphényl)sulfonyl]-3-(2-méthoxyphényl)-2-oxo-2,3-dihydro-1*H*-indol-3-yl]-4-(2-pyridinyl) homopipérazine-1-carboxamide.

(I) : $R_1$ = Cl ; $R_2$ = H ; $R_3$ = $OCH_3$ ; $R_4$ = H ; X = -NH- ; n = 2 ;

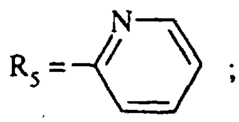

$R_6$ = 2-$OCH_3$ ; $R_7$ = $OCH_3$.

On laisse 18 heures sous agitation à TA un mélange de 0,4 g du composé obtenu à la Préparation 1.18 et 1,3 g du composé obtenu à la Préparation 2.3 dans 20 ml de DCM. On concentre sous vide, extrait le résidu à l'AcOEt, lave la phase organique par une solution saturée de NaCl, sèche sur $Na_2SO_4$ et évapore sous vide le solvant. On chromatographie le résidu sur gel de silice en éluant par le mélange DCM/MeOH (94/6 ; v/v). On obtient 0,22 g du produit attendu après cristallisation dans le mélange DCM/éther iso, F = 152°C.

EXEMPLE 16

N-[5-Chloro-1-[(2,4-diméthoxyphényl)sulfonyl]-3-(2-méthoxyphényl)-2-oxo-2,3-dihydro-1*H*-indol-3-yl]-4-(4-pyridinyl) homopipérazine-1-carboxamide, 0,8 $H_2O$.

(I) : $R_1$ = Cl ; $R_2$ = H ; $R_3$ = $OCH_3$ ; $R_4$ = H ; X = -NH- ; n = 2

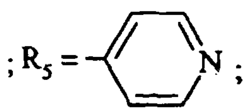

$R_6$ = 2-$OCH_3$ ; $R_7$ = $OCH_3$.

On chauffe à reflux pendant 18 heures un mélange de 0,5 g du composé obtenu à la Préparation 1.18 et 0,375 g du composé obtenu à la Préparation 2.4 dans 20 ml de DCM. On concentre sous vide, extrait le résidu à l'AcOEt, lave la phase organique par une solution à 5 % de $Na_2CO_3$, à l'eau, par une solution saturée de NaCl, sèche sur $Na_2SO_4$ et évapore sous vide le solvant. On chromatographie le résidu sur gel de silice en éluant par le mélange DCM/MeOH (94/6 ; v/v). On obtient 0,386 g du produit attendu après cristallisation dans le mélange DCM/éther iso, F = 168°C.

EXEMPLE 17

N-[5-Chloro-1-[(2,4-diméthoxyphényl)sulfonyl]-3-(2-isopropoxyphényl)-2-oxo-2,3-dihydro-1*H*-indol-3-yl]-4-(3-pyridinyl)pipérazine-1-carboxamide, isomère dextrogyre.

(I) : $R_1$ = Cl ; $R_2$ = H ; $R_3$ = $OCH(CH_3)_2$ ; $R_4$ = H ; X = -NH- ; n = 1 ;

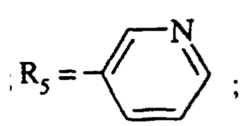

$R_6$ = 2-$OCH_3$ ; $R_7$ = $OCH_3$.

On chauffe à reflux pendant 36 heures un mélange de 0,639 g du composé obtenu à la Préparation 1.20 et 0,325 g du composé obtenu à la Préparation 2.2 dans 10 ml de chloroforme et 5 ml de THF. On chromatographie directement le mélange réactionnel sur gel de silice en éluant par le mélange DCM/MeOH (96/4 ; v/v). On obtient 0,215 g du produit attendu après cristallisation dans le mélange DCM/éther iso, F = 148°C.

$\alpha_D^{20}$ = + 25,6° (c = 0,15 ; chloroforme).

RMN$^1$H : DMSO-$d_6$ : δ (ppm) : 1,0 : d : 3H ; 1,2 : d : 3H ; 3,0 : mt : 4H ; 3,4 : mt : 4H ; 3,5 : s : 3H ; 3,9 : s : 3H ; 4,7 : mt : 1H ; 6,6 : mt : 2H ; 6,9 : t : 1H ; 7,1 : d : 1H ; 7,2 à 7,4 : m : 6H ; 7,6 : s : 1H ; 7,8 : dd : 1H ; 7,9 : d : 1H ; 8,0 : d : 1H ; 8,3 : se : 1H.

EXEMPLE 18

N-[5-Chloro-1-[(2,4-diméthoxyphényl)sulfonyl]-3-(2-isopropoxyphényl)-2-oxo-2,3-dihydro-1*H*-indol-3-yl]-4-(3-pyridinyl)pipérazine-1-carboxamide, isomère lévogyre.

(I) : $R_1$ = Cl ; $R_2$ = H ; $R_3$ = $OCH(CH_3)_2$ ; $R_4$ = H ; X = -NH- ; n = 1 ;

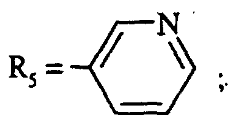

$R_6$ = 2-$OCH_3$ ; $R_7$ = $OCH_3$.

On prépare ce composé selon le mode opératoire décrit à l'Exemple 17 à partir de 0,5 g du composé obtenu à la Préparation 1.21 et 0,26 g du composé obtenu à la Préparation 2.2 dans 10 ml de chloroforme et 5 ml de THF.

$\alpha_D^{20}$ = - 33° (c = 0,15 ; chloroforme).

EXEMPLE 19

N-[5-Chloro-1-[(2,4-diméthoxyphényl)sulfonyl]-3-(2-isopropoxyphényl)-2-oxo-2,3-dihydro-1*H*-indol-3-yl]-4-(4-pyridinyl)pipérazine-1-carboxamide, isomère dextrogyre.

(I) : $R_1$ = Cl ; $R_2$ = H ; $R_3$ = $OCH(CH_3)_2$ ; $R_4$ = H ; X = -NH- ; n = 1 ;

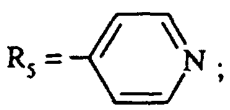

$R_6$ = 2-$OCH_3$ ; $R_7$ = $OCH_3$.

On chauffe à reflux pendant 18 heures un mélange de 0,5 g du composé obtenu à la Préparation 1.21 et 0,26 g de 1-(4-pyridinyl)pipérazine dans 5 ml de chloroforme. On chromatographie directement le mélange réactionnel sur gel de silice en éluant par le mélange DCM/MeOH de (95/5 ; v/v) à (92/8 ; v/v). On cristallise le produit obtenu par évaporation à froid d'un mélange DCM/hexane/éther iso. On obtient 0,46 g du produit attendu.

$\alpha_D^{20}$ = + 7,9° (c = 0,175 ; chloroforme).

EXEMPLE 20

N-[5-Chloro-1-[(2,4-diméthoxyphényl)sulfonyl]-3-(2-isopropoxyphényl)-2-oxo-2,3-dihydro-1*H*-indol-3-yl]-4-(4-pyridinyl)pipérazine-1-carboxamide, isomère lévogyre.

(I) : $R_1$ = Cl ; $R_2$ = H ; $R_3$ = $OCH(CH_3)_2$ ; $R_4$ = H ; X = -NH- ; n = 1 ;

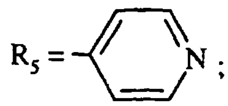

$R_6$ = 2-$OCH_3$ ; $R_7$ = $OCH_3$.

On laisse 48 heures sous agitation à TA un mélange de 0,9 g du composé obtenu à la Préparation 1.20 et 0,463 g de 1-(4-pyridinyl)pipérazine dans 10 ml de chloroforme. On chromatographie directement le mélange réactionnel sur gel de silice en éluant par le mélange DCM/MeOH (95/5 ; v/v). On cristallise le produit attendu par évaporation à froid d'un mélange DCM/hexane/éther iso. On obtient 0,789 g du produit attendu.

$\alpha^{20}_{D}$ = - 10,3° (c = 0,17 ; chloroforme).

RMN$^1$H : DMSO-$d_6$ : δ (ppm) : 1,0 : d : 6H ; 3,2 à 3,7 : m+s : 11H ; 3,8 : s : 3H ; 4,6 : mt : 1H ; 6,6 : s+mt : 2H ; 6,8 : t : 1H ; 6,9 : d : 1H ; 7,2 : d : 2H ; 7,3 : mt : 4H ; 7,6 : s : 1H ; 7,7 : d : 1H ; 7,8 : d : 1H ; 8,2 : d : 2H.

EXEMPLE 21

N-[5-Chloro-1-[(2,4-diméthoxyphényl)sulfonyl]-3-(2,5-diméthoxyphényl)-2-oxo-2,3-dihydro-1*H*-indol-3-yl]-4-(2-pyridinyl)pipérazine-1-carboxamide.

(I) : $R_1$ = Cl ; $R_2$ = H ; $R_3$ = $OCH_3$ ; $R_4$ = 5- $OCH_3$ ; X = -NH- ; n = 1 ;

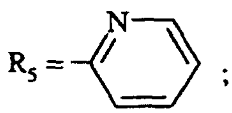

$R_6$ = 2-$OCH_3$ ; $R_7$ = $OCH_3$.

On prépare ce composé selon le mode opératoire décrit à l'Exemple 13 à partir de 0,46 g du composé obtenu à la Préparation 1.22 et 0,21 ml de 1-(2-pyridinyl)pipérazine dans 5 ml de chloroforme. On obtient 0,382 g du produit attendu après cristallisation dans le mélange DCM/éther iso/hexane.

RMN$^1$H : DMSO-$d_6$ : δ (ppm) : 3,4 à 3,8 : m+3s : 17H ; 4,0 : s : 3H ; 6,8 : mt : 2H ; 7,0 : mt : 4H ; 7,4 : mt : 3H ; 7,8 : d : 1H ; 7,9 : d : 1H ; 8,2 : mt : 2H.

EXEMPLE 22

N-[5-Chloro-1-[(2,4-diméthoxyphényl)sulfonyl]-3-(2-méthoxyphényl)-6-trifluorométhyle-2-oxo-2,3-dihydro-1*H*-indol-3-yl]-4-(4-pyridinyl)pipérazine-1-carboxamide, énantiomère unique.

(I) : $R_1$ = Cl ; $R_2$ = 6-$CF_3$ ; $R_3$ = $OCH_3$ ; $R_4$ =H ; X = -NH- ; n = 1 ;

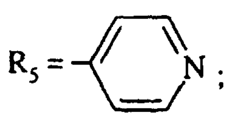

$R_6$ = 2-$OCH_3$ ; $R_7$ = $OCH_3$.

On prépare ce composé selon le mode opératoire décrit à l'Exemple 15 à partir de 0,203 g du composé obtenu à la Préparation 1.25 et 0,098 g de 1-(4-pyridinyl) pipérazine dans 3 ml de chloroforme et 3 ml de THF. On obtient 0,098 g du produit attendu après cristallisation dans le mélange DCM/éther iso, F= 174°C.

EXEMPLE 23

5-Chloro-1-[(2,4-diméthoxyphényl)sulfonyl]-3-(2-isopropoxyphényl)-3-[2-oxo-2-[4-(4-pyridinyl)-1-pipérazinyl]éthoxy]-1,3-dihydro-2*H*-indol-2-one.

(I) : $R_1$ = Cl ; $R_2$ = H ; $R_3$ = $OCH(CH_3)_2$ ; $R_4$ = H ; X = -O-$CH_2$- ; n = 1 ;

$R_5$ = 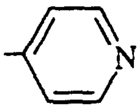 ;

$R_6$ = 2- $OCH_3$ ; $R_7$ = $OCH_3$

A) 5-Chloro-3-(2-isopropoxyphényl)-3-[2-oxo-2-[4-(4-pyridinyl)-1-pipérazinyl] éthoxy]-1,3-dihydro-2*H*-indol-2-one.

A un mélange de 1 g du composé obtenu à la Préparation 1.27 dans 20 ml de DCM, on ajoute à 20°C 1,2 g de BOP, 2 ml de DIPEA puis 0,48 g de 1-(4-pyridinyl) pipérazine et laisse 1 heure sous agitation à TA. On ajoute ensuite 20 ml de NaOH 2N, extrait à l'AcOEt, lave la phase organique à l'eau, sèche sur $Na_2SO_4$ et évapore sous vide les solvants. On chromatographie le résidu sur gel de silice en éluant au DCM puis à l'AcOEt et à l'acétone. On obtient 0,55 g du produit attendu après cristallisation dans l'éther iso, F = 115-120°C.

B) 5-Chloro-1-[(2,4-diméthoxyphényl)sulfonyl]-3-(2-isopropoxyphényl)-3-[2-oxo-2-[4-(4-pyridinyl)-1-pipérazinyl] éthoxy]-1,3-dihydro-2*H*-indol-2-one.

On prépare ce composé selon le mode opératoire décrit à l'étape B de l'Exemple 1 à partir de 0,5 g du composé obtenu à l'étape précédente, 0,04 g d'hydrure de sodium à 60 % dans l'huile, 10 ml de THF et 0,3 g de chlorure de 2,4-diméthoxybenzène sulfonyle. On obtient 0,55 g du produit attendu après cristallisation dans l'éther iso, F = 201-203°C.

EXEMPLE 24

5-Chloro-1-[(2,4-diméthoxyphényl)sulfonyl]-3-(2-méthoxyphényl)-3-[[2-oxo-2-[4-(2-pyridinyl)-1-pipérazinyl]éthyl]amino]-1,3-dihydro-2*H*-indol-2-one.

(I) : $R_1$ = Cl ; $R_2$ = H ; $R_3$ = $OCH_3$ ; $R_4$ = H ; X = $-NH-CH_2-$ ; n = 1 ;

$R_5$ = 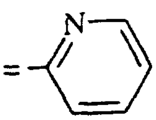 ;

$R_6$ = 2- $OCH_3$ ; $R_7$ = $OCH_3$

A une solution de 0,33 g du composé obtenu à la Préparation 1.28, 0,11 g de 1-(2-pyridinyl)pipérazine et 0,121 g de triéthylamine dans 5 ml de DCM on ajoute 0,329 g de PyBOP et laisse 3 heures sous agitation à TA. On concentre sous vide, extrait le résidu à l'AcOEt, lave la phase organique à l'eau, sèche sur $Na_2SO_4$ et évapore sous vide le solvant. On chromatographie le résidu sur gel de silice en éluant par le mélange DCM/AcOEt (90/10 ; v/v). On obtient 0,29 g du produit attendu après cristallisation dans l'éther iso, F = 155°C.

EXEMPLE 25

5-Chloro-1-[(2,4-diméthoxyphényl)sulfonyl]-3-(2-méthoxyphényl)-3-[[3-oxo-3-[4-(2-pyridinyl)-1-pipérazinyl]propyl] amino]-1,3-dihydro-2*H*-indol-2-one.

(I) : $R_1$ = Cl ; $R_2$ = H ; $R_3$ = $OCH_3$ ; $R_4$ = H ; X = $-NH-CH_2-CH_2-$ ; n = 1 ;

$R_5$ = 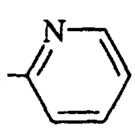 ;

$R_6$ = 2- $OCH_3$ ; $R_7$ = $OCH_3$

A une solution de 0,4 g du composé obtenu à la Préparation 1.29, 0,127 g de 1-(2-pyridinyl)pipérazine et

0,143 g de triéthylamine dans 10 ml de DCM on ajoute 0,37 g de PyBOP et laisse 2 heures sous agitation à TA. On dilue le mélange réactionnel au DCM, lave la phase organique à l'eau, sèche sur $Na_2SO_4$ et évapore sous vide le solvant. On chromatographie le résidu sur gel de silice en éluant par le mélange DCM/AcOEt (90/10 ; v/v). On obtient 0,36 g du produit attendu après cristallisation dans l'éther iso, F = 214°C.

RMN$^1$H : DMSO-$d_6$ : δ (ppm) : 2,0 à 2,5 : 2mt : 4H ; 3,1 à 3,6 : m+s : 11H ; 3,7 : s : 3H ; 3,9 : s : 3H ; 6,6 à 7,0 : m : 6H ; 7,1 : t : 1H ; 7,3 : t : 1H ; 7,5 : dd : 1H ; 7,6 : mt : 1H ; 7,7 à 8,0 : mt : 4H ; 8,2 : dd : 1H.

EXEMPLE 26

N-[5-Chloro-1-[(2,4-diméthoxyphényl)sulfonyl]-3-(2-isopropoxyphényl)-2-oxo-2,3-dihydro-1*H*-indol-3-yl]-4-(3-pyridazinyl)pipérazine-1-carboxamide, énantiomère unique.

(I) : $R_1$ = Cl ; $R_2$ = H ; $R_3$ = $OCH(CH_3)_2$ ; $R_4$ = H ; X = -NH- ; n = 1 ;

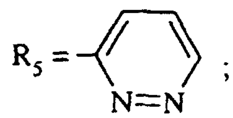

$R_6$ = 2- $OCH_3$ ; $R_7$ = $OCH_3$

A une solution de 0,054 g de 1-(3-pyridazinyl)pipérazine dans 2 ml de chloroforme on ajoute une solution de 0,07 g du composé obtenu à la Préparation 1.20 puis chauffe à 60°C pendant 24 heures. On chromatographie directement le mélange réactionnel sur gel de silice en éluant par le mélange DCM/MeOH (95/5 ; v/v). On obtient le produit attendu. Pureté CLHP : 100 %.

EXEMPLE 27

N-[5-Chloro-1-[(2,4-diméthoxyphényl)sulfonyl]-3-(2-isopropoxyphényl)-2-oxo-2,3-dihydro-1*H*-indol-3-yl]-4-(2-pyrimidinyl)pipérazine-1-carboxamide, énantiomère unique.

(I) : $R_1$ = Cl ; $R_2$ = H ; $R_3$ = $OCH(CH_3)_2$ ; $R_4$ = H ; X = -NH- ; n = 1 ;

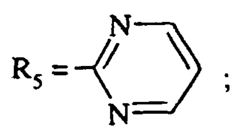

$R_6$ = 2- $OCH_3$ ; $R_7$ = $OCH_3$

On prépare ce composé selon le mode opératoire décrit à l'Exemple 26 à partir de 0,054 g de 1-(2-pyrimidinyl) pipérazine dans 2 ml de chloroforme et de 0,07 g du composé obtenu à la Préparation 1.20. On obtient le produit attendu. Pureté CLHP : 99 %.

En procédant selon les modes opératoires décrits dans les Exemples ci-dessus, à partir des composés de formule (II) dans laquelle W = H et des composés de formule (V), on prépare les composés de formule (IV) rassemblés dans le Tableau I ci-après :

TABLEAU I

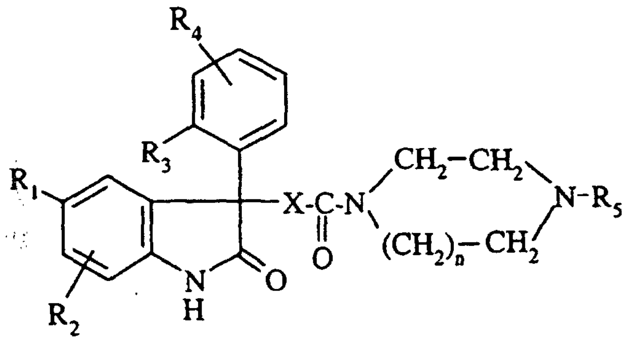

(IV)

| Composés (IV) | R1 | R2 | R3 | R4 | X | n | R5 | F°C, solvant de cristallisatio |
|---|---|---|---|---|---|---|---|---|
| IV. 1 (a) | Cl | H | $OCH_3$ | H | $-CH_2-$ | 1 | 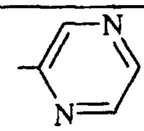 | 87 - |

| | | | | | | | | |
|---|---|---|---|---|---|---|---|---|
| IV. 2 (b) | Cl | 6-Cl | $OCH_3$ | H | $-CH_2-$ | 1 | 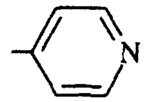 | 286-288 éther iso |
| IV. 3 (c) | Cl | 6-Cl | $OCH_3$ | H | $-CH_2-$ | 1 | 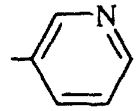 | 182-189 éther iso |
| IV. 4 (d) | Cl | $6-CH_3$ | $OCH_3$ | H | $-CH_2-$ | 1 | 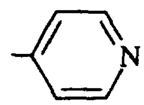 | 102-110 - |
| IV. 5 (e) | Cl | $6-CH_3$ | $OCH_3$ | H | $-CH_2-$ | 1 | 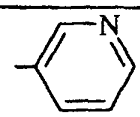 | 248-250 AcOEt |
| IV. 6 (f) | Cl | $6-CH_3$ | $OCH(CH_3)_2$ | H | $-CH_2-$ | 1 | 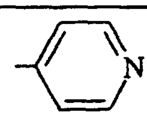 | - - |
| IV. 7 (g) | Cl | H | F | H | $-CH_2-$ | 1 | 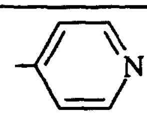 | - - |
| IV. 8 (h) | Cl | 6-Cl | F | H | $-CH_2-$ | 1 | 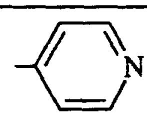 | 274-276 DCM |
| IV. 9 (i) | Cl | H | $OCH_3$ | $3-OCH_3$ | $-CH_2-$ | 1 | 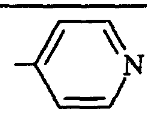 | - DCM |
| IV. 10 (j) | Cl | H | $OCH_2CH_3$ | H | $-CH_2-$ | 1 | 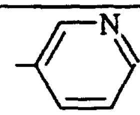 | - - |
| IV. 11 (k) | Cl | $6-CH_3$ | $OCH_2CH_3$ | H | $-CH_2-$ | 1 | 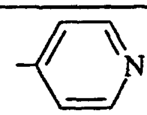 | - - |
| IV. 12 (l) | Cl | H | $OCH(CH_3)_2$ | H | $-CH_2-$ | 1 | 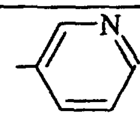 | - - |
| IV. 13 (m) | Cl | H | $OCH(CH_3)_2$ | H | $-CH_2-$ | 2 | 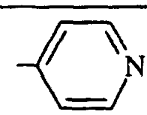 | - - |
| IV. 14 (n) | Cl | H | $OCH(CH_3)_2$ | H | $-CH_2-$ | 1 | 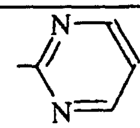 | - - |

| | | | | | | | | |
|---|---|---|---|---|---|---|---|---|
| IV. 15 (o) | Cl | H | $OCH(CH_3)_2$ | H | $-CH_2-$ | 1 | 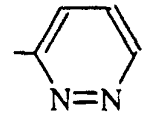 | 233 éther iso |
| IV. 16 (p) | Cl | H | $OCH(CH_3)_2$ | H | $-CH_2-$ | 1 | 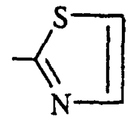 | 157 éther |
| IV. 17 (q) | Cl | $6-CH_3$ | $OCH(CH_3)_2$ | H | $-CH_2-$ | 1 | 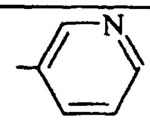 | - - |
| IV. 18 (r) | Cl | H | F | H | $-CH_2-$ | 1 | 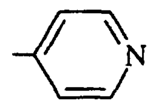 | - - $\alpha_D^{20}$ = +47° (c = 0,15 ; chloroforme) |
| IV. 19 (s) | Cl | H | $OCH(CH_3)_2$ | H | $-CH_2-$ | 1 | 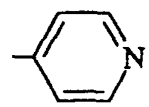 | 192-196 éther iso |

(a) Composé préparé selon le mode opératoire décrit à l'étape A de l'Exemple 1 à partir du composé de la Préparation 1.1 et du composé de la Préparation 2.1.

(b) Composé préparé selon le mode opératoire décrit à l'étape A de l'Exemple 1 à partir du composé de la Préparation 1.5 et de 1-(4-pyridinyl)pipérazine.

(c) Composé préparé selon le mode opératoire décrit à l'étape A de l'Exemple 1 à partir du composé de la Préparation 1.5 et du composé de la Préparation 2.2.

(d) Composé préparé selon le mode opératoire décrit à l'étape A de l'Exemple 3 à partir du composé de la Préparation 1.6 et de 1-(4-pyridinyl)pipérazine.

(e) Composé préparé selon le mode opératoire décrit à l'étape A de l'Exemple 3 à partir du composé de la Préparation 1.6 et du composé de la Préparation 2.2.

(f) Composé préparé selon le mode opératoire décrit à l'étape A de l'Exemple 3 à partir du composé de la Préparation 1.7 et de 1-(4-pyridinyl)pipérazine.

(g) Composé préparé selon le mode opératoire décrit à l'étape A de l'Exemple 1 à partir du composé obtenu à l'étape B de la Préparation 1.30 et de 1-(4-pyridinyl)pipérazine.

(h) Composé préparé selon le mode opératoire décrit à l'étape A de l'Exemple 1 à partir du composé de la Préparation 1.31 et de 1-(4-pyridinyl)pipérazine.

(i) Composé préparé selon le mode opératoire décrit à l'étape A de l'Exemple 1 à partir du composé de la Préparation 1.32 et de 1-(4-pyridinyl)pipérazine.

(j) Composé préparé selon le mode opératoire décrit à l'étape A de l'Exemple 1 à partir des composés des Préparations 1.2 et 2.2.

(k) Composé préparé selon le mode opératoire décrit à l'étape A de l'Exemple 1 à partir du composé de la Préparation 1.33 et de 1-(4-pyridinyl)pipérazine.

(l) Composé préparé selon le mode opératoire décrit à l'étape A de l'Exemple 1 à partir du composé de l'étape E de la Préparation 1.3 et du composé de la Préparation 2.2.

(m) Composé préparé selon le mode opératoire décrit à l'étape A de l'Exemple 1 à partir du composé de l'étape E de la Préparation 1.3 et du composé de la Préparation 2.4.

(n) Composé préparé selon le mode opératoire décrit à l'étape A de l'Exemple 1 à partir du composé de l'étape E de la Préparation 1.3 et de 1-(2-pyrimidinyl)pipérazine.

(o) Composé préparé selon le mode opératoire décrit à l'étape A de l'Exemple 1 à partir du composé de l'étape E de la Préparation 1.3 et de 1-(3-pyridazinyl)pipérazine.

(p) Composé préparé selon le mode opératoire décrit à l'étape A de l'Exemple 1 à partir du composé de l'étape E de la Préparation 1.3 et de 1-(1,3-thiazol-2-yl)pipérazine.

(q) Composé préparé selon le mode opératoire décrit à l'étape A de l'Exemple 1 à partir des composés des Préparations 1.7 et 2.2.

(r) Composé préparé selon le mode opératoire décrit à l'étape A de l'Exemple 1 à partir du composé de la Préparation 1.30 et de 1-(4-pyridinyl)pipérazine.

(s) Composé préparé selon le mode opératoire décrit à l'étape A de l'Exemple 1 à partir du composé de l'étape E de la Préparation 1.3 et de 1-(4-pyridinyl)pipérazine.

En procédant selon les modes opératoires décrits dans les Exemples ci-dessus, à partir des composés de formule (II) dans laquelle

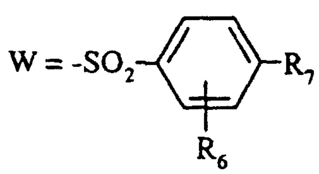

et des composés de formule (III), ou à partir des composés de formule (IV) et du chlorure de 2,4-diméthoxybenzènesulfonyle, on prépare les composés selon l'invention rassemblés dans le Tableau II) ci-après :

TABLEAU II

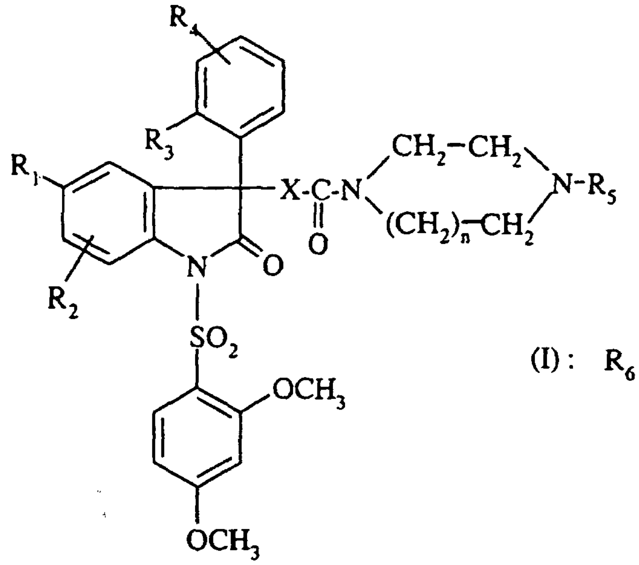

(I) : $R_6$ = 2- $OCH_3$ ; $R_7$ = $OCH_3$

| Exemples | $R_1$ | $R_2$ | $R_3$ | $R_4$ | X | n | $R_5$ | F°C ; Sel solvant de cristallisation $\alpha_D^{20}$ (chloroforme) |
|---|---|---|---|---|---|---|---|---|
| 1 | Cl | H | $OCH_3$ | H | $-CH_2-$ | 1 | 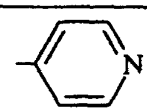 | 136-141 (déc.) éther iso - |
| 2 | Cl | H | $OCH_2CH_3$ | H | $-CH_2-$ | 1 | 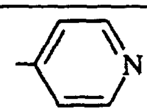 | 225 éther iso - |
| 3 | Cl | H | $OCH(CH_3)_2$ | H | $-CH_2-$ | 1 | 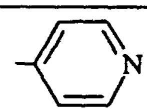 | - - -21,3° (c = 0,1 ; DCM) |
| 4 | $CH_3$ | H | Cl | H | $-CH_2-$ | 1 | 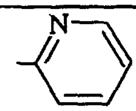 | 239 éther iso - |
| 5 | Cl | H | $OCH_3$ | H | -O- | 1 | 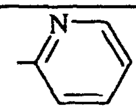 | 210-215 éther iso - |

| | | | | | | | | |
|---|---|---|---|---|---|---|---|---|
| 6 | Cl | H | $OCH_3$ | H | -O- | 1 | 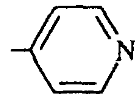 | 194-198 éther iso - |
| 7 | Cl | H | $OCH(CH_3)_2$ | H | -O- | 1 | 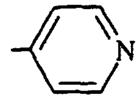 | 253 AcOEt - |
| 8 | Cl | H | $OCH(CH_3)_2$ | H | -O- | 1 | 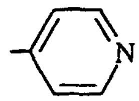 | 235 ; 1,5 fumarate ; acétonitrile - |
| 9 | Cl | H | $OCH(CH_3)_2$ | H | -O- | 1 | 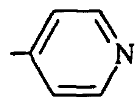 | 160-164 DCM/hexane/ éther iso -71,8°(c = 0,18) |
| 10 | Cl | H | $OCH(CH_3)_2$ | H | -O- | 1 | 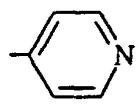 | 161-167 DCM/hexane/ éther iso +72,5°(c= 0,14) |
| 11 | Cl | H | $OCH_3$ | 5-$OCH_3$ | -O- | 1 | 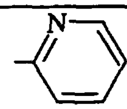 | 254 MeOH - |
| 12 | Cl | H | $OCH_3$ | 5-$OCH_3$ | -O- | 1 | 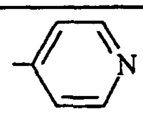 | 247-249 éther iso - |
| 13 | Cl | H | $OCH_3$ | H | -NH- | 1 | 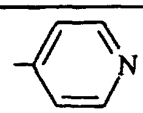 | - DCM/hexane/ éther iso - |
| 14 | Cl | H | $OCH_3$ | H | -NH- | 1 | 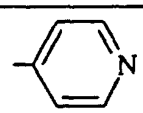 | - MeOH/éther iso -43°(c = 0,16) |
| 15 | Cl | H | $OCH_3$ | H | -NH- | 2 | 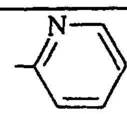 | 152 DCM/éther iso - |

| | | | | | | | | |
|---|---|---|---|---|---|---|---|---|
| 16 | Cl | H | $OCH_3$ | H | -NH- | 2 | 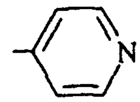 | 168<br>DCM/éther iso<br>- |
| 17 | Cl | H | $OCH(CH_3)_2$ | H | -NH- | 1 | 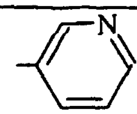 | 148<br>DCM/éther iso<br>+25,6°(c= 0,15) |
| 18 | Cl | H | $OCH(CH_3)_2$ | H | -NH- | 1 | 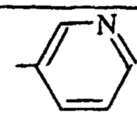 | -<br>DCM/éther iso<br>-33°(c = 0,15) |
| 19 | Cl | H | $OCH(CH_3)_2$ | H | -NH- | 1 | 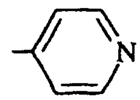 | -<br>DCM/hexane/<br>éther iso<br>+7,9°(c= 0,175) |
| 20 | Cl | H | $OCH(CH_3)_2$ | H | -NH- | 1 | 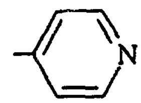 | -<br>DCM/hexane/<br>éther iso<br>-10,3°(c= 0,17) |
| 21 | Cl | H | $OCH_3$ | 5-$OCH_3$ | -NH- | 1 | 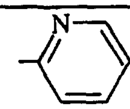 | -<br>DCM/hexane/<br>éther iso<br>- |
| 22 | Cl | 6-$CF_3$ | $OCH_3$ | H | -NH- | 1 | 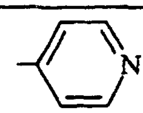 | 174<br>DCM/éther iso<br>- |
| 23 | Cl | H | $OCH(CH_3)_2$ | H | -O-<br>$CH_2$- | 1 | 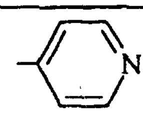 | 201-203<br>éther iso<br>- |
| 24 | Cl | H | $OCH_3$ | H | -NH-<br>$CH_2$- | 1 | 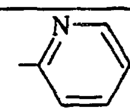 | 155<br>éther iso<br>- |
| 25 | Cl | H | $OCH_3$ | H | -NH-<br>$CH_2$-<br>$CH_2$- | 1 | 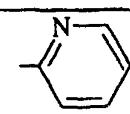 | 214<br>éther iso<br>- |
| 26 | Cl | H | $OCH(CH_3)_2$ | H | -NH- | 1 | 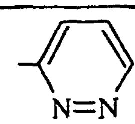 | -<br>-<br>- |

| | | | | | | | | |
|---|---|---|---|---|---|---|---|---|
| 27 | Cl | H | $OCH(CH_3)_2$ | H | -NH- | 1 | 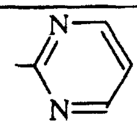 | -<br>-<br>- |
| 28<br>(a) | Cl | H | $OCH_3$ | H | $-CH_2-$ | 1 | 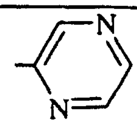 | 209-212<br>éther iso<br>- |
| 29<br>(b) | Cl | 6-Cl | $OCH_3$ | H | $-CH_2-$ | 1 | 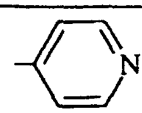 | 247-248<br>éther iso<br>- |
| 30<br>(c) | Cl | 6-Cl | $OCH_3$ | H | $-CH_2-$ | 1 | 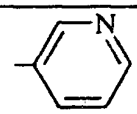 | 219-222<br>éther iso<br>- |
| 31<br>(d) | Cl | $6-CH_3$ | $OCH_3$ | H | $-CH_2-$ | 1 | 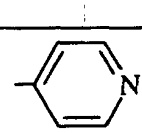 | 232-234<br>éther iso<br>- |
| 32<br>(e) | Cl | $6-CH_3$ | $OCH_3$ | H | $-CH_2-$ | 1 | 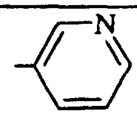 | 215-217<br>éther iso<br>- |
| 33<br>(f) | Cl | $6-CH_3$ | $OCH(CH_3)_2$ | H | $-CH_2-$ | 1 | 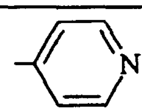 | 261-262<br>éther iso<br>- |
| 34<br>(g) | Cl | H | Cl | H | -O- | 1 | 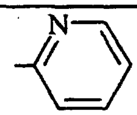 | 225-227<br>éther iso<br>- |
| 35<br>(h) | Cl | H | Cl | H | -O- | 1 | 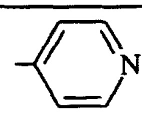 | 135-140<br>éther iso<br>- |
| 36<br>(i) | Cl | H | F | H | -O- | 1 | 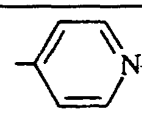 | 155<br>éther iso<br>- |
| 37<br>(j) | Cl | H | $CF_3$ | H | -O- | 1 | 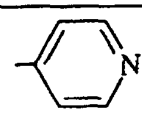 | 192-194<br>éther iso<br>- |

| | | | | | | | | |
|---|---|---|---|---|---|---|---|---|
| 38 (k) | Cl | H | $OCF_3$ | H | -O- | 1 | 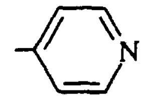 | 236<br>éther iso<br>- |
| 39 (l) | Cl | 6-$CH_3$ | $OCH_3$ | H | -O- | 1 | 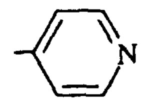 | 230 (déc)<br>éther iso<br>- |
| 40 (m) | Cl | 6-$OCH_3$ | Cl | H | -O- | 1 | 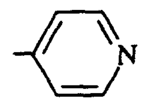 | 160-170<br>éther iso<br>- |
| 41 (n) | Cl | H | $OCH_3$ | H | -NH- | 1 | 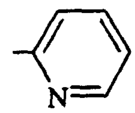 | 245<br>DCM/éther iso<br>- |
| 42 (o) | Cl | H | Cl | H | -NH- | 1 | 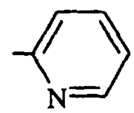 | -<br>DCM/éther iso<br>- |
| 43 (p) | Cl | H | Cl | H | -NH- | 1 | 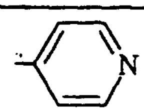 | 234<br>DCM/éther iso<br>- |
| 44 (q) | Cl | H | Cl | H | -NH- | 1 | 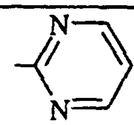 | 278<br>DCM/éther iso<br>- |
| 45 (r) | Cl | 6-$CH_3$ | $OCH_3$ | H | -NH- | 1 | 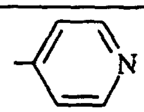 | 186<br>DCM/éther iso<br>- |
| 46 (s) | $CH_3$ | 6-Cl | Cl | H | -NH- | 1 | 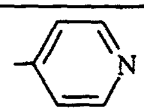 | 225 ; RMN<br>DCM/éther iso<br>- |
| 47 (t) | Cl | H | $OCH_3$ | H | -NH-$CH_2$-$CH_2$- | 1 | 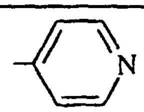 | >250<br>éther<br>- |

| | | | | | | | | |
|---|---|---|---|---|---|---|---|---|
| 48 (u) | Cl | H | F | H | $-CH_2-$ | 1 | 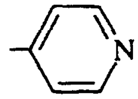 | 129-135 éther iso - |
| 49 (v) | Cl | 6-Cl | F | H | $-CH_2-$ | 1 | 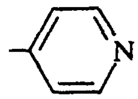 | 142-148 (déc) éther iso - |
| 50 (w) | Cl | H | $OCH_3$ | $3-OCH_3$ | $-CH_2-$ | 1 | 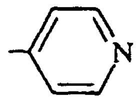 | 175 - - |
| 51 (x) | Cl | H | $OCH_2CH_3$ | H | $-CH_2-$ | 1 | 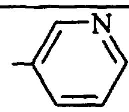 | 226 éther iso - |
| 52 (y) | Cl | $6-CH_3$ | $OCH_2CH_3$ | H | $-CH_2-$ | 1 | 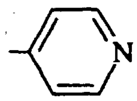 | 243 éther iso/AcOEt - |
| 53 (z) | Cl | H | $OCH(CH_3)_2$ | H | $-CH_2-$ | 1 | 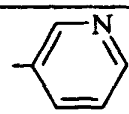 | 217 éther iso - |
| 54 (aa) | Cl | H | $OCH(CH_3)_2$ | H | $-CH_2-$ | 2 | 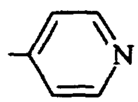 | 157 éther iso - |
| 55 (ab) | Cl | H | $OCH(CH_3)_2$ | H | $-CH_2-$ | 1 | 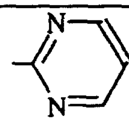 | 157-159 éther iso - |
| 56 (ac) | Cl | H | $OCH(CH_3)_2$ | H | $-CH_2-$ | 1 | 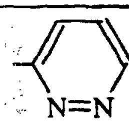 | 230 éther iso/AcOEt - |
| 57 (ad) | Cl | H | $OCH(CH_3)_2$ | H | $-CH_2-$ | 1 | 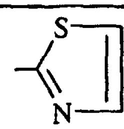 | 235 éther iso - |
| 58 (ae) | Cl | $6-CH_3$ | $OCH(CH_3)_2$ | H | $-CH_2-$ | 1 | 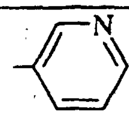 | 222 éther iso/AcOEt - |

| | | | | | | | | |
|---|---|---|---|---|---|---|---|---|
| 59 (af) | Cl | H | $OCH_2CH_3$ | H | -O- | 1 | 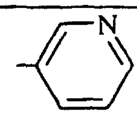 | 255-260 éther iso - |
| 60 (ag) | Cl | H | $OCH_2CH_3$ | H | -O- | 1 | 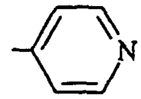 | 255-260 AcOEt - |
| 61 (ah) | Cl | H | F | H | $-CH_2-$ | 1 | 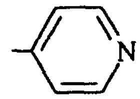 | - - -79° (c=0,13) |
| 62 (ai) | Cl | H | $OCH(CH_3)_2$ | H | $-CH_2-$ | 1 | 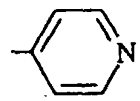 | 223 (déc) éther iso - |
| 63 (aj) | Cl | H | $OCH(CH_3)_2$ | H | $-CH_2-$ | 1 | 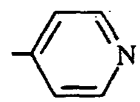 | 184 ; HCl propan-2-ol -13,5°(c = 0,14 ; MeOH) |
| 64 (ak) | Cl | H | $OCH(CH_3)_2$ | H | $-CH_2-$ | 1 | 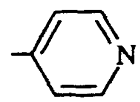 | 202 ; fumarate acétonitrile -11,7°(c = 0,12 ; MeOH) |
| 65 (al) | Cl | H | $OCH(CH_3)_2$ | H | $-CH_2-$ | 1 | 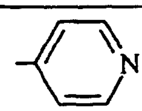 | 248 ; $H_3PO_4$ EtOH -7,7°(c = 0,13 ; MeOH) |

(a) Composé préparé selon le mode opératoire décrit à l'étape B de l'Exemple 1 à partir du composé IV.1.

(b) Composé préparé selon le mode opératoire décrit à l'étape B de l'Exemple 1 à partir du composé IV.2.

(c) Composé préparé selon le mode opératoire décrit à l'étape B de l'Exemple 1 à partir du composé IV.3.

(d) Composé préparé selon le mode opératoire décrit à l'étape B de l'Exemple 1 à partir du composé IV.4.

(e) Composé préparé selon le mode opératoire décrit à l'étape B de l'Exemple 1 à partir du composé IV.5.

(f) Composé préparé selon le mode opératoire décrit à l'étape B de l'Exemple 1 à partir du composé IV.6.

(g) Composé préparé selon le mode opératoire décrit à l'Exemple 5 à partir du composé de la Préparation 1.11 et de 1-(2-pyridinyl)pipérazine.

(h) Composé préparé selon le mode opératoire décrit à l'Exemple 5 à partir du composé de la Préparation 1.11 et de 1-(4-pyridinyl)pipérazine.

(i) Composé préparé selon le mode opératoire décrit à l'Exemple 6 à partir du composé de la Préparation 1.12 et de 1-(4-pyridinyl)pipérazine.

(j) Composé préparé selon le mode opératoire décrit à l'Exemple 6 à partir du composé de la Préparation 1.13 et de 1-(4-pyridinyl)pipérazine.

(k) Composé préparé selon le mode opératoire décrit à l'Exemple 7 à partir du composé de la Préparation 1.14 et de 1-(4-pyridinyl)pipérazine.

(l) Composé préparé selon le mode opératoire décrit à l'Exemple 5 à partir du composé de la Préparation 1.15 et de 1-(4-pyridinyl)pipérazine.

(m) Composé préparé selon le mode opératoire décrit à l'Exemple 5 à partir du composé de la Préparation 1.16 et de 1-(4-pyridinyl)pipérazine.

(n) Composé préparé selon le mode opératoire décrit à l'Exemple 13 à partir du composé de la Préparation 1.18 et de 1-(2-pyridinyl)pipérazine.

(o) Composé préparé selon le mode opératoire décrit à l'Exemple 13 à partir du composé de la Préparation 1.23 et de 1-(2-pyridinyl)pipérazine.

(p) Composé préparé selon le mode opératoire décrit à l'Exemple 13 à partir du composé de la Préparation 1.23 et de 1-(4-pyridinyl)pipérazine.

(q) Composé préparé selon le mode opératoire décrit à l'Exemple 13 à partir du composé de la Préparation 1.23 et de 1-(2-pyrimidinyle)pipérazine.

(r) Composé préparé selon le mode opératoire décrit à l'Exemple 15 à partir du composé de la Préparation 1.24 et de 1-(4-pyridinyl)pipérazine.

(s) Composé préparé selon le mode opératoire décrit à l'Exemple 13 à partir du composé de la Préparation 1.26 et de 1-(4-pyridinyl)pipérazine.

(t) Composé préparé selon le mode opératoire décrit à l'Exemple 25 à partir du composé de la Préparation 1.29 et de 1-(4-pyridinyl)pipérazine.

(u) Composé préparé selon le mode opératoire décrit à l'étape B de l'Exemple 1 à partir du composé IV. 7.

(v) Composé préparé selon le mode opératoire décrit à l'étape B de l'Exemple 1 à partir du composé IV. 8.

(w) Composé préparé selon le mode opératoire décrit à l'étape B de l'Exemple 1 à partir du composé IV. 9.

(x) Composé préparé selon le mode opératoire décrit à l'étape B de l'Exemple 1 à partir du composé IV. 10.

(y) Composé préparé selon le mode opératoire décrit à l'étape B de l'Exemple 1 à partir du composé IV. 11.

(z) Composé préparé selon le mode opératoire décrit à l'étape B de l'Exemple 1 à partir du composé IV. 12.

(aa) Composé préparé selon le mode opératoire décrit à l'étape B de l'Exemple 1 à partir du composé IV. 13.

(ab) Composé préparé selon le mode opératoire décrit à l'étape B de l'Exemple 1 à partir du composé IV. 14.

(ac) Composé préparé selon le mode opératoire décrit à l'étape B de l'Exemple 1 à partir du composé IV. 15.

(ad) Composé préparé selon le mode opératoire décrit à l'étape B de l'Exemple 1 à partir du composé IV. 16.

(ae) Composé préparé selon le mode opératoire décrit à l'étape B de l'Exemple 1 à partir du composé IV. 17.

(af) On laisse 4 jours sous agitation à TA un mélange de 0,3 g du composé obtenu à la Préparation 1.34, 0,3 g du composé de la Préparation 2.2 et 2 g de DIPEA dans 15 ml de DMF. On concentre sous vide, reprend le résidu à l'eau, extrait à l'AcOEt, lave la phase organique à l'eau, sèche sur $Na_2SO_4$ et évapore le solvant sous vide. On chromatographie le résidu sur gel de silice en éluant au DCM puis à l'acétone. On obtient 0,27 g du produit attendu après trituration dans l'éther iso.

(ag) On laisse 5 jours sous agitation à TA un mélange de 0,4 g du composé obtenu à la Préparation 1.34, 0,6 g de 1-(4-pyridinyl)pipérazine dans 0,6 ml de THF. On concentre sous vide, reprend le résidu dans 20 ml d'eau et 20 ml d'AcOEt, laisse sous agitation, essore le précipité formé et le lave à l'éther iso. On obtient 0,4 g du produit attendu.

(ah) Composé préparé selon le mode opératoire décrit à l'étape B de l'Exemple 1 à partir du composé IV. 18.

(ai) Composé préparé selon le mode opératoire décrit à l'étape B de l'Exemple 1 à partir du composé IV. 19.

(aj) A une solution de 0,2 g du composé de l'Exemple 3 dans 20 ml d'EtOH, on ajoute une solution d'HCl 2N dans l'éther et concentre sous vide. On reprend le résidu dans le propan-2-ol, essore le précipité formé, le lave à l'éther et le sèche. On obtient 0,12 g du chlorhydrate.

(ak) On chauffe à reflux pendant 10 minutes un mélange de 0,3 g du composé de l'Exemple 3 et 0,059 g d'acide fumarique dans 20 ml d'acétonitrile. Après refroidissement, on essore le précipité formé et obtient 0,24 g du fumarate.

(al) On chauffe à 60°C pendant 10 minutes un mélange de 0,3 g du composé de l'Exemple 3 et 0,051 g d'$H_3PO_4$ à 85 % dans 20 ml d'EtOH. Après refroidissement, on essore le précipité formé et obtient 0,3 g du produit attendu.

EXEMPLE 46 : RMN$^1$H : DMSO-$d_6$ : δ (ppm) : 2,3 : s : 3H ; 3,2 à 3,8 : m+s : 1H ; 3,9 : s : 3H ; 6,7 : mt : 2H ; 6,9 : d : 2H ; 7,2 à 7,4 : m : 5H ; 7,8 : s : 1H ; 7,9 : d : 1H ; 8,0 : s : 1H ; 8,2 : d : 2H.

Les composés selon l'invention ont fait l'objet d'études biochimiques.

L'affinité des composés de formule (I) selon l'invention pour les récepteurs $V_{1b}$ de l'arginine-vasopressine a été déterminée *in vitro* en utilisant la méthode décrite par Y. DE KEYSER et al., Febs Letters, 1994, 356, 215-220. Cette méthode consiste à étudier *in vitro* le déplacement de l'arginine-vasopressine tritiée ([$^3$H]-AVP) aux récepteurs $V_{1b}$ présents sur des préparations membranaires adénohypophysaires ou cellulaires portant les récepteurs $V_{1b}$ de rat ou humains. Les concentrations inhibitrices de 50 % ($CI_{50}$) de la fixation de l'arginine-vasopressine tritiée des composés selon l'invention sont faibles et varient de $10^{-6}$ à $10^{-9}$M.

L'affinité des composés de formule (I) selon l'invention pour les récepteurs $V_{1a}$ de l'arginine-vasopressine a été déterminée *in vitro* en utilisant la méthode décrite par M. THIBONNIER et al., J. Biol. Chem., 1994, 269, 3304-3310. Cette méthode consiste à étudier *in vitro* le déplacement de l'arginine-vasopressine tritiée ([$^3$H]-AVP) aux récepteurs $V_{1a}$ présents sur des préparations membranaires ou cellulaires portant les récepteurs $V_{1a}$ de rat ou humains. Les composés de formule (I) présentent une affinité pour les récepteurs $V_{1a}$ de l'arginine-vasopressine avec des $CI_{50}$ qui varient de $10^{-6}$ à $10^{-9}$M.

L'affinité des composés de formule (I) selon l'invention pour les récepteurs $V_2$ de la vasopressine a également été étudiée (méthode décrite par M. Bimbaumer et al., Nature (Lond.), 1992, 357, 333-335). Les composés étudiés sont peu ou pas affins pour les récepteurs $V_2$ avec des $CI_{50}$ généralement supérieures à $10^{-6}$M.

L'affinité des composés selon l'invention pour les récepteurs de l'ocytocine a été déterminée dans un test de liaison *in vitro* en utilisant la méthode décrite par J. Elands et al. dans Eur. J. Pharmacol., 1987, 147, 197-207. Cette méthode consiste à étudier *in vitro* le déplacement d'un analogue radioiodé de l'ocytocine aux récepteurs de l'ocytocine dans une préparation membranaire de cellules transfectée avec le récepteur ocytocique humain utérin. Les $IC_{50}$ (concentration inhibitrice de 50 % de la liaison de l'analogue radioiodé de l'ocytocine à ses récepteurs) sont faibles et varient de $10^{-6}$ à $10^{-9}$M dans ce dernier test.

Les composés de la présente invention sont notamment des principes actifs de compositions pharmaceutiques, dont la toxicité est compatible avec leur utilisation en tant que médicaments.

Selon un autre de ses aspects, la présente invention concerne l'utilisation des composés de formule (I), ou l'un de leurs sels, solvats et/ou hydrates pharmaceutiquement acceptables, pour la préparation de médicaments destinés à traiter toute pathologie où l'arginine-vasopressine et/ou ses récepteurs $V_{1b}$ et/ou ses récepteurs $V_{1a}$ et/ou l'ocytocine et/ou ses récepteurs sont impliqués.

Ainsi les composés selon l'invention peuvent être utilisés, chez l'homme ou chez l'animal, dans le traitement ou la prévention de différentes affections vasopressine-dépendantes tels que les affections cardiovasculaires, comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase ; les affections du système nerveux central comme la migraine, le vasospasme cérébral, l'hémorragie cérébrale, les oedèmes cérébraux, la dépression, l'anxiété, le stress, les troubles émotionnels, le trouble obsessionnel-compulsif, les attaques de panique, les états psychotiques, les troubles de la mémoire par exemple ; les affections du système rénal comme le vasospasme rénal, la nécrose du cortex rénal ; le diabète insipide néphrogénique ; les affections du système gastrique comme le vasospasme gastrique, l'hépatocirrhose, les ulcères, la pathologie des vomissements, par exemple la nausée y compris la nausée due à une chimiothérapie, le mal des transports ; la néphropathie diabétique. Les composés selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules ; des encéphalopathies hyponatrémiques ; du syndrome pulmonaire, de la maladie de Ménière ; du glaucome, de la cataracte ; de l'obésité ; du diabète de type I et II ; de l'athérosclérose ; du syndrome de Cushing ; de la résistance à l'insuline ; de

l'hypertriglycéridémie ; dans les traitements postopératoires, notamment après une chirurgie abdominale.

Les composés selon l'invention peuvent aussi être utilisés dans le traitement ou la prévention de toutes les pathologies consécutives au stress comme la fatigue et ses syndromes, les désordres ACTH dépendants, les troubles cardiaques, la douleur, les modifications de la vidange gastrique, de l'excrétion fécale (colite, syndrome du colon irritable, maladie de Crohn), de la sécrétion acide, l'hyperglycémie, l'immunosuppression, les processus inflammatoires (arthrite rhumatoïde et ostéoarthrite), les infections multiples, les cancers, l'asthme, le psoriasis, les allergies et les désordres neuropsychiatriques variés tel que l'anorexie nerveuse, la boulimie, les troubles de l'humeur, la dépression, l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance, le sevrage, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysosurrénalien.

Les composés selon l'invention peuvent également être utilisés comme psychostimulants, provoquant l'augmentation de l'éveil, la réactivité émotionnelle face à l'environnement et facilitant l'adaptation.

Les composés selon l'invention ayant une affinité pour les récepteurs de l'ocytocine sont particulièrement intéressants et ce, dans la prévention et/ou le traitement des désordres ocytocine-dépendants. Les composés selon la présente invention sont intéressants dans la cicatrisation, dans l'analgésie, dans l'anxiolyse, dans la prévention de la douleur, dans la prévention de l'anxiété, la dépression, la schizophrénie, l'autisme, les syndromes obsessionnels compulsifs, dans le comportement maternel (facilitation de la reconnaissance et de l'acceptation de la mère par l'enfant) et social, la mémoire ; la régulation de la prise de nourriture et de boisson, la dépendance aux drogues, le sevrage et la motivation sexuelle ; ils peuvent également être utilisés avantageusement dans les désordres de la sphère urogénitale notamment dans les domaines obstétrique et gynécologique, notamment comme agent tocolytique ou relaxant utérin ou pour contrôler les contractions de l'utérus avant que la grossesse soit arrivée à terme, pour contrôler le travail prénatal, ou encore contrôler le travail préparatoire en vue d'un accouchement par césarienne, pour résoudre les problèmes de stérilité, fertilité, contrôler les naissances (usage vétérinaire en particulier), contrôler l'oestrus, l'arrêt de l'allaitement, le sevrage, le transfert et l'implantation d'embryon lors de la fécondation in vitro ; traiter l'endométriose, les dysmenorrhées, ainsi que l'incontinence urinaire à l'effort ou d'urgence, l'hypertrophie bégnine de la prostate et les dysfonctions érectiles, l'hypertension, l'hyponatrémie, l'insuffisance cardiaque, l'arthérosclérose, l'angiogénèse, la prolifération de tumeurs, le sarcome de Kaposi et réguler le stockage des graisses par l'adipocyte.

Par ailleurs, étant donné le rôle de l'ocytocine sur le contrôle de l'hormone lutéïnisante (J.J. Evans, J. Endocrin. 1996, *151*, 169-174) les composés de l'invention pourront être utilisés pour induire la contraception.

De plus, les composés selon l'invention pourront être utilisés pour leurs effets antitumoraux, dans les tumeurs ocytocine sécrétantes, en particulier les cancers mammaires et prostatiques.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels, solvats et/ou hydrates pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (I), ou l'un de ses sels, solvats et/ou hydrates pharmaceutiquement acceptables, ainsi que un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Dans chaque unité de dosage le principe actif de formule (I) est présent dans les quantités adaptées aux doses journalières envisagées. En général chaque unité de dosage est convenablement ajustée selon le dosage et

le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention ou un de ses sels pharmaceutiquement acceptables.

## Revendications

1. Un composé de formule :

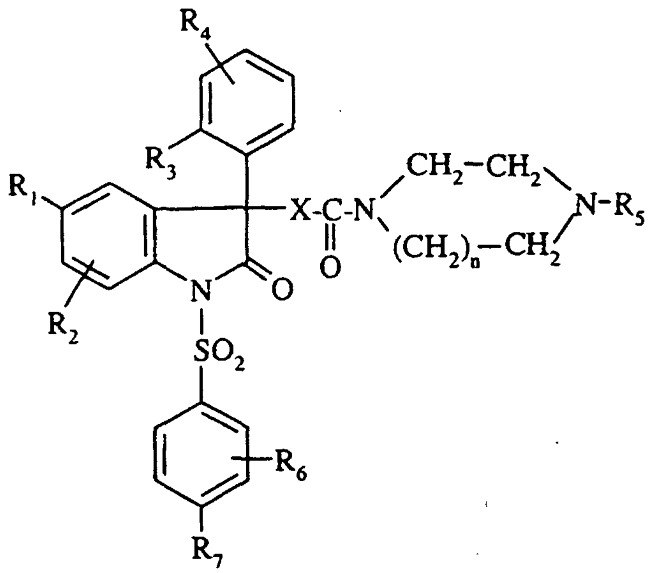

(I)

dans laquelle :

- n est 1 ou 2 ;
- X représente un groupe $-CH_2-$ ; -O- ; -NH- ; $-O-CH_2-$ ; $-NH-CH_2-$ ; $-NH-CH_2-CH_2-$ ;
- $R_1$ représente un atome d'halogène ; un $(C_1-C_4)$alkyle ; un $(C_1-C_4)$alcoxy ;
- $R_2$ représente un atome d'hydrogène ; un atome d'halogène ; un $(C_1-C_4)$alkyle ; un $(C_1-C_4)$alcoxy ; un radical trifluorométhyle ;
- $R_3$ représente un atome d'halogène ; un $(C_1-C_3)$alkyle ; un $(C_1-C_3)$alcoxy ; un radical trifluorométhyle ; un radical trifluorométhoxy ;
- $R_4$ représente un atome d'hydrogène ; un atome d'halogène ; un $(C_1-C_3)$alkyle ; un $(C_1-C_3)$alcoxy ;
- $R_5$ représente un radical choisi parmi :

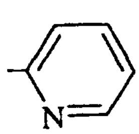 ; 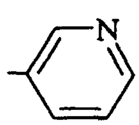 ; 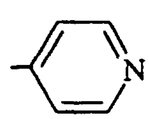 ; 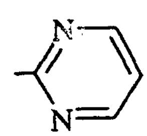 ; 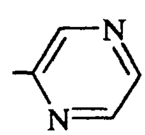 ; 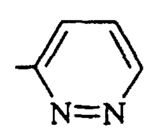 ;

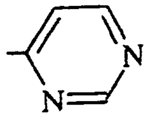 ; 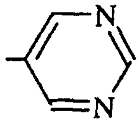 ; 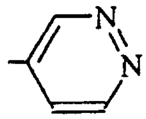 ; 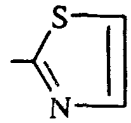 ; 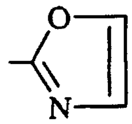 ; 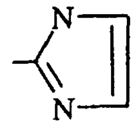

- $R_6$ représente un ($C_1$-$C_4$)alcoxy ;
- $R_7$ représente un ($C_1$-$C_4$)alcoxy ;

ainsi que ses sels avec des acides minéraux ou organiques, ses solvats et/ou ses hydrates.

**2.** Composé selon la revendication 1 dans lequel :

- n et X sont tels que définis pour un composé de formule (I) dans la revendication 1 ;
- $R_1$ représente un atome de chlore ou un radical méthyle ;
- $R_2$ représente un atome d'hydrogène ou est en position -6- de l'indol-2-one et représente un atome de chlore, un radical méthyle, un radical méthoxy ou un radical trifluorométhyle ;
- $R_3$ représente un atome de chlore, un atome de fluor, un radical méthoxy, un radical éthoxy, un radical isopropoxy, un radical trifluorométhyle ou un radical trifluorométhoxy ;
- $R_4$ représente un atome d'hydrogène ou un radical méthoxy ;
- $R_5$ représente un 2-pyridinyle, un 3-pyridinyle, un 4-pyridinyle, un 2-pyrimidinyle, un 2-pyrazinyle, un 3-pyridazinyle, un 1,3-thiazol-2-yle ;
- $R_6$ est en position -2- du phényle et représente un radical méthoxy ;
- $R_7$ représente un radical méthoxy ;

ainsi que ses sels avec des acides minéraux ou organiques, ses solvats et/ou hydrates.

**3.** Un composé choisi parmi :

- 5-Chloro-3-(2-éthoxyphényl)-1-[(2,4-diméthoxyphényl)sulfonyl]-3-[2-oxo-2-[4-(4-pyridinyl)-1-pipérazinyl] éthyl]-1,3-dihydro-2*H*-indol-2-one ;
- 5-Chloro-3-(2-isopropoxyphényl)-1-[(2,4-diméthoxyphényl)sulfonyl]-3-[2-oxo-2-[4-(4-pyridinyl)-1-pipérazinyl] éthyl]-1,3-dihydro-2*H*-indol-2-one ;
- 4-(2-Pyridinyl)-1-pipérazinecarboxylate de 5-chloro-1-[(2,4-diméthoxyphényl) sulfonyl]-3-(2-méthoxyphényl)-2-oxo-2,3-dihydro-1*H*-indol-3-yle ;
- 4-(4-Pyridinyl)-1-pipérazinecarboxylate de 5-chloro-1-[(2,4-diméthoxyphényl) sulfonyl]-3-(2-isopropoxyphényl)-2-oxo-2,3-dihydro-1*H*-indol-3-yle ;
- N-[5-Chloro-1-[(2,4-diméthoxyphényl)sulfonyl]-3-(2-méthoxyphényl)-2-oxo-2,3-dihydro-1*H*-indol-3-yl]-4-(4-pyridinyl)homopipérazine-1-carboxamide ;
- N-[5-Chloro-1-[(2,4-diméthoxyphényl)sulfonyl]-3-(2-isopropoxyphényl)-2-oxo-2,3-dihydro-1*H*-indol-3-yl]-4-(3-pyridinyl)pipérazine-1-carboxamide ;
- N-[5-Chloro-1-[(2,4-diméthoxyphényl)sulfonyl]-3-(2-isopropoxyphényl)-2-oxo-2,3-dihydro-1*H*-indol-3-yl]-4-(4-pyridinyl)pipérazine-1-carboxamide ;
- 5-Chloro-1-[(2,4-diméthoxyphényl)sulfonyl]-3-(2-méthoxyphényl)-3-[[3-oxo-3-[4-(2-pyridinyl)-1-pipérazinyl] propyl]amino]-1,3-dihydro-2*H*-indol-2-one ;
- 5,6-Dichloro-1-[(2,4-diméthoxyphényl)sulfonyl]-3-(2-méthoxyphényl)-3-[2-oxo-2-[4-(4-pyridinyl)-1-pipérazinyl]éthyl]-1,3-dihydro-2*H*-indol-2-one ;
- 5-Chloro-1-[(2,4-diméthoxyphényl)sulfonyl]-3-(2-méthoxyphényl)-6-méthyl-3-[2-oxo-2-[4-(4-pyridinyl)-1-pipérazinyl]éthyl]-1,3-dihydro-2*H*-indol-2-one ;
- 4-(4-Pyridinyl)-1-pipérazinecarboxylate de 5-chloro-1-[(2,4-diméthoxyphényl) sulfonyl]-3-(2-méthoxyphényl)-6-méthyl-2-oxo-2,3-dihydro-1*H*-indol-3-yle ;
- 4-(4-Pyridinyl)-1-pipérazinecarboxylate de 5-chloro-1-[(2,4-diméthoxyphényl) sulfonyl]-6-méthoxy-3-(2-méthoxyphényl)-2-oxo-2,3-dihydro-1*H*-indol-3-yle;
- N-[5-Chloro-3-(2-chlorophényl)-1-[(2,4-diméthoxyphényl)sulfonyl]-2-oxo-2,3-dihydro-1*H*-indol-3-yl]-4-(2-pyridinyl)pipérazine-1-carboxamide ;
- N-[5-Chloro-1-[(2,4-diméthoxyphényl)sulfonyl]-3-(2-méthoxyphényl)-6-méthyl-2-oxo-2,3-dihydro-1*H*-indol-3-yl]-4-(4-pyridinyl)pipérazine-1-carboxamide ;

- N-[6-Chloro-3-(2-chlorophényl)-1-[(2,4-diméthoxyphényl)sulfonyl]-5-méthyl-2-oxo-2,3-dihydro-1*H*-indol-3-yl]-4-(4-pyridinyl)pipérazine-1-carboxamide ;
- 5-Chloro-1-[(2,4-diméthoxyphényl)sulfonyl]-3-(2-fluorophényl)-3-[2-oxo-2-[4-(4-pyridinyl)-1-pipérazinyl] éthyl]-1,3-dihydro-2*H*-indol-2-one ;
- 5,6-Dichloro-1-[(2,4-diméthoxyphényl)sulfonyl]-3-(2-fluorophényl)-3-[2-oxo-2-[4-(4-pyridinyl)-1-pipérazinyl] éthyl]-1,3-dihydro-2*H*-indol-2-one ;
- 5-Chloro-3-(2,3-diméthoxyphényl)-1-[(2,4-diméthoxyphényl)sulfonyl]-3-[2-oxo-2-[4-(4-pyridinyl)-1-pipérazinyl]éthyl]-1,3-dihydro-2*H*-indol-2-one ;
- 5-Chloro-1-[(2,4-diméthoxyphényl)sulfonyl]-3-(2-éthoxyphényl)-3-[2-oxo-2-[4-(3-pyridinyl)-1-pipérazinyl] éthyl]-1,3-dihydro-2*H*-indol-2-one ;
- 5-Chloro-1-[(2,4-diméthoxyphényl)sulfonyl]-3-(2-isopropoxyphényl)-3-[2-oxo-2-[4-(3-pyridinyl)-1-pipérazinyl] éthyl]-1,3-dihydro-2*H*-indol-2-one ;
- 5-Chloro-1-[(2,4-diméthoxyphényl)sulfonyl]-3-(2-isopropoxyphényl)-3-[2-oxo-2-[4-(4-pyridinyl)-1-homopipérazine]éthyl]-1,3-dihydro-2*H*-indol-2-one ;
- 5-Chloro-1-[(2,4-diméthoxyphényl)sulfonyl]-3-(2-isopropoxyphényl)-3-[2-oxo-2-[4-(1,3-thiazol-2-yl)-1-pipérazinyl]éthyl]-1,3-dihydro-2*H*-indol-2-one ;
- 4-(3-Pyridinyl)-1-pipérazinecarboxylate de 5-chloro-1-[(2,4-diméthoxyphényl) sulfonyl]-3-(2-éthoxyphényl)-2-oxo-2,3-dihydro-1*H*-indol-3-yle ;
- 4-(4-Pyridinyl)-1-pipérazinecarboxylate de 5-chloro-1-[(2,4-diméthoxyphényl) sulfonyl]-3-(2-éthoxyphényl)-2-oxo-2,3-dihydro-1*H*-indol-3-yle ;

sous forme d'isomères optiquement purs ou sous forme de mélange, ainsi que ses sels avec des acides minéraux ou organiques, ses solvats et/ou hydrates.

**4.** Procédé de préparation des composés de formule (I) selon la revendication 1, **caractérisé en ce que** : on fait réagir un composé de formule :

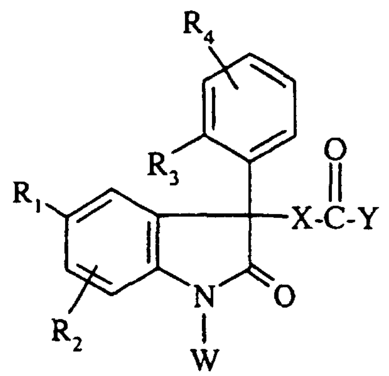

(II)

dans laquelle $R_1$, $R_2$, $R_3$, $R_4$ et X sont tels que définis pour un composé de formule (I) dans la revendication 1, et :

- Y représente un hydroxy ou un atome de chlore lorsque X représente un groupe $-CH_2-$ ; $-OCH_2-$ ; $-NH-CH_2-$ ; $-NH-CH_2-CH_2-$ ;
- ou Y représente un phénoxy lorsque X représente un groupe -O- ; -NH- ;
- W représente un atome d'hydrogène lorsque X représente un groupe $-CH_2-$ ; $-OCH_2-$ ;
- ou W représente un groupe

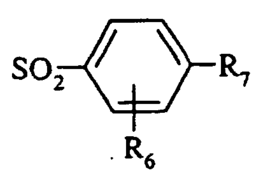

dans lequel $R_6$ et $R_7$ sont

tels que définis pour un composé de formule (I) dans la revendication 1, lorsque X représente un groupe -O- ; -NH- ; $-NH-CH_2-$ ; $-NH-CH_2-CH_2-$ ;
avec un composé de formule :

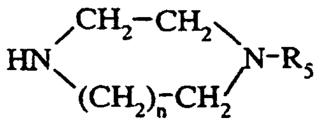

(III)

dans laquelle n et $R_5$ sont tels que définis pour un composé de formule (I) dans la revendication 1 ;

- lorsque W représente un groupe

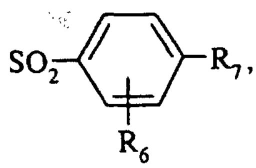

on obtient le composé de formule (I) attendu ;

- ou lorsque W représente un atome d'hydrogène, on fait réagir, en présence d'une base, le composé ainsi obtenu de formule :

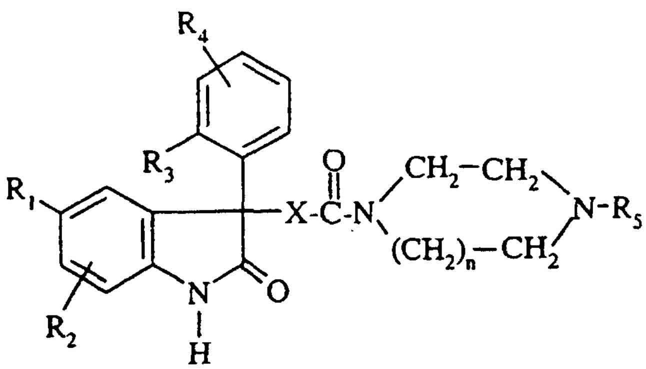

(IV)

avec un halogénure de sulfonyle de formule :

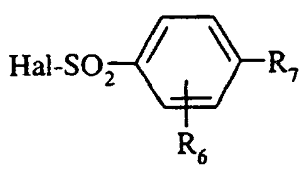

(V)

dans laquelle R6 et $R_7$ sont tels que définis pour un composé de formule (I) dar la revendication 1 et Hal représente un atome d'halogène.

**5.** Composé de formule :

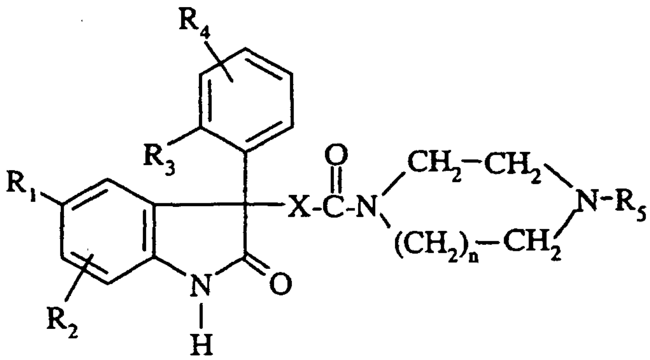 (IV

dans laquelle :

- n est 1 ou 2 ;
- X représente un groupe $-CH_2-$ ; $-O-CH_2-$ ;
- $R_1$ représente un atome d'halogène ; un $(C_1-C_4)$alkyle ; un $(C_1-C_4)$alcoxy ;
- $R_2$ représente un atome d'hydrogène ; un atome d'halogène ; un $(C_1-C_4)$alkyle ; un $(C_1-C_4)$alcoxy ; un radical trifluorométhyle ;
- $R_3$ représente un atome d'halogène ; un $(C_1-C_3)$alkyle ; un $(C_1-C_3)$alcoxy ; un radical trifluorométhyle ; un radical trifluorométhoxy ;
- $R_4$ représente un atome d'hydrogène ; un atome d'halogène ; un $(C_1-C_3)$alkyle ; un $(C_1-C_3)$alcoxy ;
- $R_5$ représente un radical choisi parmi :

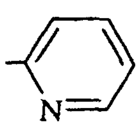 ; 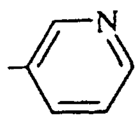 ; 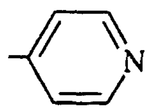 ; 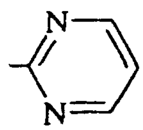 ; 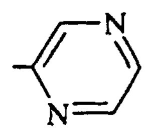 ; 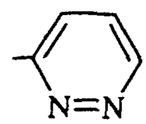 ;

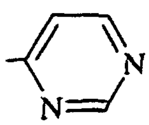 ; 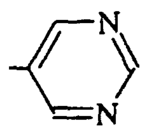 ; 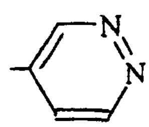 ; 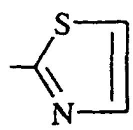 ; 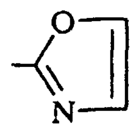 ; 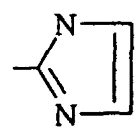

ainsi que ses sels avec des acides minéraux ou organiques, sous forme d'isomères optiquement purs ou sous forme de mélange.

6. Médicament **caractérisé en ce qu'**il est constitué d'un composé selon l'une quelconque des revendications 1 à 3 ou d'un de ses sels avec des acides minéraux ou organiques, un de ses solvats et/ou hydrates pharmaceutiquement acceptables.

7. Composition pharmaceutique **caractérisé en ce qu'**elle comprend, en tant que principe actif, un composé selon l'une quelconque des revendications 1 à 3, ou un de ses sels avec des acides minéraux ou organiques, de ses solvats et/ou hydrates pharmaceutiquement acceptables ainsi que un ou plusieurs excipients pharmaceutiquement acceptables.

8. Utilisation d'un composé selon l'une quelconque des revendications 1 à 3, de ses sels avec des acides minéraux ou organiques, de ses solvats et/ou hydrates pharmaceutiquement acceptables, pour la préparation de médicaments destinés à traiter les affections cardiovasculaires, le stress, l'anxiété, la dépression, le trouble obsessionnel compulsif, les attaques de paniques, les affections du système rénal, les affections du système gastrique, les cancers pulmonaires à petites cellules, l'obésité, le diabète de type II, la résistance à l'insuline, l'hypertriglycéridémie, l'athérosclérose, le syndrome de Cushing, la dysménorrhée et le travail prématuré.

## Patentansprüche

1. Eine Verbindung der Formel:

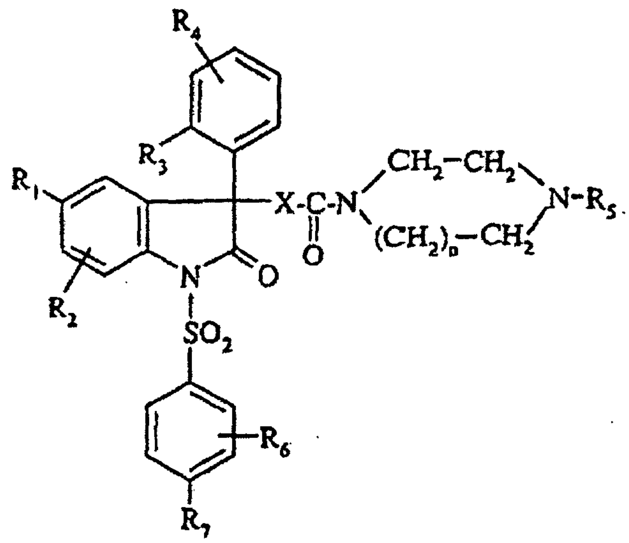

(I)

in der:

- n 1 oder 2 bedeutet;
- X eine Gruppe $-CH_2-$; -O-; -NH-; $-O-CH_2-$; $-NH-CH_2-$; $-NH-CH_2-CH_2$ bedeutet;
- $R_1$ ein Halogenatom; $(C_1-C_4)$-Alkyl; $(C_1-C_4)$-Alkoxy darstellt;
- $R_2$ ein Wasserstoffatom; ein Halogenatom; $(C_1-C_4)$-Alkyl; $(C_1-C_4)$-Alkoxy; eine Trifluormethylgruppe darstellt;
- $R_3$ ein Halogenatom; $(C_1-C_3)$-Alkyl; $(C_1-C_3)$-Alkoxy; eine Trifluormethylgruppe; eine Trifluormethoxygruppe darstellt;
- $R_4$ ein Wasserstoffatom; ein Halogenatom; $(C_1-C_3)$-Alkyl-, $(C_1-C_3)$-Alkoxy bedeutet;
- $R_5$ eine Gruppe bedeutet, ausgewählt aus:

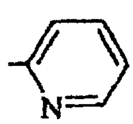 ; 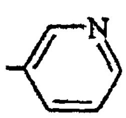 ; 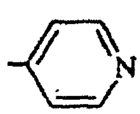 ; 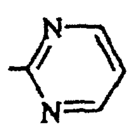 ; 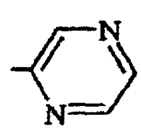 ; 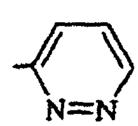 ;

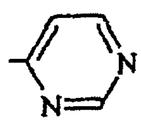 ; 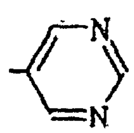 ; 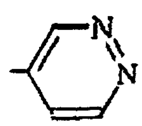 ; 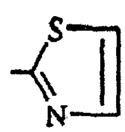 ; 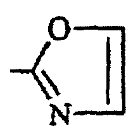 ; 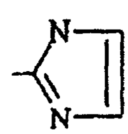

- $R_6$ $(C_1-C_4)$-Alkoxy bedeutet;
- $R_7$ $(C_1-C_4)$-Alkoxy bedeutet;

sowie deren Salze mit anorganischen oder organischen Säuren, deren Solvate und/oder deren Hydrate.

2. Verbindung nach Anspruch 1,worin:

- n und X die in Anspruch 1 für die Verbindung der Formel (I) angegebenen Bedeutungen besitzen;
- $R_1$ ein Chloratom oder eine Methylgruppe darstellt;
- $R_2$ ein Wasserstoffatom bedeutet oder in der Position -6- des Indol-2-on-Rests steht und ein Chloratom, eine

Methylgruppe, eine Methoxygruppe oder eine Trifluormethylgruppe bedeutet;

- $R_3$ ein Chloratom, ein Fluoratom, eine Methoxygruppe, eine Ethoxygruppe, eine Isopropoxygruppe, eine Trifluormethylgruppe oder eine Trifluormethoxygruppe darstellt;
- $R_4$ ein Wasserstoffatom oder eine Methoxygruppe bedeutet;
- $R_5$ 2-Pyridinyl, 3-Pyridinyl, 4-Pyridinyl, 2-Pyrimidinyl, 2-Pyrazinyl, 3-Pyridazinyl, 1,3-Thiazol-2-yl bedeutet;
- $R_6$ in der -2-Stellung des Phenylrests steht und eine Methoxygruppe bedeutet;
- $R_7$ eine Methoxygruppe darstellt;

sowie deren Salze mit anorganischen oder organischen Säuren, deren Solvate und/oder Hydrate.

**3.** Eine Verbindung ausgewählt aus:

- 5-Chlor-3-(2-ethoxyphenyl)-1-[(2,4-dimethoxyphenyl)-sulfonyl]-3-[2-oxo-2-[4-(4-pyridinyl)-1-piperazinyl]-ethyl]-1,3-dihydro-2*H*-indol-2-on;
- 5-Chlor-3-(2-isopropoxyphenyl)-1-[(2,4-dimethoxyphenyl)-sulfonyl]-3-[2-oxo-2-[4-(4-pyridinyl)-1-piperazinyl]-ethyl]-1,3-dihydro-2*H*-indol-2-on;
- 5-Chlor-1-[(2,4-dimethoxyphenyl)-sulfonyl]-3-(2-methoxyphenyl)-2-oxo-2,3-dihydro-1*H*-indol-3-yl-4-(2-pyridinyl)-1-piperazincarboxylat;
- 5-Chlor-1-[(2,4-dimethoxyphenyl)-sulfonyl]-3-(2-isopropoxyphenyl)-2-oxo-2,3-dihydro- 1*H*-indol-3-yl-4-(4-pyridinyl)-1-piperazincarboxylat;
- N-[5-Chlor-1-[(2,4-dimethoxyphenyl)-sulfonyl]-3-(2-methoxyphenyl)-2-oxo-2,3-dihydro-1*H*-indol-3-yl]-4-(4-pyridinyl)-homopiperazin-1-carboxamid;
- N-[5-Chlor-1-[(2,4-dimethoxyphenyl)-sulfonyl]-3-(2-isopropoxyphenyl)-2-oxo-2,3-dihydro-1*H*-indol-3-yl]-4-(3-pyridinyl)-piperazin-1-carboxamid;
- N-[5-Chlor-1-[(2,4-dimethoxyphenyl)-sulfonyl]-3-(2-isopropoxyphenyl)-2-oxo-2, 3-dihydro-1*H*-indol-3-yl]-4-(4-pyridinyl)-piperazin-1-carboxamid;
- 5-Chlor-1-[(2,4-dimethoxyphenyl)-sulfonyl]-3-(2-methoxyphenyl)-3-[[3-oxo-3-[4-(2-pyridinyl)-1-piperazinyl]-propyl]-amino]-1,3-dihydro-2*H*-indol-2-on;
- 5,6-Dichlor-1-[(2,4-dimethoxyphenyl)-sulfonyl]-3-(2-methoxyphenyl)-3-[2-oxo-2-[4-(4-pyridinyl)-1-piperazinyl]-ethyl]-1,3-dihydro-2*H*-indol-2-on;
- 5-Chlor-1-[(2,4-dimethoxyphenyl)-sulfonyl]-3-(2-methoxyphenyl)-6-methyl-3-[2-oxo-2-[4-(4-pyridinyl)-1-piperazinyl]-ethyl]-1,3-dihydro-2*H*-indol-2-on;
- 5-Chlor-1-[(2,4-dimethoxyphenyl)-sulfonyl]-3-(2-methoxyphenyl)-6-methyl-2-oxo-2,3-dihydro-1*H*-indol-3-yl-4-(4-pyridinyl)-1-piperazincarboxylat;
- 5-Chlor-1-[(2,4-dimethoxyphenyl)-sulfonyl]-6-methoxy-3-(2-methoxyphenyl)-2-oxo-2,3-dihydro-1*H*-indol-3-yl-4-(4-pyridinyl)-1-piperazincarboxylat;
- N-[5-Chlor-3-(2-chlorphenyl)-1-[(2,4-dimethoxyphenyl)-sulfonyl]-2-oxo-2,3-dihydro-1*H*-indol-3-yl]-4-(2-pyridinyl)-1-carboxamid;
- N-[5-Chlor-1-[(2,4-dimethoxyphenyl)-sulfonyl]-3-(2-methoxyphenyl)-6-methyl-2-oxo-2,3-dihydro-1*H*-indol-3-yl]-4-(4-pyridinyl)-piperazin-1-carboxamid;
- N-[6-Chlor-3-(2-chlorphenyl)-1-[(2,4-dimethoxyphenyl)-sulfonyl]-5-methyl-2-oxo-2,3-dihydro-1*H*-indol-3-yl]-4-(4-pyridinyl)-piperazin-1-carboxamid;
- 5-Chlor-1-[(2,4-dimethoxyphenyl)-sulfonyl]-3-(2-fluorphenyl)-3-[2-oxo-2-[4-(4-pyridinyl)-1-piperazinyl]-ethyl]-1,3-dihydro-2*H*-indol-2-on;
- 5,6-Dichlor-1-[(2,4-dimethoxyphenyl)-sulfonyl]-3-(2-fluorphenyl)-3-[2-oxo-2-[4-(4-pyridinyl)-1-piperazinyl]-ethyl]-1,3-dihydro-2*H*-indol-2-on;
- 5-Chlor-3-(2,3-dimethoxyphenyl)-1-[(2,4-dimethoxyphenyl)-sulfonyl]-3-[2-oxo-2-[4-(4-pyridinyl)-1-piperazinyl]-ethyl]-1,3-dihydro-2*H*-indol-2-on;
- 5-Chlor-1-[(2,4-dimethoxyphenyl)-sulfonyl]-3-(2-ethoxyphenyl)-3-[2-oxo-2-[4-(3-pyridinyl)-1-piperazinyl]-ethyl]-1,3-dihydro-2*H*-indol-2-on;
- 5-Chlor-1-[(2,4-dimethoxyphenyl)-sulfonyl]-3-(2-isopropoxyphenyl)-3-[2-oxo-2-[4-(3-pyridinyl)-1-piperazinyl]-ethyl]-1,3-dihydro-2*H*-indol-2-on;
- 5-Chlor-1-[(2,4-dimethoxyphenyl)-sulfonyl]-3-(2-isopropoxyphenyl)-3-[2-oxo-2-[4-(4-pyridinyl)-1-homopiperazin]-ethyl]-1,3-dihydro-2*H*-indol-2-on;
- 5-Chlor-1-[(2,4-dimethoxyphenyl)-sulfonyl]-3-(2-isopropoxyphenyl)-3-[2-oxo-2-[4-(1,3-thiazol-2-yl)-1-piperazinyl]-ethyl]-1,3-dihydro-2*H*-indol-2-on;
- 5-Chlor-1-[(2,4-dimethoxyphenyl)-sulfonyl]-3-(2-ethoxyphenyl)-2-oxo-2,3-dihydro-1*H*-indol-3-yl-4-(3-pyridinyl)-1-piperazincarboxylat;

- 5-Chlor-1-[(2,4-dimethoxyphenyl)-sulfonyl]-3-(2-ethoxyphenyl)-2-oxo-2,3-dihydro-1*H*-indol-3-yl-4-(4-pyridinyl)-1-piperazincarboxylat;

in Form der optisch reinen Isomeren oder in Form einer Mischung, sowie deren Salze mit anorganischen oder organischen Säuren, deren Solvate und/oder Hydrate.

**4.** Verfahren zur Herstellung der Verbindungen der Formel (I) nach Anspruch 1, **dadurch gekennzeichnet, daß** man: eine Verbindung der Formel:

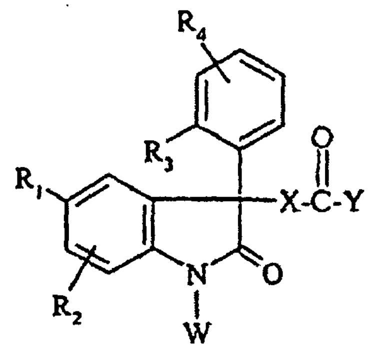

(II)

in der $R_1$, $R_2$, $R_3$, $R_4$ und X die in Anspruch 1 für die Verbindung der Formel (I) angegebenen Bedeutungen besitzen, und:

- Y eine Hydroxygruppe oder ein Chloratom darstellt, wenn X eine Gruppe der Formel $-CH_2-$; $-OCH_2-$; $-NH-CH_2-$; $-NH-CH_2-CH_2-$ bedeutet;
- oder Y eine Phenoxygruppe bedeutet, wenn X eine Gruppe -O-; oder -NH- darstellt;
- W ein Wasserstoffatom bedeutet, wenn X eine Gruppe $-CH_2-$; oder $-OCH_2-$ darstellt;
- oder W eine Gruppe der Formel

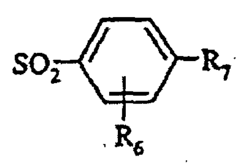

bedeutet, in der $R_6$ und $R_7$ die in Anspruch 1 für die Verbindung der Formel (I) angegebenen Bedeutungen besitzen, wenn X eine Gruppe -O-; -NH-; $-NH-CH_2-$; oder $-NH-CH_2-CH_2-$ darstellt;
mit einer Verbindung der Formel:

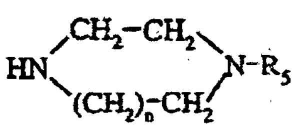

(III)

in der n und $R_5$ die in Anspruch 1 für die Verbindung der Formel (I) angegebenen Bedeutungen besitzen; umsetzt, so daß man

- wenn W eine Gruppe

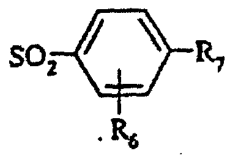

bedeutet, die erwartete Verbindung der Formel (I) erhält;

- oder wenn W ein Wasserstoffatom bedeutet, man die in dieser Weise erhaltene Verbindung der Formel:

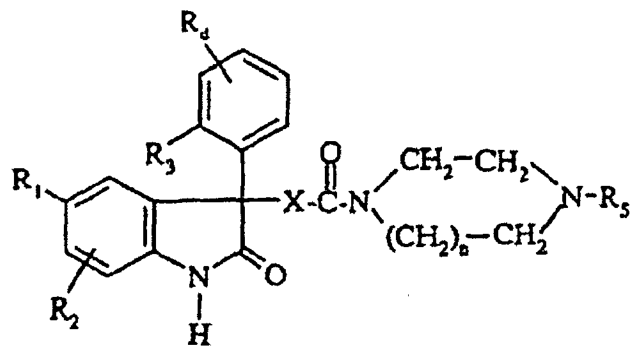

(IV)

in Gegenwart einer Base mit einem Sulfonylhalogenid der Formel umsetzt:

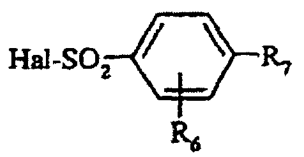

(V)

in der $R_6$ und $R_7$ die in Anspruch 1 für die Verbindung der Formel (I) angegebenen Bedeutungen besitzen und Hal ein Halogenatom darstellt.

**5.** Verbindung der Formel:

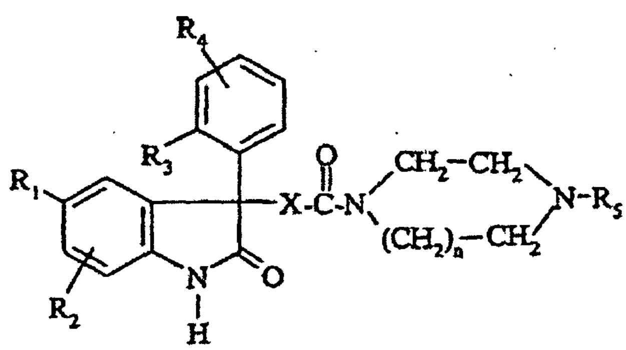

(IV)

in der:

- n 1 oder 2 bedeutet;
- X eine Gruppe $-CH_2-$; $-O-CH_2-$ darstellt;
- $R_1$ ein Halogenatom; $(C_1-C_4)$-Alkyl; $(C_1-C_4)$-Alkoxy bedeutet;
- $R_2$ ein Wasserstoffatom; ein Halogenatom; $(C_1-C_4)$-Alkyl; $(C_1-C_4)$-Alkoxy; eine Trifluormethylgruppe bedeutet;

- $R_3$ ein Halogenatom; $(C_1\text{-}C_3)$-Alkyl; $(C_1\text{-}C_3)$-Alkoxy; eine Trifluormethylgruppe; eine Trifluormethoxygruppe bedeutet;
- $R_4$ ein Wasserstoffatom; ein Halogenatom; $(C_1\text{-}C_3)$-Alkyl; $(C_1\text{-}C_3)$-Alkoxy darstellt;
- $R_5$ eine Gruppe bedeutet, ausgewählt aus:

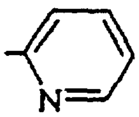 ; 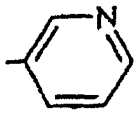 ; 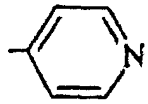 ; 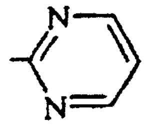 ; 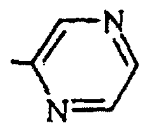 ; 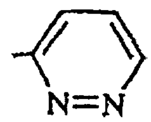 ;

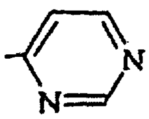 ; 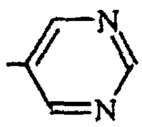 ; 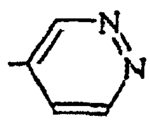 ; 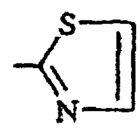 ; 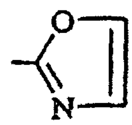 ; 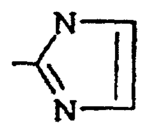

sowie deren Salze mit anorganischen oder organischen Säuren, in Form der optisch reinen Isomeren oder in Form einer Mischung.

**6.** Arzneimittel, **dadurch gekennzeichnet, daß** es aus einer Verbindung nach einem der Ansprüche 1 bis 3 oder eines ihrer pharmazeutisch annehmbaren Salze mit anorganischen oder organischen Säuren, eines ihrer Solvate und/oder Hydrate gebildet ist.

**7.** Pharmazeutische Zubereitung, **dadurch gekennzeichnet, daß** sie als Wirkstoff eine Verbindung nach einem der Ansprüche 1 bis 3 oder eines ihrer pharmazeutisch annehmbaren Salze mit anorganischen oder organischen Säuren, Solvate und/oder Hydrate sowie ein oder mehrere pharmazeutisch annehmbare Trägermaterialien enthält.

**8.** Verwendung einer Verbindung nach einem der Ansprüche 1 bis 3, ihrer pharmazeutisch annehmbaren Salze mit anorganischen oder organischen Säuren, ihrer Solvate und/oder Hydrate für die Herstellung von Arzneimitteln zur Behandlung von kardiovaskulären Erkrankungen, Stress, Angst, der Depression, krampfartigen Besessenheitsstörungen, Panikanfällen, Erkrankungen des Nierensystems, Erkrankungen des Magensystems, Lungenkrebs mit kleinen Zellen, Fettsucht, Diabetes Typ II, Insulinresistenz, Hypertriglyceridämie, Atherosklerose, des Cushing-Syndroms, der Dysmenorrhoe und von vorzeitigen Wehen.

## Claims

**1.** Compound of formula:

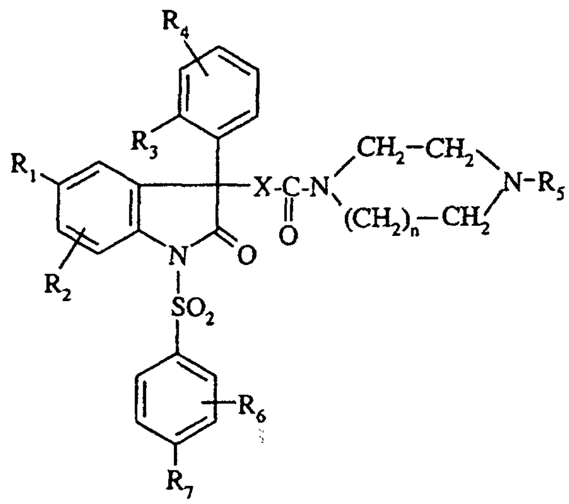

(I)

in which:

- n is 1 or 2;
- X represents a group $-CH_2-$; -O-; -NH-; $-O-CH_2-$; $-NH-CH_2-$; $-NH-CH_2-CH_2-$;
- $R_1$ represents a halogen atom; a $(C_1-C_4)$alkyl; a $(C_1-C_4)$alkoxy;
- $R_2$ represents a hydrogen atom; a halogen atom; a $(C_1-C_4)$alkyl; a $(C_1-C_4)$alkoxy; a trifluoromethyl radical;
- $R_3$ represents a halogen atom; a $(C_1-C_3)$alkyl; a $(C_1-C_3)$alkoxy; a trifluoromethyl radical; a trifluoromethoxy radical;
- $R_4$ represents a hydrogen atom; a halogen atom; a $(C_1-C_3)$alkyl; a $(C_1-C_3)$alkoxy;
- $R_5$ represents a radical chosen from:

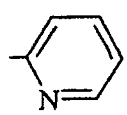 ; 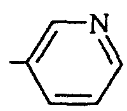 ; 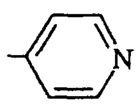 ; 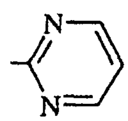 ; 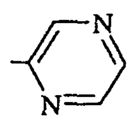 ; 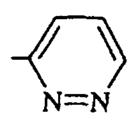 ;

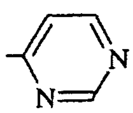 ; 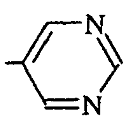 ; 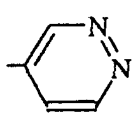 ; 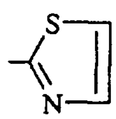 ; 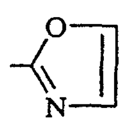 ; 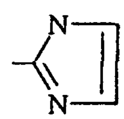

- $R_6$ represents a $(C_1-C_4)$alkoxy;
- $R_7$ represents a $(C_1-C_4)$alkoxy;

and also the salts thereof with mineral or organic acids, the solvates thereof and/or the hydrates thereof.

**2.** Compound according to Claim 1, in which:

- n and X are as defined for a compound of formula (I) in Claim 1;
- $R_1$ represents a chlorine atom or a methyl radical;
- $R_2$ represents a hydrogen atom or is in position -6 of the indol-2-one and represents a chlorine atom, a methyl radical, a methoxy radical or a trifluoromethyl radical;
- $R_3$ represents a chlorine atom, a fluorine atom, a methoxy radical, an ethoxy radical, an isopropoxy radical, a trifluoromethyl radical or a trifluoromethoxy radical;
- $R_4$ represents a hydrogen atom or a methoxy radical;
- $R_5$ represents a 2-pyridyl, a 3-pyridyl, a 4-pyridyl, a 2-pyrimidinyl, a 2-pyrazinyl, a 3-pyridazinyl or a 1,3-thiazol-2-yl;

- $R_6$ is in position -2 of the phenyl and represents a methoxy radical;
- $R_7$ represents a methoxy radical; and also the salts thereof with mineral or organic acids, and the solvates and/or hydrates thereof.

**3.** Compound chosen from:

- 5-chloro-3-(2-ethoxyphenyl)-1-[(2,4-dimethoxyphenyl)sulphonyl]-3-[2-oxo-2-[4-(4-pyridyl)-1-piperazinyl]ethyl]-1,3-dihydro-2*H*-indol-2-one;
- 5-chloro-3-(2-isopropoxyphenyl)-1-[(2,4-dimethoxyphenyl)sulphonyl]-3-[2-oxo-2-[4-(4-pyridyl)-1-piperazinyl]ethyl]-1,3-dihydro-2*H*-indol-2-one;
- 5-chloro-1-[(2,4-dimethoxyphenyl)sulphonyl]-3-(2-methoxyphenyl)-2-oxo-2,3-dihydro-1*H*-indol-3-yl 4-(2-pyridyl)-1-piperazinecarboxylate;
- 5-chloro-1-[(2,4-dimethoxyphenyl)sulphonyl]-3-(2-isopropoxyphenyl)-2-oxo-2,3-dihydro-1*H*-indol-3-yl4-(4-pyridyl)-1-piperazinecarboxylate;
- N-[5-chloro-1-[(2,4-dimethoxyphenyl)-sulphonyl]-3-(2-methoxyphenyl)-2-oxo-2,3-dihydro-1*H*-indol-3-yl]-4-(4-pyridyl)homopiperazine-1-carboxamide;
- N-[5-chloro-1-[(2,4-dimethoxyphenyl)-sulphonyl]-3-(2-isopropoxyphenyl)-2-oxo-2,3-dihydro-1*H*-indol-3-yl]-4-(3-pyridyl)piperazine-1-carboxamide;
- N-[5-chloro-1-[(2,4-dimethoxyphenyl)-sulphonyl]-3-(2-isopropoxyphenyl)-2-oxo-2,3-dihydro-1*H*-indol-3-yl]-4-(4-pyridyl)piperazine-1-carboxamide;
- 5-chloro-1-[(2,4-dimethoxyphenyl)sulphonyl]-3-(2-methoxyphenyl)-3-[[3-oxo-3-[4-(2-pyridyl)-1-piperazinyl]propyl]amino]-1,3-dihydro-2*H*-indol-2-one;
- 5,6-dichloro-1-[(2,4-dimethoxyphenyl)-sulphonyl]-3-(2-methoxyphenyl)-3-[2-oxo-2-[4-(4-pyridyl)-1-piperazinyl]ethyl]-1,3-dihydro-2*H*-indol-2-one;
- 5-chloro-1-[(2,4-dimethoxyphenyl)sulphonyl]-3-(2-methoxyphenyl)-6-methyl-3-[2-oxo-2-[4-(4-pyridyl)-1-piperazinyl]ethyl]-1,3-dihydro-2*H*-indol-2-one;
- 5-chloro-1-[(2,4-dimethoxyphenyl)sulphonyl]-3-(2-methoxyphenyl)-6-methyl-2-oxo-2,3-dihydro-1*H*-indol-3-yl 4-(4-pyridyl)-1-piperazinecarboxylate;
- 5-chloro-1-[(2,4-dimethoxyphenyl)sulphonyl]-6-methoxy-3-(2-methoxyphenyl)-2-oxo-2,3-dihydro-1*H*-indol-3-yl 4-(4-pyridyl)-1-piperazinecarboxylate;
- N-[5-chloro-3-(2-chlorophenyl)-1-[(2,4-dimethoxyphenyl)sulphonyl]-2-oxo-2,3-dihydro-1*H*-indol-3-yl]-4-(2-pyridyl)piperazine-1-carboxamide;
- N-[5-chloro-1-[(2,4-dimethoxyphenyl)-sulphonyl]-3-(2-methoxyphenyl)-6-methyl-2-oxo-2,3-dihydro-1*H*-indol-3-yl]-4-(4-pyridyl)piperazine-1-carboxamide;
- N-[6-chloro-3-(2-chlorophenyl)-1-[(2,4-dimethoxyphenyl)sulphonyl]-5-methyl-2-oxo-2,3-dihydro-1*H*-indol-3-yl]-4-(4-pyridyl)piperazine-1-carboxamide;
- 5-chloro-1-[(2,4-dimethoxyphenyl)sulphonyl]-3-(2-fluorophenyl)-3-[2-oxo-2-[4-(4-pyridyl)-1-piperazinyl]ethyl]-1,3-dihydro-2*H*-indol-2-one;
- 5,6-dichloro-1-[(2,4-dimethoxyphenyl)-sulphonyl]-3-(2-fluorophenyl)-3-[2-oxo-2-[4-(4-pyridyl)-1-piperazinyl]ethyl]-1,3-dihydro-2*H*-indol-2-one;
- 5-chloro-3-(2,3-dimethoxyphenyl)-1-[(2,4-dimethoxyphenyl)sulphonyl]-3-[2-oxo-2-[4-(4-pyridyl)-1-piperazinyl]ethyl]-1,3-dihydro-2*H*-indol-2-one;
- 5-chloro-1-[(2,4-dimethoxyphenyl)sulphonyl]-3-(2-ethoxyphenyl)-3-[2-oxo-2-[4-(3-pyridyl)-1-piperazinyl]ethyl]-1,3-dihydro-2*H*-indol-2-one;
- 5-chloro-1-[(2,4-dimethoxyphenyl)sulphonyl]-3-(2-isopropoxyphenyl)-3-[2-oxo-2-[4-(3-pyridyl)-1-piperazinyl]ethyl]-1,3-dihydro-2*H*-indol-2-one;
- 5-chloro-1-[(2,4-dimethoxyphenyl)sulphonyl]-3-(2-isopropoxyphenyl)-3-[2-oxo-2-[4-(4-pyridyl)-1-homopiperazine]ethyl]-1,3-dihydro-2*H*-indol-2-one;
- 5-chloro-1-[(2,4-dimethoxyphenyl)sulphonyl]-3-(2-isopropoxyphenyl)-3-[2-oxo-2-[4-(1,3-thiazol-2-yl)-1-piperazinyl]ethyl]-1,3-dihydro-2*H*-indol-2-one;
- 5-chloro-1-[(2,4-dimethoxyphenyl)sulphonyl]-3-(2-ethoxyphenyl)-2-oxo-2,3-dihydro-1*H*-indol-3-yl 4-(3-pyridyl)-1-piperazinecarboxylate;
- 5-chloro-1-[(2,4-dimethoxyphenyl)sulphonyl]-3-(2-ethoxyphenyl)-2-oxo-2,3-dihydro-1*H*-indol-3-yl 4-(4-pyridyl)-1-piperazinecarboxylate;

in the form of optically pure isomers or in the form of a mixture, and also the salts thereof with mineral or organic acids, and the solvates and/or hydrates thereof.

**4.** Process for preparing the compounds of formula (I) according to Claim 1, **characterized in that**:
a compound of formula:

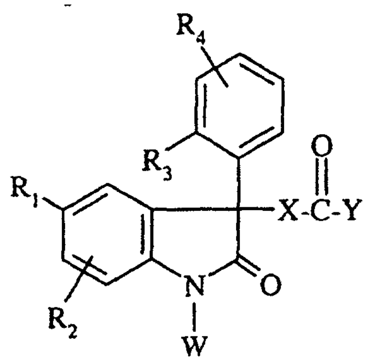

(II)

in which $R_1$, $R_2$, $R_3$, $R_4$ and X are as defined for a compound of formula (I) in Claim 1, and:

- Y represents a hydroxyl or a chlorine atom when X represents a group $-CH_2-$; $-OCH_2-$; $-NH-CH_2-$; $-NH-CH_2-CH_2-$;
- or Y represents a phenoxy when X represents a group -O-; -NH-;
- W represents a hydrogen atom when X represents a group $-CH_2-$; $-OCH_2-$;
- or W represents a group

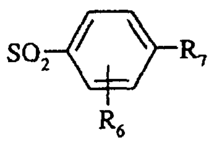

in which $R_6$ and $R_7$ are as defined for a compound of formula (I) in Claim 1 when X represents a group -O-; -NH-; $-NH-CH_2-$; $-NH-CH_2-CH_2-$;

is reacted with a compound of formula:

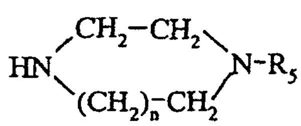

(III)

in which n and $R_5$ are as defined for a compound of formula (I) in Claim 1;

- when W represents a group

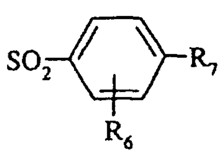

,

to give the expected compound of formula (I);
- or, when W represents a hydrogen atom, the compound thus obtained of formula:

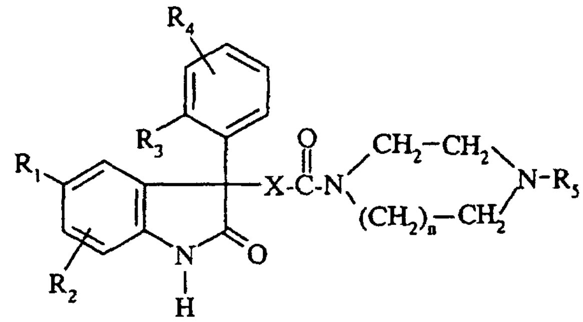 (IV)

is reacted, in the presence of a base, with a sulphonyl halide of formula:

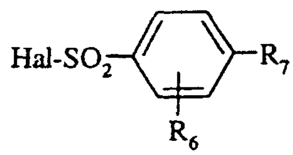 (V)

in which $R_6$ and $R_7$ are as defined for a compound of formula (I) in Claim 1 and Hal represents a halogen atom.

**5.** Compound of formula:

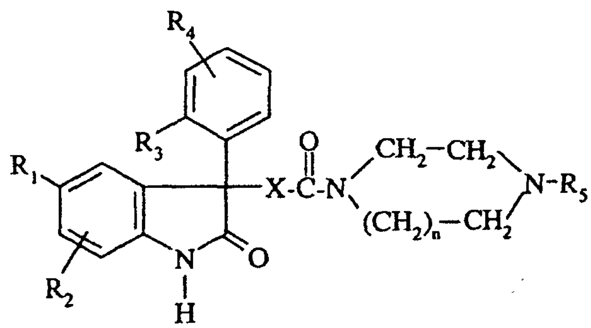 (IV)

in which:

- n is 1 or 2;
- X represents a group $-CH_2-$; $-O-CH_2-$;
- $R_1$ represents a halogen atom; a $(C_1-C_4)$alkyl; a $(C_1-C_4)$alkoxy;
- $R_2$ represents a hydrogen atom; a halogen atom; a $(C_1-C_4)$alkyl; a $(C_1-C_4)$alkoxy; a trifluoromethyl radical;
- $R_3$ represents a halogen atom; a $(C_1-C_3)$alkyl; a $(C_1-C_3)$alkoxy; a trifluoromethyl radical; a trifluoromethoxy radical;
- $R_4$ represents a hydrogen atom; a halogen atom; a $(C_1-C_3)$alkyl; a $(C_1-C_3)$alkoxy;
- $R_5$ represents a radical chosen from:

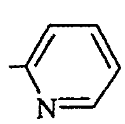 ; 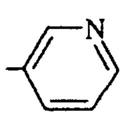 ; 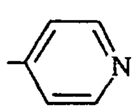 ; 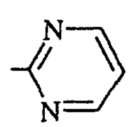 ; 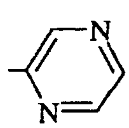 ; 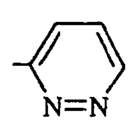 ;

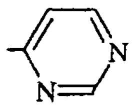 ; 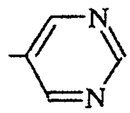 ; 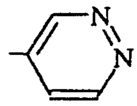 ; 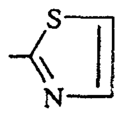 ; 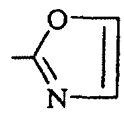 ; 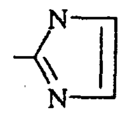

and also the salts thereof with mineral or organic acids, in the form of optically pure isomers or in the form of a mixture.

**6.** Medicinal product, **characterized in that** it consists of a compound according to any one of Claims 1 to 3, or of a pharmaceutically acceptable salt thereof with mineral or organic acids, and of a pharmaceutically acceptable solvate and/or hydrate thereof.

**7.** Pharmaceutical composition, **characterized in that** it comprises, as active principle, a compound according to any one of Claims 1 to 3, or a pharmaceutically acceptable salt thereof with mineral or organic acids, and a pharmaceutically acceptable solvate and/or hydrate thereof, and also one or more pharmaceutically acceptable excipients.

**8.** Use of a compound according to any one of Claims 1 to 3, of pharmaceutically acceptable salts thereof with mineral or organic acids, and of pharmaceutically acceptable solvates and/or hydrates thereof, for the preparation of medicinal products for treating cardiovascular conditions, stress, anxiety, depression, obsessive compulsive disorder, panic attacks, complaints of the renal system, complaints of the gastric system, small-cell lung cancers, obesity, type II diabetes, insulin resistance, hypertriglyceridaemia, atherosclerosis, Cushing's syndrome, dysmenorrhoea and premature labour.